# EUROPEAN PATENT APPLICATION

(11) **EP 4 631 937 A1**
(43) Date of publication of application: **15.10.2025**
(21) Application number: 23900015.1
(22) Date of filing: 06.12.2023
(51) Int. Cl.: C07D 401/04, A61K 31/454, A61K 31/445, A61P 35/00

(54) **CRYSTALLINE FORM OR AMORPHOUS FORM OF OXOISOINDOLE-5-FORMAMIDE COMPOUND OR SALT AND SOLVATE THEREOF**

(30) Priority: 07.12.2022 CN 202211567374
(71) Applicant: Hangzhou Glubio Pharmaceutical Co., Ltd., Hangzhou, Zhejiang 310018 (CN)
(72) Inventor: FU, Liqiang, Hangzhou, Zhejiang 310018 (CN); QI, Zude, Hangzhou, Zhejiang 310018 (CN); WANG, Zheng, Hangzhou, Zhejiang 310018 (CN); JIA, Miao, Hangzhou, Zhejiang 310018 (CN); ZHANG, Lei, Hangzhou, Zhejiang 310018 (CN)
(74) Representative: Ipsilon
(86) International application number: PCT/CN2023/136788
(87) International publication number: WO 2024/120441

(57) **Abstract**

The present invention relates to a crystalline form or an amorphous form of an oxoisoindole-5-formamide compound or a salt and solvate thereof. Also disclosed are a composition having the crystalline form or the amorphous form, a preparation method therefor and a use thereof. The obtained crystalline form or amorphous form has good solid stability, and has very important value for drug development, and preparation development and production.

## Description

### TECHNICAL FIELD

The present invention belongs to the field of medicine, and particularly relates to a crystalline form or an amorphous form of an oxoisoindole-5-formamide compound or a salt and solvate thereof used as a CK1α selective molecular glue degrading agent, and a preparation method therefor and use thereof.

### BACKGROUND

Casein kinase 1α (CK1α), encoded by the gene CSNK1A1, is a ubiquitously expressed serine/threonine protein kinase in the CK1 kinase family. CK1α participates in regulating a variety of physiological and pathological processes in cells and coordinates the orderly progress of life through different signal transduction pathways (Jiang et al., Cell Commun Signal (2018) 16: 23). For example, CK1α, as a key regulator of the Wnt/β-catenin pathway, directly phosphorylates β-catenin at Ser45, making it a target for protein degradation (Liu et al., Cell (2002) 108: 837-847). CK1α is also considered to regulate the protein stability of the tumor suppressor p53 by regulating the activity of the MDM2/MDMX E3 ligase active complex (Huart et al., J Biol Chem (2009) 284: 32384-94; Wu et al., Mol Cell Biol (2012) 32:4821-4832). It is reported that CK1α is overexpressed in many types of human cancers. However, the exact role of CK1α in the development of multiple tumor types has not been clearly elucidated (Richter et al., BMC Cancer (2018) 18: 140). The Cancer Dependency Map (DepMap) database showed that inactivation of CK1α by CRISPR/cas9-mediated gene knockout or shRNA-mediated gene inhibition significantly reduced the proliferation and/or survival of many cancer cell lines in multiple cancer types (Tshemiak et al., Cell (2017) 170:564-576; Behan et al., Nature (2019) 568: 511-516). In addition, inhibition of CK1α activity by using shRNA interference or D4476 (a CK1α inhibitor) may effectively inhibit the progression of MLL-AF9 leukemia and had little effect on normal hematopoietic stem and progenitor cells (HSPCs) (Jaras et al, J Exp Med (2014) 211(4): 605-612).In summary, these data indicate that CK1α is a potential target for treating indications of hematological malignancies and solid tumor.

The compound of formula A is a new generation of CK1α selective molecular glue degrader and is currently in the early stage of clinicalstudy.

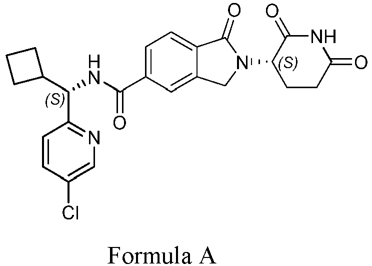

However, there is no research or report on the crystalline form or amorphous form of the compound of formula A or salt or solvate thereof, and preparation method therefor, use, and formulation thereof.

Due to the influence of various factors such as the configuration and conformation of the molecular structure, molecular arrangement, molecular forces, and eutectic substances, solid substances have different molecular lattice spatial arrangements, forming two or more different crystal structures. This phenomenon is called "Polymorphism Phenomenon" or "Allomorphism Phenomenon". "Polymorphism Phenomenon" widely occurs in solid drugs. There may be differences in physical and chemical properties among different crystal forms of the same drug, such as appearance, density, hardness, melting point, solubility, stability, dissolution, dissolution rate, bioavailability, etc. This phenomenon is particularly evident in oral solid formulations. In addition, the form and quantity of polymorphic compounds are unpredictable. Different crystalline forms of the same drug have significant differences in solubility, melting point, density, stability, etc., which affect the uniformity, bioavailability, efficacy and safety of the drug to different extent.

In addition to polymorphs, some solid compounds may also be present in amorphous forms. Amorphous form refers to the structure of some non-completely crystalline amorphous regions (non-crystalline regions) or the composition of some amorphous solids (non-crystalline). For a specific solid drug, the form and quantity of its amorphous form are also unpredictable, and may have a significant effect on the solubility, melting point, density, stability, etc. of the drug as well.

Therefore, in the process of new drug development, it is necessary to screen the drug compounds comprehensively and consider multiple factors. In particular, for the above-mentioned compound of formula A for treating proliferative disorders, developing a dosage form of the compound or a derivative, a crystalline form, an amorphous form, or a pharmaceutically acceptable salt, a hydrate or a solvate thereof that may have pharmaceutical value has potential medicinal and clinical value for improving the stability, solubility, bioavailability and other properties of the compound.

### SUMMARY OF THE INVENTION

The present invention provides a crystalline form or amorphous form of an oxoisoindole-5-carboxamide compound or a salt or solvate thereof used as a CK1α selective molecular glue degrading agent, and a preparation method therefor, and application thereof. The crystalline form or amorphous form of the present invention has very important value for drug development, formulation development and production.

In the following description, certain specific details are set forth in order to provide a thorough understanding of various embodiments of the present invention. However, it will be understood by those skilled in the art that the present invention may be practiced without these details. The following description of several embodiments is carried out with the understanding that the present disclosure is considered as an example of the claimed subject matter, and is not intended to limit the appended claims to the displayed specific embodiments. The titles used throughout the present invention are provided only for convenience and should not be interpreted as limiting the claims in any way. The embodiment shown under any title may be combined with an embodiment displayed under any other title.

When referring to, for example, an XRPD pattern, a TGA pattern, a DSC pattern, etc., the term "substantially as shown" refers to patterns that are not necessarily the same as those described herein, but that fall within the limits of experimental error or deviation when considered by an ordinary technician in the art.

In one aspect, the present invention provides an amorphous form or a crystalline form of the compound of formula A or a pharmaceutically acceptable salt or solvate thereof.

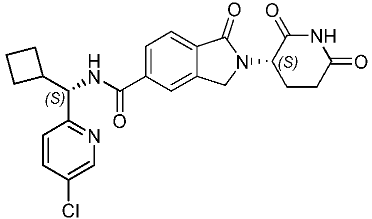

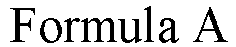

The chemical name of the compound is N-((S)-(5-chloropyridin-2-yl)(cyclobutyl)methyl)-2-((*S*)-2,6-dioxopiperidin-3-yl)-1-oxoisoindoline-5-carboxamide.

The present invention includes, but is not limited to, crystalline form A1, crystalline form A2, crystalline form A3, crystalline form A4, crystalline form A5, crystalline form B, crystalline form C, crystalline form D1, crystalline form D2, crystalline form E, crystalline form F, crystalline form G, crystalline form H and amorphous form. Specifically as follows:

### Solvate crystalline form A1 of the compound of formula A

In one embodiment, the form is a solvate crystalline form A1 of the compound of formula A, which has at least three, at least four, at least five, at least six or seven characteristic peaks at the following positions in an X-ray powder diffraction (XRPD) pattern in 2θ angle: 6.54 ± 0.2°, 19.64 ± 0.2°, 9.21 ± 0.2°, 16.35 ± 0.2°, 18.48 ± 0.2°, 9.79 ± 0.2° and 17.23 ± 0.2°.

In some preferred embodiments, the form has XRPD characteristic peaks at positions substantially as shown in Table 1 below:

**Table 1**

| Index | Angle | Intensity | Rel. Intensity |
|---|---|---|---|
| 1 | 3.263 ° | 265.832 | 13.2% |
| 2 | 6.548 ° | 786.273 | 100.0% |
| 3 | 9.217 ° | 583.802 | 70.2% |
| 4 | 9.797 ° | 347.535 | 36.7% |
| 5 | 10.833 ° | 135.383 | 7.2% |
| 6 | 13.062 ° | 285.795 | 28.0% |
| 7 | 15.687 ° | 264.759 | 22.4% |
| 8 | 16.355 ° | 548.609 | 61.3% |
| 9 | 17.238 ° | 353.582 | 32.6% |
| 10 | 17.532 ° | 350.953 | 31.6% |
| 11 | 118.487 ° | 546.661 | 57.9% |
| 12 | 19.640 ° | 773.791 | 89.5% |
| 13 | 20.137 ° | 224.539 | 12.6% |
| 14 | 121.100 ° | 239.25 | 15.7% |
| 15 | 21.244 ° | 236.479 | 15.5% |
| 16 | 21.723 ° | 156.74 | 5.1% |
| 17 | 22.503 ° | 232.72 | 17.0% |
| 18 | 22.876 ° | 1234.013 | 17.8% |
| 19 | 24.673 ° | 317.436 | 28.4% |
| 20 | 125.066 ° | 1326.442 | 29.3% |
| 21 | 26.225 ° | 243.521 | 17.5% |
| 22 | 27.442 ° | 267.3 | 22.0% |
| 23 | 28.980 ° | 122.264 | 3.4% |
| 24 | 129.169 ° | 151.168 | 7.7% |
| 25 | 131.556 ° | 277.499 | 24.5% |
| 26 | 35.036 ° | 120.866 | 3.1% |
| 27 | 37.471 ° | 1146.855 | 7.2% |

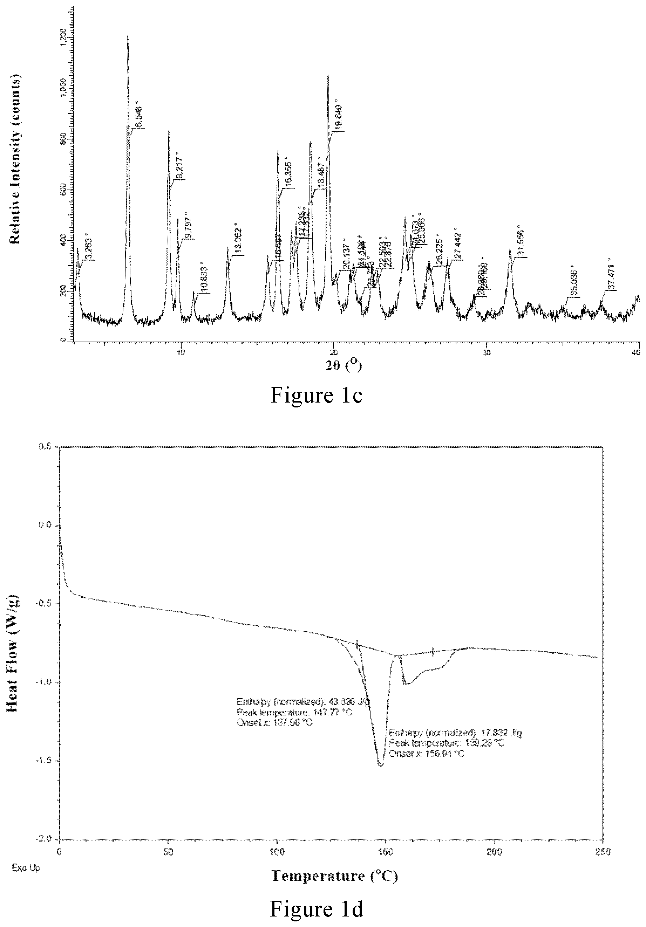

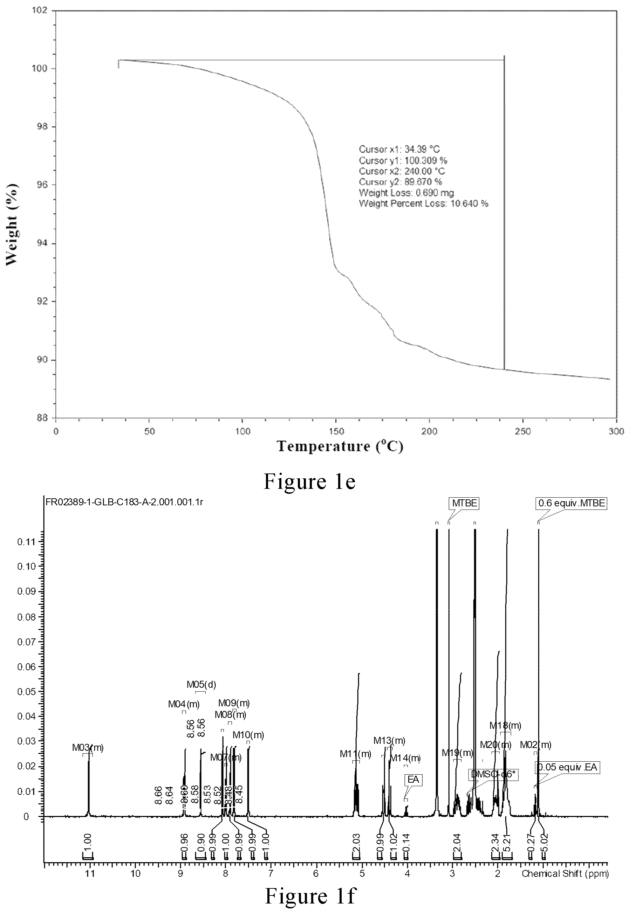

In some preferred embodiments, the form has an XRPD pattern substantially as shown in Figure 1c, and optionally has one or more of the following features:
1) having an endothermic peak at 147.77 °C ± 2 °C and an endothermic peak at 159.25 °C ± 2 °C in DSC pattern;
2) having a weight loss of 10.64 ± 0.2 wt% before reaching 240 °C in TGA pattern;
3) having a DSC pattern substantially as shown in Figure 1d; and/or
4) having a TGA pattern substantially as shown in Figure 1e.

### Solvate crystalline form A2 of the compound of formula A

In one embodiment, the form is a solvate crystalline form A2 of the compound of formula A, which has at least three, at least four, at least five, at least six or seven characteristic peaks at the following positions in an X-ray powder diffraction (XRPD) pattern in 2θ angle: 18.57 ± 0.2°, 19.67 ± 0.2°, 16.38 ± 0.2°, 9.28 ± 0.2°, 17.38 ± 0.2°, 25.18 ± 0.2° and 13.11 ± 0.2°.

In some preferred embodiments, the form has XRPD characteristic peaks at positions substantially as shown in Table 2 below:

**Table 2**

| Index | Angle | Intensity | Rel. Intensity |
|---|---|---|---|
| 1 | 3.285 ° | 324.214 | 11.5% |
| 2 | 6.564 ° | 583.794 | 30.5% |
| 3 | 9.287 ° | 923.068 | 50.1% |
| 4 | 9.828 ° | 407.678 | 19.5% |
| 5 | 10.876 ° | 308.096 | 13.6% |
| 6 | 13.118 ° | 624.252 | 32.2% |
| 7 | 15.723 ° | 449.546 | 21.9% |
| 8 | 16.383 ° | 963.664 | 51.9% |
| 9 | 17.387 ° | 789.735 | 41.1% |
| 10 | 17.694 ° | 429.127 | 19.5% |
| 11 | 18.069 ° | 141.442 | 2.3% |
| 12 | 18.579 ° | 1794.86 | 100.0% |
| 13 | 19.267 ° | 225.12 | 7.1% |
| 14 | 19.676 ° | 1262.25 | 68.6% |
| 15 | 20.122 ° | 473.809 | 22.2% |
| 16 | 20.886 ° | 116.081 | 1.3% |
| 17 | 21.179 ° | 547.507 | 26.9% |
| 18 | 21.801 ° | 233.406 | 8.5% |
| 19 | 22.632 ° | 559.87 | 28.1% |
| 20 | 22.987 ° | 194.161 | 6.4% |
| 21 | 23.962 ° | 164.277 | 4.4% |
| 22 | 24.610 ° | 389.116 | 17.4% |
| 23 | 24.757 ° | 470.533 | 22.1% |
| 24 | 25.185 ° | 652.793 | 32.9% |
| 25 | 26.060 ° | 296.637 | 11.8% |
| 26 | 26.358 ° | 329.123 | 13.7% |
| 27 | 26.608 ° | 220.216 | 7.3% |
| 28 | 27.299 ° | 159.418 | 4.0% |
| 29 | 27.619 ° | 361.905 | 16.2% |
| 30 | 28.016 ° | 153.524 | 4.2% |
| 31 | 28.998 ° | 99.3357 | 12% |
| 32 | 29.351 ° | 274.275 | 11.5% |
| 33 | 30.301 ° | 121.754 | 2.3% |
| 34 | 30.834 ° | 106.824 | 1.2% |
| 35 | 31.666 ° | 552.308 | 27.2% |
| 36 | 31.988 ° | 164.339 | 4.2% |
| 37 | 32.722 ° | 162.666 | 4.1% |
| 38 | 32.933 ° | 192.538 | 5.9% |
| 39 | 33.166 ° | 157.172 | 3.9% |
| 40 | 34.270 ° | 111.997 | 1.4% |
| 41 | 35.096 ° | 181.867 | 5.6% |
| 42 | 36.542 ° | 197.133 | 6.7% |
| 43 | 36.563 ° | 157.907 | 4.4% |
| 44 | 36.999 ° | 122.046 | 21% |
| 45 | 37.543 ° | 189.35 | 6.2% |
| 46 | 38.446 ° | 113.449 | 1.6% |
| 47 | 39.904 ° | 244.172 | 3.2% |

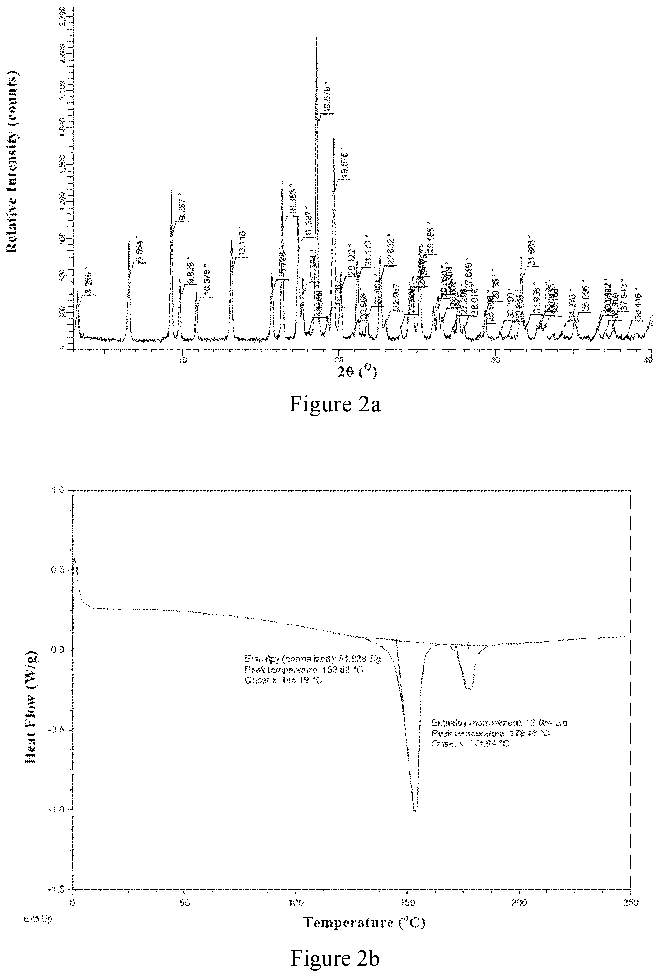

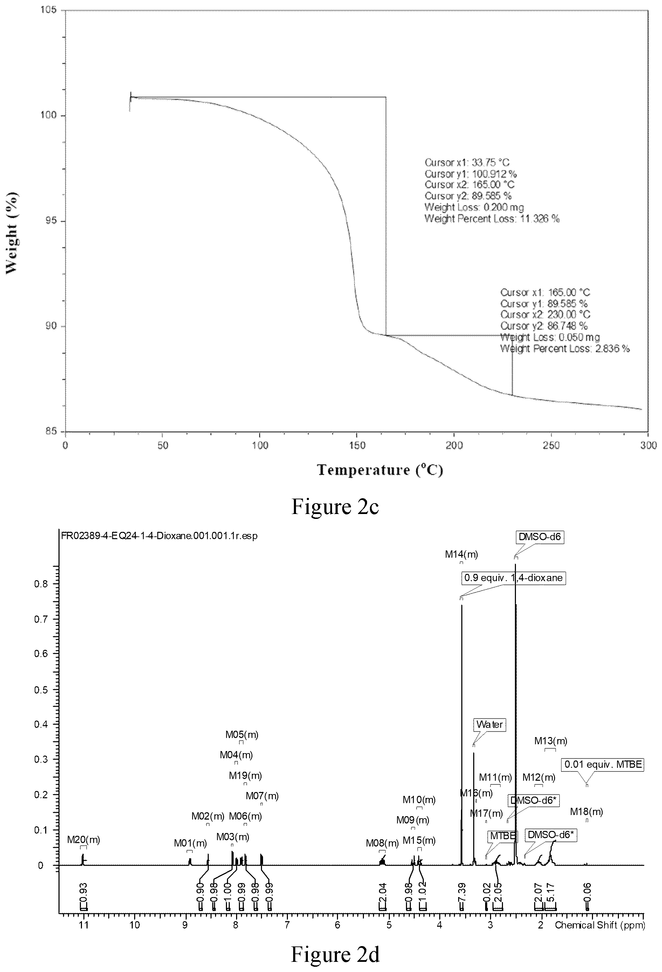

In some preferred embodiments, the form has an XRPD pattern substantially as shown in Figure 2a, and optionally has one or more of the following features:
1) having an endothermic peak at 153.88 °C ± 2 °C and an endothermic peak at 178.46 °C ± 2 °C in DSC pattern;
2) having a weight loss of 11.32 ± 0.2 wt% before reaching 165.00 °C, and a weight loss of 2.83 ± 0.2 wt% between 165.00°C and 230.00°C in TGA pattern;
3) having a DSC pattern substantially as shown in Figure 2b; and/or
4) having a TGA pattern substantially as shown in Figure 2c.

### Solvate crystalline form A3 of the compound of formula A

In one embodiment, the form is a solvate crystalline form A3 of the compound of formula A, which has at least three, at least four, at least five, at least six or seven characteristic peaks at the following positions in an X-ray powder diffraction (XRPD) pattern in 2θ angle: 19.61 ± 0.2°, 18.45 ± 0.2° 9.22 ± 0.2°, 16.33 ± 0.2°, 6.54 ± 0.2°, 17.24 ± 0.2° and 9.80 ± 0.2°.

In some preferred embodiments, the form has XRPD characteristic peaks at positions substantially as shown in Table 3 below:

**Table 3**

| Index | Angle | Intensity | Rel. Intensity |
|---|---|---|---|
| 1 | 3.273 ° | 216.139 | 10.6% |
| 2 | 6.546 ° | 669.056 | 64.9% |
| 3 | 9.220 ° | 843.144 | 82.9% |
| 4 | 9.802 ° | 398.84 | 35.3% |
| 5 | 10.813 ° | 329.24 | 27.8% |
| 6 | 12.570 ° | 85.9328 | 1.9% |
| 7 | 13.051 ° | 372.198 | 32.4% |
| 8 | 15.651 ° | 318.097 | 26.6% |
| 9 | 16.332 ° | 761.577 | 74.2% |
| 10 | 17.243 ° | 405.083 | 35.8% |
| 11 | 17.550 ° | 293.501 | 23.4% |
| 12 | 18.458 ° | 998.066 | 98.1% |
| 13 | 18.942 ° | 121.497 | 3.9% |
| 14 | 19.612 ° | 1018.29 | 100.0% |
| 15 | 19.853 ° | 181.844 | 10.4% |
| 16 | 20.193 ° | 281.808 | 21.3% |
| 17 | 20.437 ° | 114.124 | 3.5% |
| 18 | 20.849 ° | 122.084 | 4.8% |
| 19 | 21.343 ° | 162.29 | 9.3% |
| 20 | 21.668 ° | 124.884 | 5.3% |
| 21 | 22.248 ° | 195.438 | 13.0% |
| 22 | 22.913 ° | 210.459 | 14.9% |
| 23 | 24.042 ° | 113.894 | 4.2% |
| 24 | 24.580 ° | 242.769 | 17.5% |
| 25 | 24.915 ° | 373.519 | 31.3% |
| 26 | 25.706 ° | 140.247 | 6.2% |
| 27 | 26.170 ° | 335.118 | 27.2% |
| 28 | 27.086 ° | 232.238 | 16.7% |
| 29 | 27.416 ° | 145.74 | 7.5% |
| 30 | 27.793 ° | 159.028 | 9.3% |
| 31 | 28.084 ° | 107.172 | 4.0% |
| 32 | 28.787 ° | 91.7793 | 2.9% |
| 33 | 29.137 ° | 166.444 | 10.8% |
| 34 | 29.675 ° | 95.3432 | 3.1% |
| 35 | 30.950 ° | 107.785 | 4.4% |
| 36 | 31.458 ° | 235.675 | 17.7% |
| 37 | 31.912 ° | 105.23 | 3.6% |
| 38 | 32.703 ° | 154.353 | 8.6% |
| 39 | 33.612 ° | 118.412 | 5.0% |
| 40 | 34.507 ° | 104.555 | 3.7% |
| 41 | 35.063 ° | 106.2 | 3.9% |
| 42 | 36.285 ° | 111.087 | 4.5% |
| 43 | 37.404 ° | 144.34 | 7.6% |
| 44 | 39.706 ° | 129.602 | 6.0% |

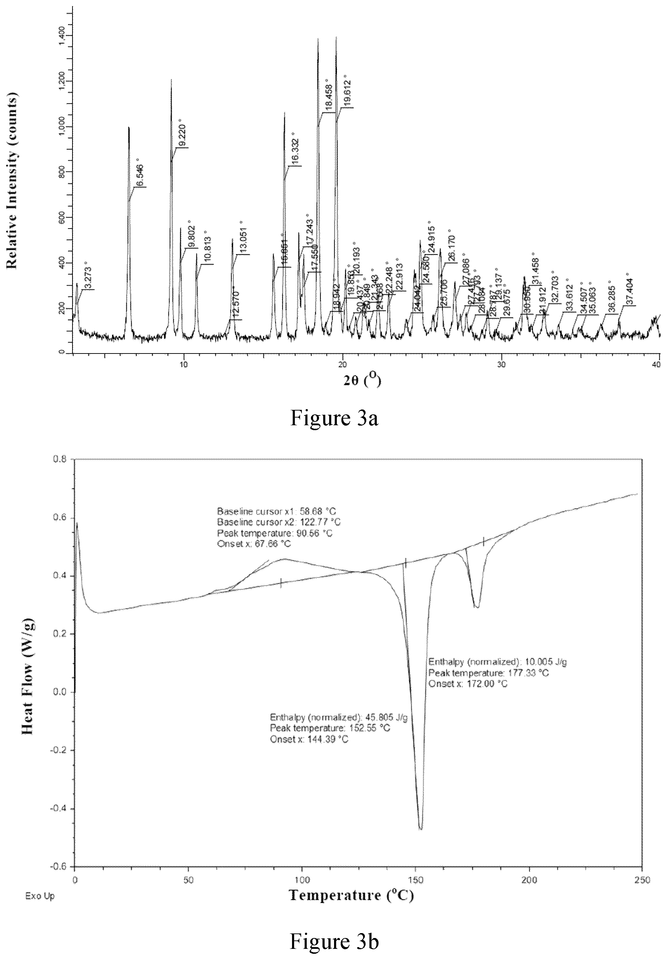

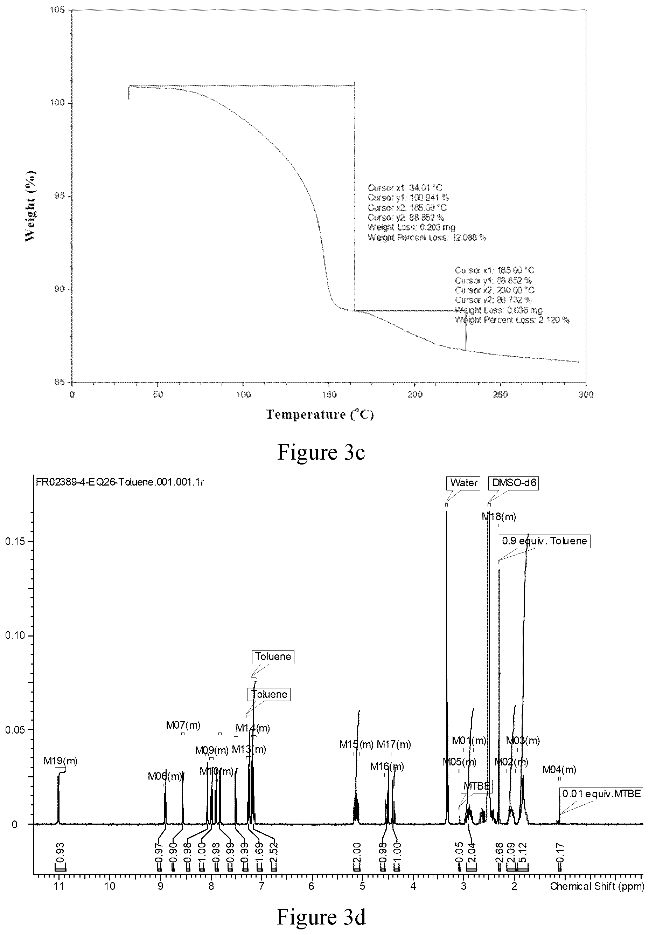

In some preferred embodiments, the form has an XRPD pattern substantially as shown in Figure 3a, and optionally has one or more of the following features:
1) having an exothermic peak at 90.56°C ± 2 °C, and an endothermic peak at 152.55 °C ± 2°C and an endothermic peak at 177.33 °C ± 2 °C respectively in DSC pattern;
2) having a weight loss of 12.08 ± 0.2 wt% before reaching 165.00 °C, and a weight loss of 2.12 ± 0.2 wt% between 165.00 °C and 230.00 °C in TGA pattern;
3) having a DSC pattern substantially as shown in Figure 3b; and/or
4) having a TGA pattern substantially as shown in Figure 3c.

### Solvate crystalline form A4 of the compound of formula A

In one embodiment, the form is a solvate crystalline form A4 of the compound of formula A, which has at least three, at least four, at least five, at least six or seven characteristic peaks at the following positions in an X-ray powder diffraction (XRPD) pattern in 2θ angle: 9.22 ± 0.2°, 17.51 ± 0.2°, 6.54 ± 0.2°, 19.59 ± 0.2°, 25.03 ± 0.2°, 16.37 ± 0.2° and 18.51 ± 0.2°.

In some preferred embodiments, the form has XRPD characteristic peaks at positions substantially as shown in Table 4 below:

**Table 4**

| Index | Angle | Intensity | Rel. Intensity |
|---|---|---|---|
| 1 | 3.275 ° | 74.8549 | 25.9% |
| 2 | 6.548 ° | 147.037 | 81.2% |
| 3 | 9.225 ° | 192.479 | 100.0% |
| 4 | 9.809 ° | 118.394 | 28.6% |
| 5 | 16.379 ° | 192.229 | 61.7% |
| 6 | 17.510 ° | 1239.22 | 94.5% |
| 7 | 18.514 ° | 206.525 | 60.5% |
| 8 | 19.059 ° | 163.093 | 20.3% |
| 9 | 19.591 ° | 227.215 | 77.0% |
| 10 | 21.170 ° | 1167.727 | 27.4% |
| 11 | 22.486 ° | 167.613 | 33.3% |
| 12 | 24.641 ° | 184.115 | 51.7% |
| 13 | 25.036 ° | 202.345 | 68.1% |
| 14 | 31.539 ° | 128.091 | 36.4% |

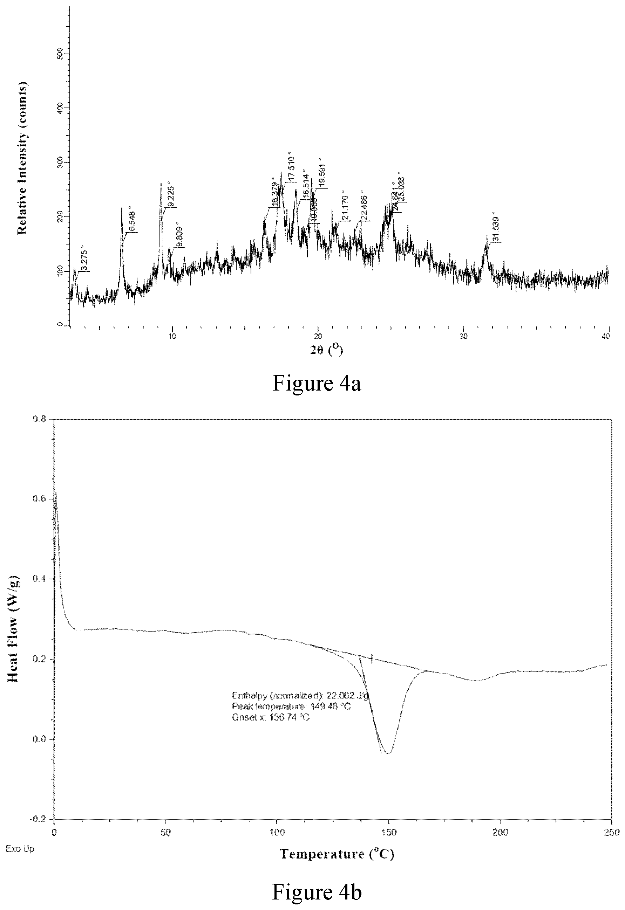

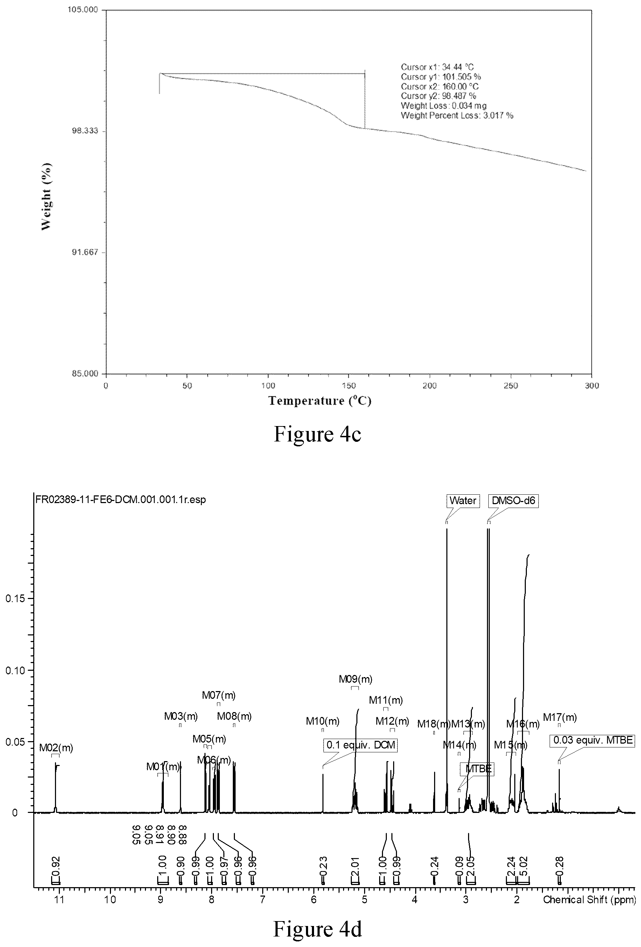

In some preferred embodiments, the form has an XRPD pattern substantially as shown in Figure 4a, and optionally has one or more of the following features:
1) having an endothermic peak at 149.48 °C ± 2 °C in DSC pattern;
2) having a weight loss of 3.01 ± 0.2 wt% before reaching 160 °C in TGA pattern;
3) having a DSC pattern substantially as shown in Figure 4b; and/or
4) having a TGA pattern substantially as shown in Figure 4c.

### Solvate crystalline form A5 of the compound of formula A

In one embodiment, the form is a solvate crystalline form A5 of the compound of formula A, which has at least three, at least four, at least five, at least six or seven characteristic peaks at the following positions in an X-ray powder diffraction (XRPD) pattern in 2θ angle: 16.43 ± 0.2°, 19.74 ± 0.2°, 6.58 ± 0.2°, 9.27 ± 0.2°, 18.53 ± 0.2°, 17.37 ± 0.2° and 9.88 ± 0.2°.

In some preferred embodiments, the form has XRPD characteristic peaks at positions substantially as shown in Table 5 below:

**Table 5**

| Index | Angle | Intensity | Rel. Intensity |
|---|---|---|---|
| 1 | 6.589 ° | 289.596 | 185.7% |
| 2 | 8.030 ° | 1127.435 | 118.2% |
| 3 | 9.272 ° | 297.6 | 79.9% |
| 4 | 9.889 ° | 199.759 | 40.9% |
| 5 | 13.139 ° | 153.748 | 18.0% |
| 6 | 14.22 ° | 128.837 | 10.5% |
| 7 | 15.804 ° | 182.386 | 26.3% |
| 8 | 16.430 ° | 385.534 | 100.0% |
| 9 | 17.370 ° | 1262.044 | 45.8% |
| 10 | 17.590 ° | 299.516 | 59.1% |
| 11 | 18.531 ° | 354.582 | 76.1% |
| 12 | 19.744 ° | 424.384 | 100.0% |
| 13 | 23.062 ° | 222.304 | 26.7% |
| 14 | 24.719 ° | 217.249 | 22.8% |
| 15 | 25.119 ° | 206.485 | 18.0% |
| 16 | 27.532 ° | 192.727 | 15.0% |
| 17 | 31.605 ° | 184.447 | 24.5% |

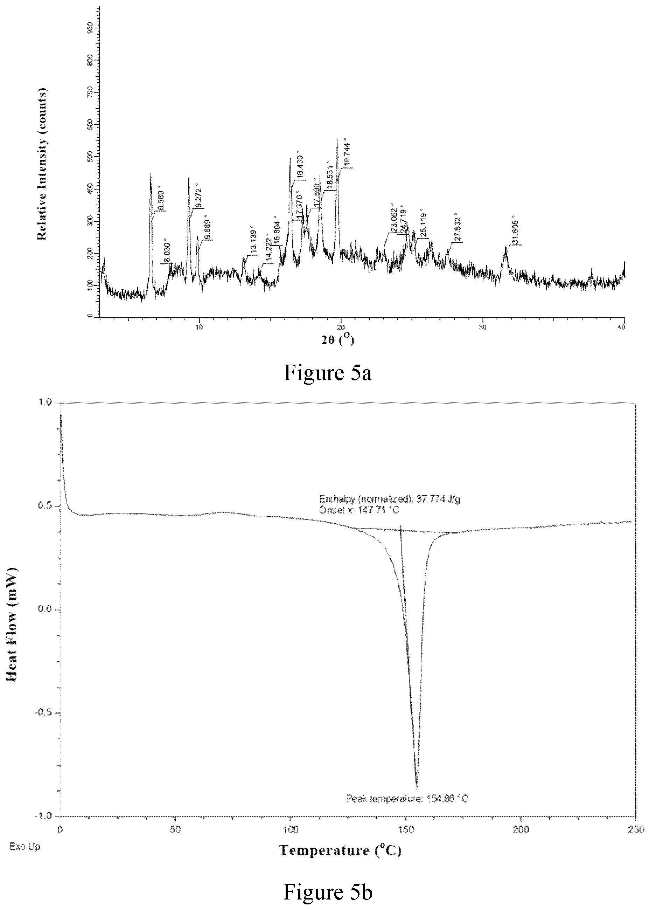

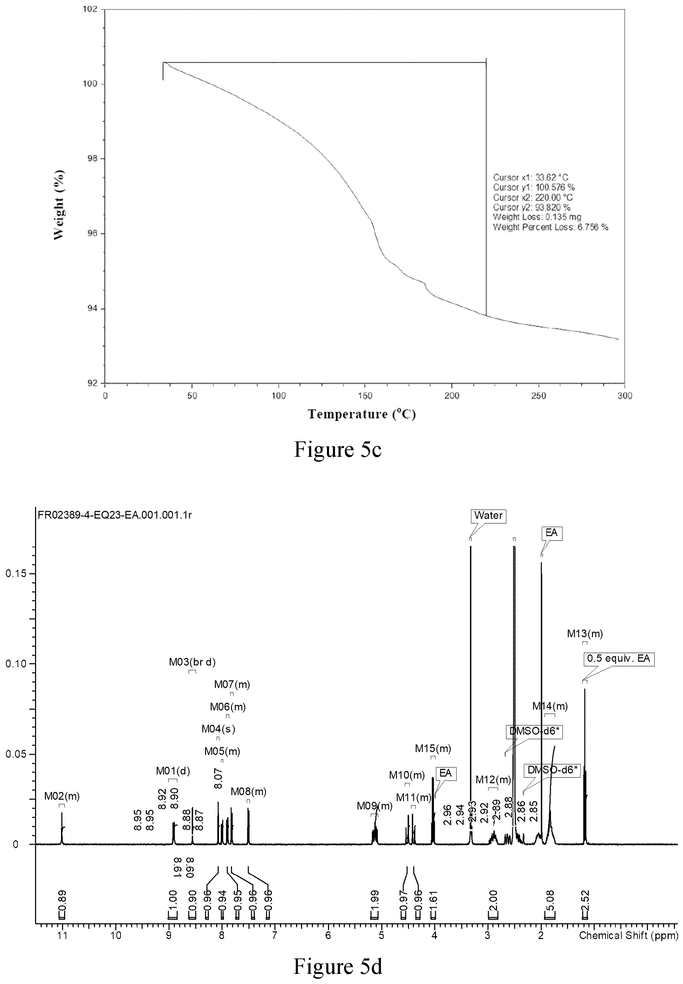

In some preferred embodiments, the form has an XRPD pattern substantially as shown in Figure 5a, and optionally has one or more of the following features:
1) having an endothermic peak at 154.86 °C ± 2 °C in DSC pattern;
2) having a weight loss of 6.75 ± 0.2 wt% before reaching 220 °C in TGA pattern;
3) having a DSC pattern substantially as shown in Figure 5b; and/or
4) having a TGA pattern substantially as shown in Figure 5c.

### Crystalline form B of the compound of formula A

In one embodiment, the form is a crystalline form B of the compound of formula A, which has at least three, at least four, at least five, at least six or seven characteristic peaks at the following positions in an X-ray powder diffraction (XRPD) pattern in 2θ angle: 3.98 ± 0.2°, 12.35 ± 0.2°, 12.05 ± 0.2°, 19.09 ± 0.2°, 7.92 ± 0.2°, 15.78 ± 0.2° and 14.32 ± 0.2°.

In some preferred embodiments, the form has XRPD characteristic peaks at positions substantially as shown in Table 6 below:

**Table 6**

| Index | Angle | Intensity | Rel. Intensity |
|---|---|---|---|
| 1 | 3.980 ° | 738.592 | 100.0% |
| 2 | 5.255 ° | 204.407 | 19.7% |
| 3 | 6.868 ° | 251.308 | 27.8% |
| 4 | 7.193 ° | 122.394 | 7.4% |
| 5 | 7.926 ° | 523.18 | 69.3% |
| 6 | 8.623 ° | 495.923 | 65.2% |
| 7 | 9.111 ° | 274.148 | 31.2% |
| 8 | 10.518 ° | 328.353 | 38.0% |
| 9 | 11.108 ° | 377.686 | 44.8% |
| 10 | 12.057 ° | 697.594 | 93.8% |
| 11 | 12.357 ° | 716.292 | 96.6% |
| 12 | 13.057 ° | 244.358 | 23.6% |
| 13 | 13.732 ° | 257.976 | 26.1% |
| 14 | 14.320 ° | 512.673 | 66.2% |
| 15 | 15.069 ° | 188.075 | 16.6% |
| 16 | 15.780 ° | 509.646 | 66.7% |
| 17 | 16.196 ° | 199.737 | 18.9% |
| 18 | 17.032 ° | 121.533 | 6.8% |
| 19 | 17.317 ° | 218.754 | 21.5% |
| 20 | 18.259 ° | 393.42 | 48.0% |
| 21 | 19.099 ° | 549.835 | 72.5% |
| 22 | 19.622 ° | 476.19 | 61.6% |
| 23 | 19.796 ° | 397.212 | 49.6% |
| 24 | 20.606 ° | 112.485 | 6.5% |
| 25 | 21.023 ° | 182.181 | 17.7% |
| 26 | 21.197 ° | 143.221 | 11.8% |
| 27 | 21.681 ° | 91.3707 | 3.9% |
| 28 | 22.381 ° | 194.785 | 19.5% |
| 29 | 22.611 ° | 220.275 | 23.3% |
| 30 | 23.525 ° | 141.68 | 11.1% |
| 31 | 24.238 ° | 130.074 | 9.9% |
| 32 | 24.824 ° | 107.332 | 6.7% |
| 33 | 25.026 ° | 145.034 | 12.5% |
| 34 | 25.933 ° | 185.825 | 19.0% |
| 35 | 26.392 ° | 98.6315 | 5.6% |
| 36 | 26.754 ° | 122.012 | 9.6% |
| 37 | 27.965 ° | 100.238 | 6.6% |
| 38 | 28.719 ° | 109.597 | 7.9% |
| 39 | 30.720 ° | 85.0549 | 3.5% |
| 40 | 31.228 ° | 137.216 | 11.3% |
| 41 | 31.551 ° | 91.5736 | 4.1% |
| 42 | 32.132 ° | 93.4716 | 4.6% |
| 43 | 33.654 ° | 73.8956 | 2.9% |
| 44 | 34.217 ° | 75.3033 | 3.5% |

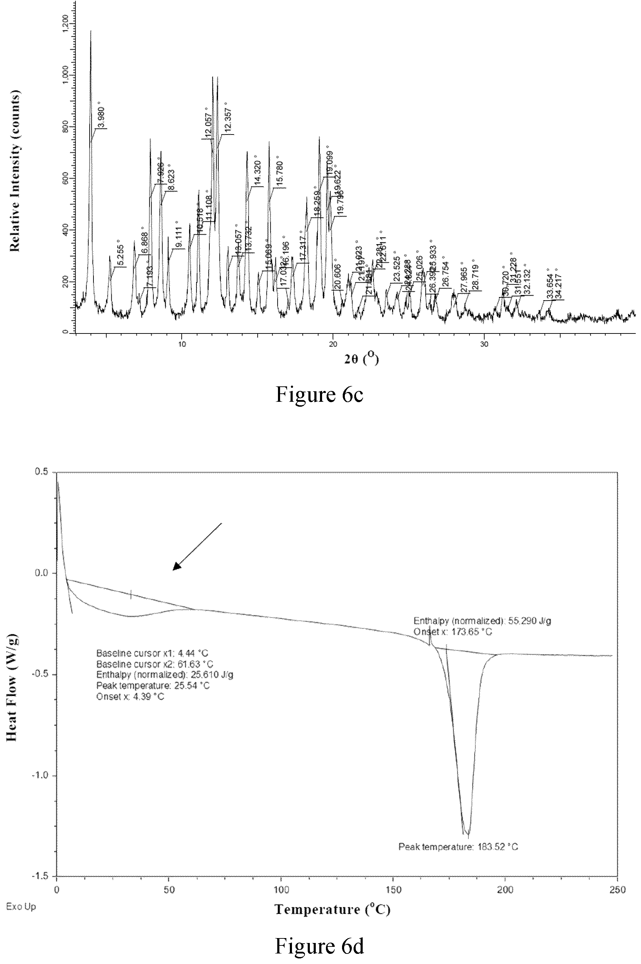

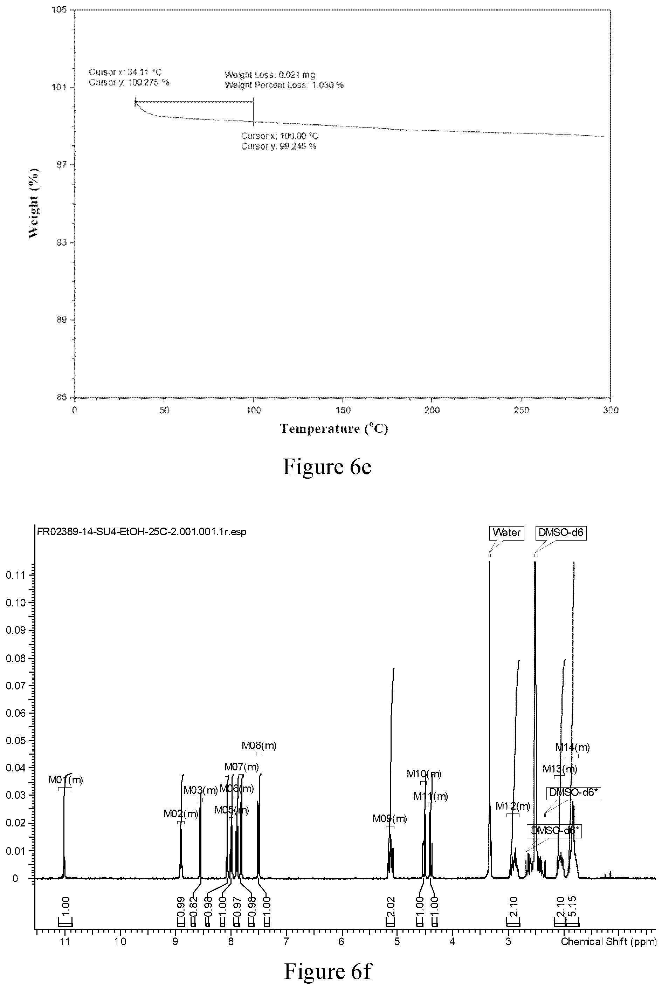

In some preferred embodiments, the form has an XRPD pattern substantially as shown in Figure 6c, and optionally has one or more of the following features:
1) having an endothermic peak at 25.54 °C ± 2 °C and an endothermic peak at 183.52 °C ± 2 °C in DSC pattern;
2) having a weight loss of 1.03 ± 0.2 wt% before reaching 100 °C in TGA pattern;
3) having a DSC pattern substantially as shown in Figure 6d; and/or
4) having a TGA pattern substantially as shown in Figure 6e.

### Solvate crystalline form C of the compound of formula A

In one embodiment, the form is a solvate crystalline form C of the compound of formula A, which has at least three, at least four, at least five, at least six or seven characteristic peaks at the following positions in an X-ray powder diffraction (XRPD) pattern in 2θ angle: 20.60 ± 0.2°, 17.94 ± 0.2°, 13.110 ± 0.2°, 19.42 ± 0.2°, 23.97 ± 0.2°, 26.31 ± 0.2° and 11.95 ± 0.2°.

In some preferred embodiments, the form has XRPD characteristic peaks at positions substantially as shown in Table 7 below:

**Table 7**

| Index | Angle | Intensity | Rel. Intensity |
|---|---|---|---|
| 1 | 6.003 ° | 137.809 | 3.6% |
| 2 | 8.975 ° | 193.481 | 6.2% |
| 3 | 9.183 ° | 107.904 | 2.3% |
| 4 | 9.621 ° | 118.055 | 2.7% |
| 5 | 10.354 ° | 95.7835 | 1.6% |
| 6 | 11.104 ° | 966.108 | 41.7% |
| 7 | 11.953 ° | 716.162 | 30.1% |
| 8 | 13.110 ° | 1198.11 | 52.4% |
| 9 | 14.357 ° | 113.888 | 2.8% |
| 10 | 15.440 ° | 171.301 | 5.7% |
| 11 | 16.811 ° | 82.4975 | 1.5% |
| 12 | 17.941 ° | 1501.19 | 66.2% |
| 13 | 18.306 ° | 582.257 | 23.5% |
| 14 | 19.244 ° | 540.03 | 21.2% |
| 15 | 19.426 ° | 1035.74 | 44.1% |
| 16 | 19.992 ° | 106.812 | 1.2% |
| 17 | 20.606 ° | 2247.29 | 100.0% |
| 18 | 21.174 ° | 100.313 | 1.0% |
| 19 | 21.545 ° | 123.426 | 2.3% |
| 20 | 21.805 ° | 95.3241 | 1.1% |
| 21 | 22.157 ° | 155.752 | 4.0% |
| 22 | 22.867 ° | 104.101 | 1.7% |
| 23 | 23.408 ° | 219.072 | 6.8% |
| 24 | 23.970 ° | 987.271 | 42.2% |
| 25 | 24.248 ° | 99.374 | 1.2% |
| 26 | 24.954 ° | 212.46 | 6.5% |
| 27 | 25.357 ° | 152.189 | 3.6% |
| 28 | 26.314 ° | 933.192 | 39.3% |
| 29 | 27.023 ° | 533.026 | 20.6% |
| 30 | 27.788 ° | 173.402 | 4.2% |
| 31 | 27.971 ° | 257.6 | 8.1% |
| 32 | 28.554 ° | 157.91 | 3.8% |
| 33 | 29.622 ° | 98.9549 | 1.2% |
| 34 | 30.189 ° | 107.021 | 1.6% |
| 35 | 30.729 ° | 204.806 | 6.1% |
| 36 | 31.115 ° | 141.78 | 3.2% |
| 37 | 31.740 ° | 114.936 | 2.0% |
| 38 | 32.134 ° | 85.671 | 0.8% |
| 39 | 33.138 ° | 149.303 | 3.5% |
| 40 | 33.614 ° | 94.5579 | 0.9% |
| 41 | 35.065 ° | 146.555 | 3.1% |
| 42 | 36.311 ° | 107.151 | 1.3% |
| 43 | 36.681 ° | 184.799 | 5.0% |
| 44 | 37.605 ° | 140.555 | 3.1% |
| 45 | 38.111 ° | 136.83 | 2.7% |
| 46 | 39.059 ° | 162.956 | 3.8% |
| 47 | 39.825 ° | 119.636 | 1.2% |

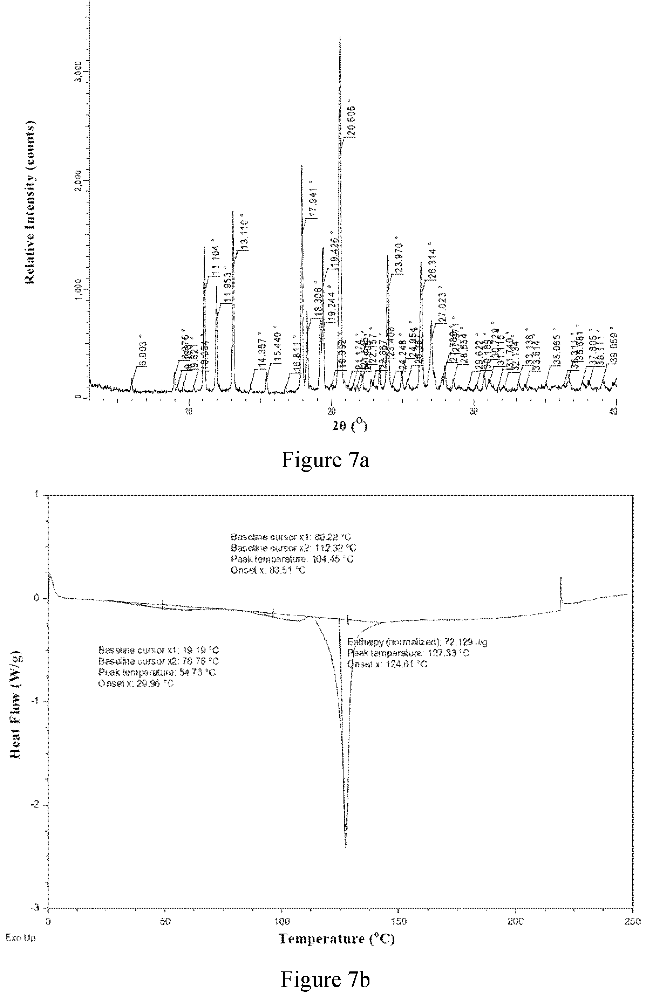

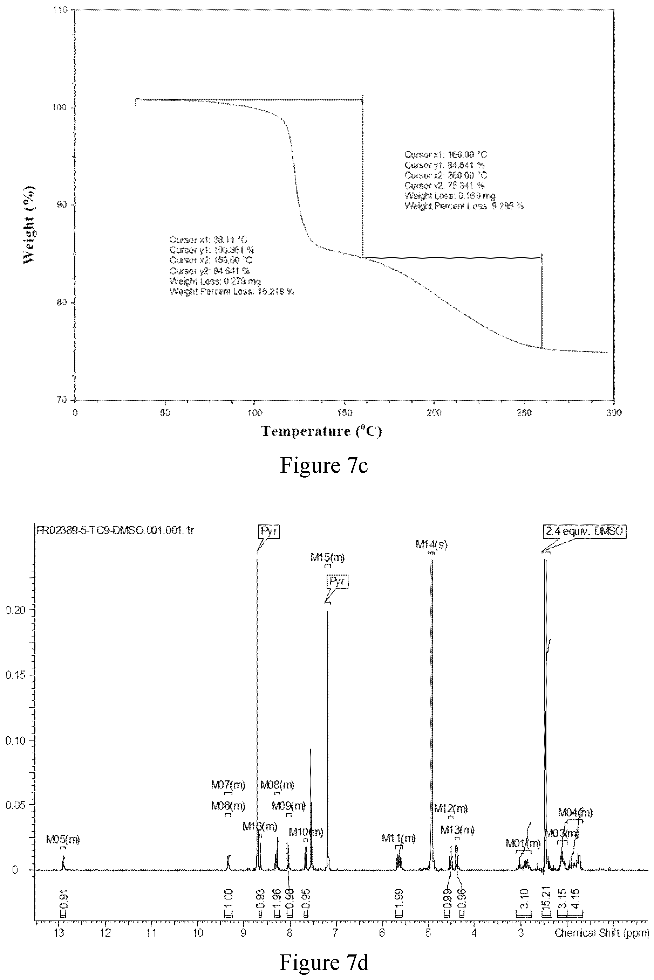

In some preferred embodiments, the form has an XRPD pattern substantially as shown in Figure 7a, and optionally has one or more of the following features:
1) having an endothermic peak at 127.33 °C ± 2 °C in DSC pattern;
2) having a weight loss of 16.21 ± 0.2 wt% before reaching 160.00 °C, and a weight loss of 9.29 ± 0.2 wt% between 160.00 °C and 260.00 °C in TGA pattern;
3) having a DSC pattern substantially as shown in Figure 7b; and/or
4) having a TGA pattern substantially as shown in Figure 7c.

### Solvate crystalline form D1 of the compound of formula A

In one embodiment, the form is a solvate crystalline form D1 of the compound of formula A, which has at least three, at least four, at least five, at least six or seven characteristic peaks at the following positions in an X-ray powder diffraction (XRPD) pattern in 2θ angles: 17.99 ± 0.2°, 8.82 ± 0.2°, 17.32 ± 0.2°, 9.26 ± 0.2°, 19.14 ± 0.2°, 9.95 ± 0.2° and 31.53 ± 0.2°.

In some preferred embodiments, the form has XRPD characteristic peaks at positions substantially as shown in Table 8 below:

**Table 8**

| Index | Angle | Intensity | Rel.Intensity |
|---|---|---|---|
| 1 | 3.283 ° | 157.732 | 1.9% |
| 2 | 6.572 ° | 387.739 | 9.6% |
| 3 | 8.827 ° | 3337.38 | 96.5% |
| 4 | 9.263 ° | 1379.71 | 38.5% |
| 5 | 9.955 ° | 1092.28 | 30.0% |
| 6 | 10.859 ° | 215.017 | 4.1% |
| 7 | 14.358 ° | 631.665 | 16.5% |
| 8 | 15.693 ° | 193.573 | 3.5% |
| 9 | 16.374 ° | 283.937 | 5.9% |
| 10 | 17.324 ° | 2899.12 | 82.8% |
| 11 | 17.612 ° | 656.795 | 16.3% |
| 12 | 17.991 ° | 3485.93 | 100.0% |
| 13 | 18.507 ° | 743.752 | 18.8% |
| 14 | 19.149 ° | 1315.22 | 35.8% |
| 15 | 19.645 ° | 386619 | 8.6% |
| 16 | 20.585 ° | 157.054 | 2.3% |
| 17 | 21.177 ° | 202.178 | 3.7% |
| 18 | 21.859 ° | 210.223 | 4.0% |
| 19 | 22.659 ° | 155.342 | 2.4% |
| 20 | 22.976 ° | 126.824 | 1.5% |
| 21 | 23.530 ° | 102.439 | 0.6% |
| 22 | 23.857 ° | 102.269 | 0.6% |
| 23 | 24.653 ° | 270.25 | 5.2% |
| 24 | 24.876 ° | 369.714 | 8.1% |
| 25 | 25.138 ° | 402.791 | 9.0% |
| 26 | 25.523 ° | 304.884 | 6.0% |
| 27 | 26.092 ° | 491.584 | 11.5% |
| 28 | 26.519 ° | 577.703 | 14.1% |
| 29 | 27.868 ° | 292.978 | 6.0% |
| 30 | 28.503 ° | 190.294 | 3.0% |
| 31 | 29.214 ° | 557.815 | 13.8% |
| 32 | 30.023 ° | 162.067 | 2.0% |
| 33 | 30.217 ° | 158.007 | 1.8% |
| 34 | 31.537 ° | 718.526 | 18.2% |
| 35 | 31.824 ° | 292.53 | 5.5% |
| 36 | 32.595 ° | 136.1 | 1.0% |
| 37 | 32.778 ° | 229.837 | 3.8% |
| 38 | 33.035 ° | 155.219 | 1.7% |
| 39 | 33.603 ° | 117.545 | 0.8% |
| 40 | 33.889 ° | 118.053 | 0.8% |
| 41 | 34.225 ° | 109.361 | 0.5% |
| 42 | 34.913 ° | 532.114 | 12.9% |
| 43 | 35.560 ° | 137.994 | 1.4% |
| 44 | 36.337 ° | 230.035 | 4.2% |
| 45 | 36.829 ° | 174.24 | 2.5% |
| 46 | 37.447 ° | 120.306 | 1.0% |
| 47 | 38.747 ° | 120.562 | 0.8% |
| 48 | 39.866 ° | 199.184 | 2.0% |

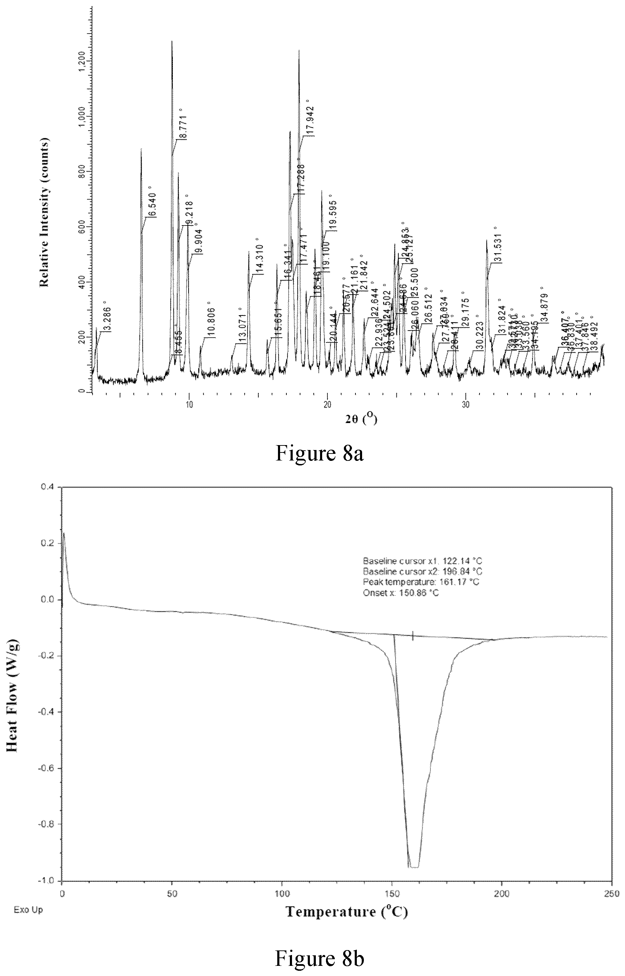

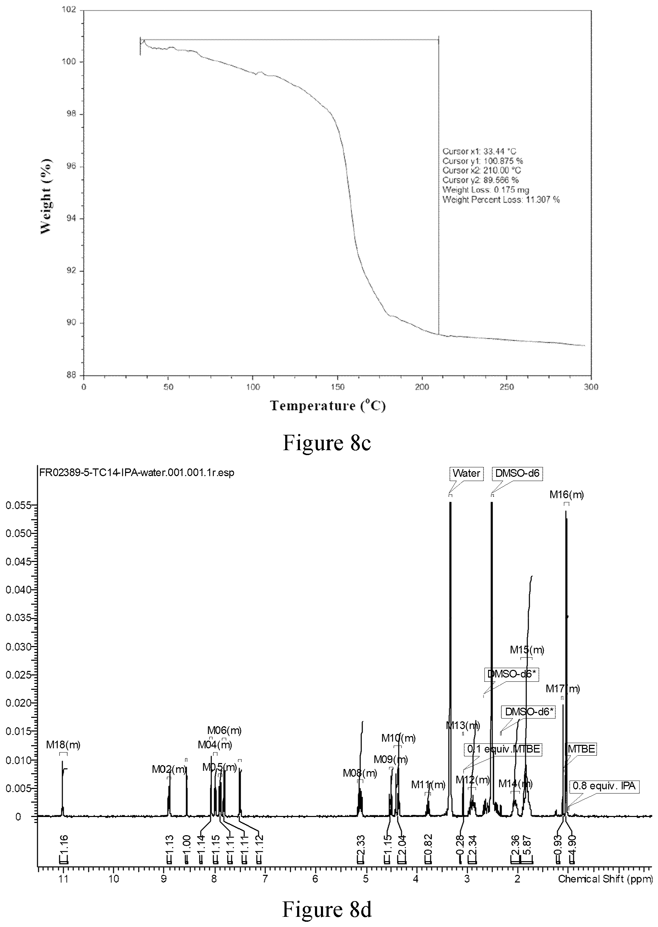

In some preferred embodiments, the form has an XRPD pattern substantially as shown in Figure 8a, and optionally has one or more of the following features:
1) having an endothermic peak at 161.17 °C ± 2 °C in a DSC pattern;
2) having a weight loss of 11.30 ± 0.2 wt% before reaching 210 °C in a TGA pattern;
3) having a DSC pattern substantially as shown in Figure 8b; and/or
4) having a TGA pattern substantially as shown in Figure 8c.

### Solvate crystalline form D2 of the compound of formula A

In one embodiment, the form is a solvate crystalline form D2 of the compound of formula A, which has at least three, at least four, at least five, at least six or seven characteristic peaks at the following positions in an X-ray powder diffraction (XRPD) pattern in 2θ angle: 17.38 ± 0.2°, 17.99 ± 0.2°, 19.57 ± 0.2°, 9.80 ± 0.2°, 31.55 ± 0.2°, 25.05 ± 0.2° and 6.55 ± 0.2°.

In some preferred embodiments, the form has XRPD characteristic peaks at positions substantially as shown in Table 9 below:

**Table 9**

| Index | Angle | Intensity | Rel. Intensity |
|---|---|---|---|
| 1 | 3.278 ° | 143.79 | 6.5% |
| 2 | 6.550 ° | 266.267 | 37.4% |
| 3 | 8.806 ° | 216.663 | 26.7% |
| 4 | 9.252 ° | 199.82 | 23.1% |
| 5 | 9.806 ° | 330.932 | 46.0% |
| 6 | 10.828 ° | 107.527 | 6.6% |
| 7 | 13.067 ° | 112.021 | 5.9% |
| 8 | 14.362 ° | 158.266 | 13.9% |
| 9 | 16.339 ° | 161.615 | 15.3% |
| 10 | 17.384 ° | 654.666 | 100.0% |
| 11 | 17.996 ° | 581.611 | 85.3% |
| 12 | 18.519 ° | 271.771 | 29.0% |
| 13 | 19.151 ° | 288.651 | 31.0% |
| 14 | 19.573 ° | 569.078 | 80.3% |
| 15 | 20.016 ° | 149.399 | 5.5% |
| 16 | 20.553 ° | 257.543 | 24.7% |
| 17 | 21.131 ° | 242.818 | 22.4% |
| 18 | 21.818 ° | 294.712 | 32.4% |
| 19 | 22.590 ° | 146.784 | 6.3% |
| 20 | 22.904 ° | 161.374 | 8.5% |
| 21 | 23.456 ° | 162.708 | 8.0% |
| 22 | 24.838 ° | 319.096 | 33.9% |
| 23 | 25.057 ° | 349.065 | 39.1% |
| 24 | 25.475 ° | 278.68 | 26.6% |
| 25 | 26.536 ° | 229.539 | 18.6% |
| 26 | 27.662 ° | 151.236 | 6.8% |
| 27 | 30.134 ° | 131.3 | 7.4% |
| 28 | 31.558 ° | 351.989 | 44.4% |
| 29 | 33.055 ° | 127.673 | 4.6% |
| 30 | 34.958 ° | 130.661 | 6.1% |
| 31 | 36.330 ° | 133.373 | 6.7% |
| 32 | 39.819 ° | 179.373 | 6.2% |

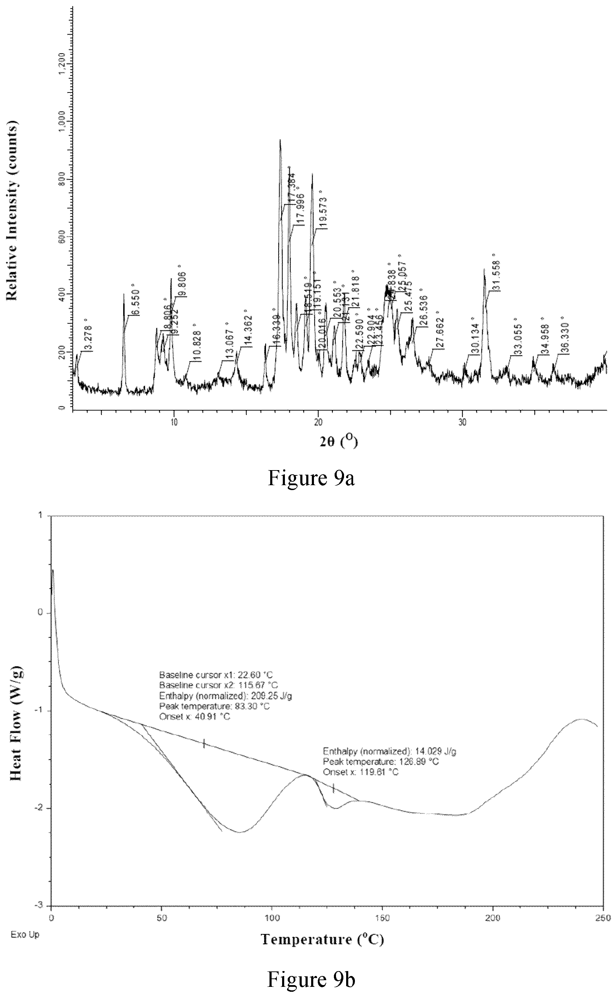

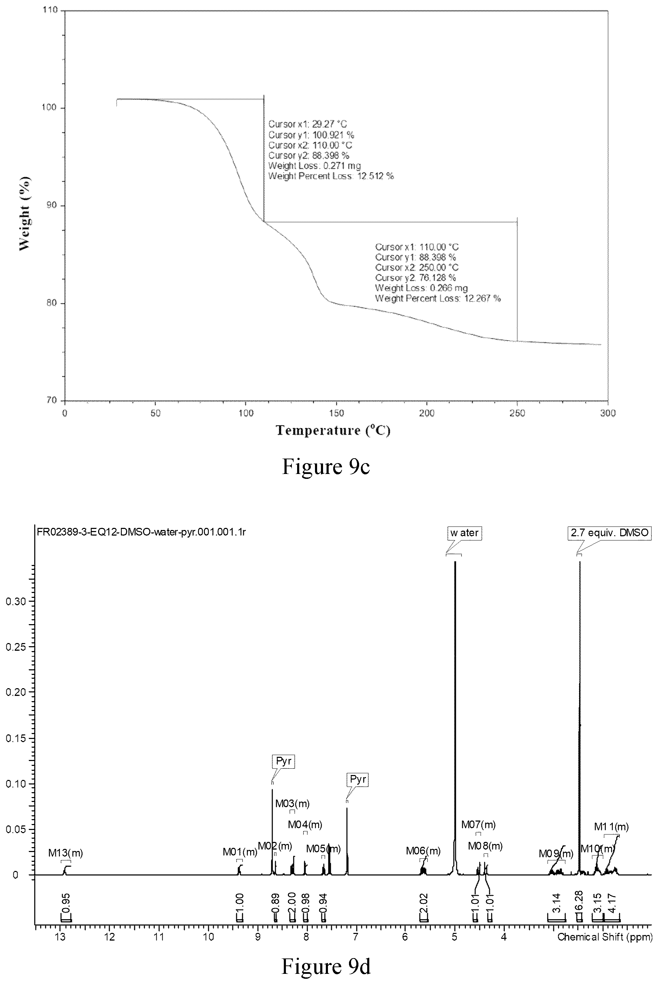

In some preferred embodiments, the form has an XRPD pattern substantially as shown in Figure 9a, and optionally has one or more of the following features:
1) having an endothermic peak at 83.30 °C ± 2 °C and an endothermic peak at 126.89 °C ± 2 °C in DSC pattern;
2) having a weight loss of 12.51 ± 0.2 wt% before reaching 110.00 °C, and a weight loss of 12.26 ± 0.2 wt% between 110.00 °C and 250.00 °C in TGA pattern;
3) having a DSC pattern substantially as shown in Figure 9b; and/or
4) having a TGA pattern substantially as shown in Figure 9c.

### Crystalline form E of the compound of formula A

In one embodiment, the form is a crystalline form E of the compound of formula A, which has at least three, at least four, at least five, at least six or seven characteristic peaks at the following positions in an X-ray powder diffraction (XRPD) pattern in 2θ angle: 11.14 ± 0.2°, 15.76 ± 0.2°, 12.59 ± 0.2°, 19.71 ± 0.2°, 9.56 ± 0.2°, 17.83 ± 0.2° and 13.58 ± 0.2°.

In some preferred embodiments, the form has XRPD characteristic peaks at positions substantially as shown in Table 10 below:

**Table 10**

| Index | Angle | Intensity | Rel. Intensity |
|---|---|---|---|
| 1 | 3.557 ° | 132.05 | 10.7% |
| 2 | 3.969 ° | 286.338 | 33.3% |
| 3 | 4.384 ° | 299.05 | 35.3% |
| 4 | 4.799 ° | 185.083 | 18.9% |
| 5 | 5.771 ° | 123.886 | **10.5%** |
| 6 | 7.083 ° | 182.887 | 17.9% |
| 7 | 7.438 ° | 217.182 | 226% |
| 8 | 7.880 ° | 272.83 | 30.5% |
| 9 | 8.330 ° | 148.933 | 12.2% |
| 10 | 9.230 ° | 125.26 | 8.3% |
| 11 | 9.560 ° | 562.461 | 72.2% |
| 12 | 9.826 ° | 305.942 | 34.2% |
| 13 | 10.027 ° | 128.033 | 7.9% |
| 14 | 10.625 ° | 128.671 | 7.2% |
| 15 | 11.147 ° | 764.67 | 100.0% |
| 16 | 11.556 ° | 184.006 | 14.4% |
| 17 | 11.745 ° | 155.886 | 10.1% |
| 18 | 12.591 ° | 666.843 | 84.6% |
| 19 | 13.073 ° | 307.389 | 31.2% |
| 20 | 13.588 ° | 492.041 | 58.0% |
| 21 | 13.954 ° | 136.617 | 5.6% |
| 22 | 14.317 ° | 463.243 | 53.6% |
| 23 | 14.654 ° | 170.71 | 10.8% |
| 24 | 14.863 ° | 304.966 | 30.6% |
| 25 | 15.473 ° | 131.41 | 5.1% |
| 26 | 15.767 ° | 760.056 | 96.9% |
| 27 | 16.333 ° | 184.467 | 11.5% |
| 28 | 16.738 ° | 189.624 | 11.8% |
| 29 | 16.798 ° | 221.934 | 16.5% |
| 30 | 17.444 ° | 376.044 | 38.0% |
| 31 | 17.837 ° | 538.492 | 61.0% |
| 32 | 18.477 ° | 217.582 | 12.8% |
| 33 | 18.858 ° | 353.273 | 32.3% |
| 34 | 19.139 ° | 547.761 | 60.6% |
| 35 | 19.373 ° | 227.318 | 13.4% |
| 36 | 19.710 ° | 677.001 | 79.3% |
| 37 | 20.102 ° | 363.719 | 33.3% |
| 38 | 20.322 ° | 248.625 | 16.4% |
| 39 | 20.631 ° | 250.842 | 16.9% |
| 40 | 20.973 ° | 225.826 | 13.5% |
| 41 | 21.280 ° | 259.955 | 18.9% |
| 42 | 21.624 ° | 193.339 | 9.6% |
| 43 | 21.702 ° | 206.541 | 11.7% |
| 44 | 22.065 ° | 279.338 | 23.1% |
| 45 | 22.353 ° | 268.052 | 22.1% |
| 46 | 22.771 ° | 147.429 | 4.6% |
| 47 | 23.126 ° | 361.751 | 35.7% |
| 48 | 23.653 ° | 216.875 | 14.1% |
| 49 | 24.068 ° | 229.154 | 15.9% |
| 50 | 24.449 ° | 195.75 | 11.1% |

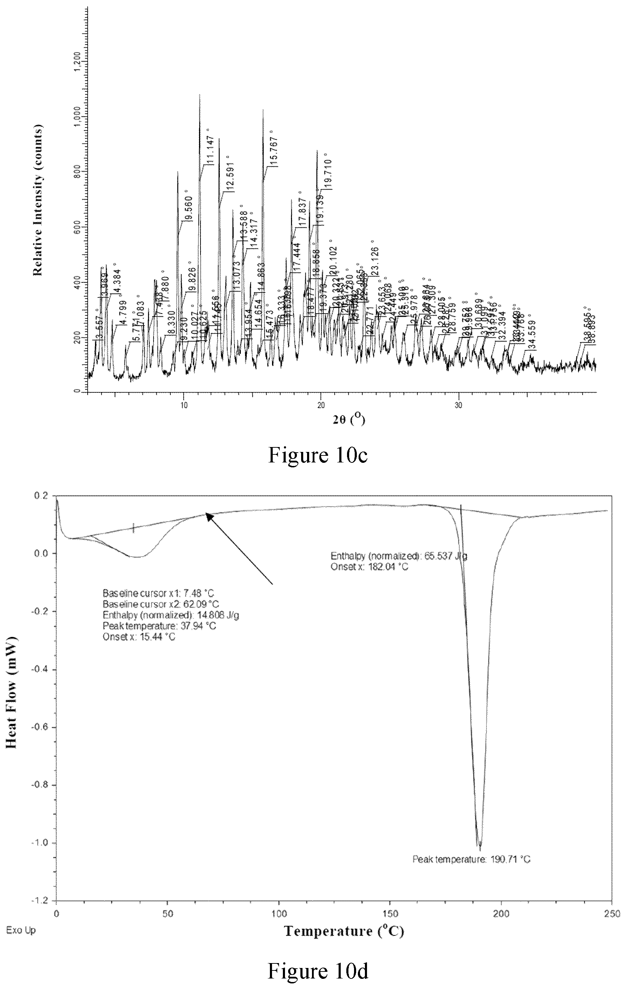

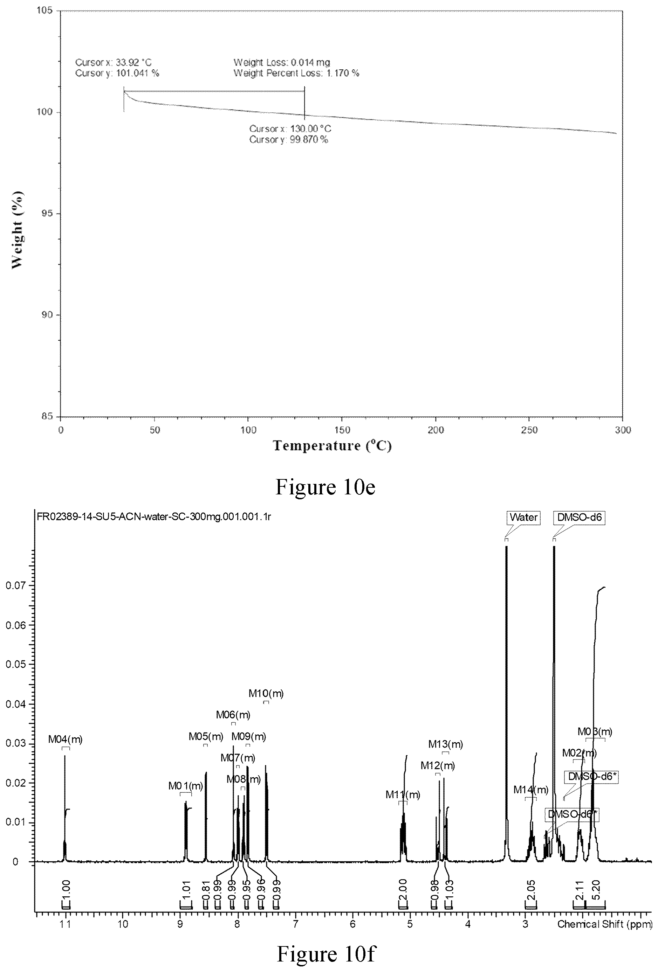

In some preferred embodiments, the form has an XRPD pattern substantially as shown in Figure 10c, and optionally has one or more of the following features:
1) having an endothermic peak at 37.94 °C ± 2 °C and an endothermic peak at 190.71 °C ± 2 °C in DSC pattern;
2) having a weight loss of 1.17 ± 0.2 wt% before reaching 130 °C in TGA pattern;
3) having a DSC pattern substantially as shown in Figure 10d; and/or
4) having a TGA pattern substantially as shown in Figure 10e.

### Crystalline form F of the compound of formula A

In one embodiment, the form is a crystalline form F of the compound of formula A, which has at least three, at least four, at least five, at least six or seven characteristic peaks at the following positions in an X-ray powder diffraction (XRPD) pattern in 2θ angle: 17.70 ± 0.2°, 21.46 ± 0.2°, 27.66 ± 0.2°, 19.20 ± 0.2°, 17.17 ± 0.2°, 19.44 ± 0.2° and 22.01 ± 0.2°.

In some preferred embodiments, the form has XRPD characteristic peaks at positions substantially as shown in Table 11 below:

**Table 11**

| Index | Angle | Intensity | Rel. Intensity |
|---|---|---|---|
| 1 | 4.604 ° | 109.552 | 1.9% |
| 2 | 5.546 ° | 170.319 | 6.4% |
| 3 | 5.806 ° | 188.453 | 7.8% |
| 4 | 6.150 ° | 164.024 | 6.1% |
| 5 | 7.887 ° | 137.324 | 4.2% |
| 6 | 8.315 ° | 154.104 | 5.2% |
| 7 | 8.872 ° | 268.894 | 13.3% |
| 8 | 10.488 ° | 382.153 | 20.5% |
| 9 | 11.019 ° | 185.002 | 6.2% |
| 10 | 11.548 ° | 569.554 | 33.3% |
| 11 | 13.015 ° | 496.565 | 27.7% |
| 12 | 13.247 ° | 297.978 | 13.5% |
| 13 | 13.759 ° | 289.336 | 12.9% |
| 14 | 14.966 ° | 162.817 | 3.5% |
| 15 | 15.791 ° | 501.556 | 25.5% |
| 16 | 16.583 ° | 389.482 | 15.0% |
| 17 | 17.177 ° | 998.268 | 56.1% |
| 18 | 17.709 ° | 1640.35 | 100.0% |
| 19 | 18.283 ° | 727.739 | 33.7% |
| 20 | 18.894 ° | 497.223 | 16.0% |
| 21 | 19.201 ° | 1318.69 | 73.6% |
| 22 | 19.447 ° | 993.646 | 50.1% |
| 23 | 19.822 ° | 376.284 | 5.8% |
| 24 | 20.155 ° | 465.805 | 11.7% |
| 25 | 20.680 ° | 748.442 | 31.1% |
| 26 | 20.963 ° | 541.226 | 16.1% |
| *27* | 21.462 ° | 1375.01 | 74.8% |
| 28 | 21.749 ° | 965.132 | 45.6% |
| 29 | 22.019 ° | 1008.26 | 48.5% |
| 30 | 22.343 ° | 501.928 | 12,6% |
| 31 | 22.810 ° | 492.802 | 12.0% |
| 32 | 23.232 ° | 543.864 | 15.8% |
| 33 | 23.523 ° | 609.678 | 20.6% |
| 34 | 23.903 ° | 407.481 | 6.6% |
| 35 | 24.377 ° | 630.518 | 22.5% |
| 36 | 24.631 ° | 458.758 | 10.3% |
| 37 | 25.393 ° | 495.092 | 13.2% |
| 38 | 25.912 ° | 582.672 | 19.8% |
| 39 | 26.777 ° | 405.138 | 8.3% |
| 40 | 27.210 ° | 403.998 | 9.0% |
| 41 | 27.660 ° | 1317.18 | 74.7% |
| 42 | 28.106 ° | 724.397 | 33.7% |
| 43 | 28.977 ° | 390.177 | 12.4% |
| 44 | 29.742 ° | 316.504 | 7.5% |
| 45 | 30.118 ° | 283.089 | 4.9% |
| 46 | 30.545 ° | 252.713 | 26% |
| 47 | 31.146 ° | 282.776 | 4.8% |
| 48 | 31.937 ° | 304.295 | 6.7% |
| 49 | 32.690 ° | 325.014 | 8.9% |
| 50 | 33.634 ° | 289.331 | 6.9% |

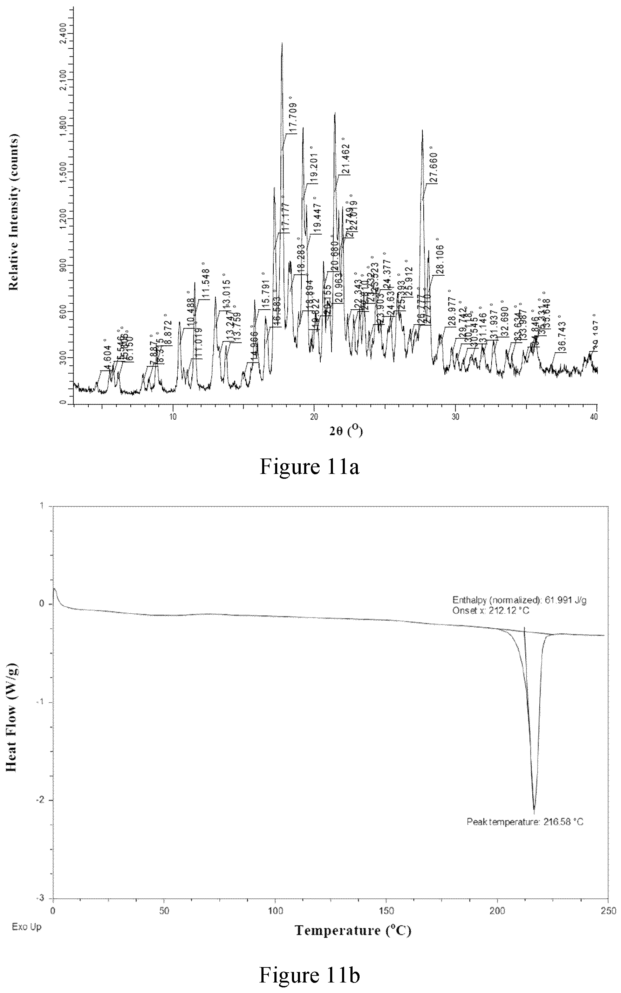

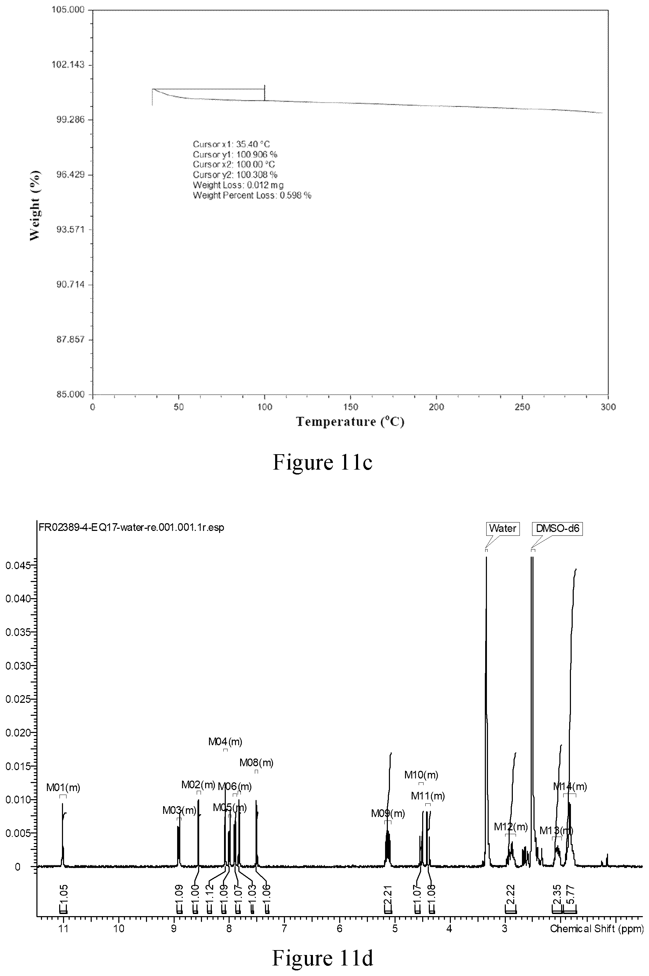

In some preferred embodiments, the form has an XRPD pattern substantially as shown in Figure 11a, and optionally has one or more of the following features:
1) having an endothermic peak at 216.58 °C ± 2 °C in DSC pattern;
2) having a weight loss of 0.598 ± 0.2 wt% before reaching 100 °C in TGA pattern;
3) having a DSC pattern substantially as shown in Figure 11b; and/or
4) having a TGA pattern substantially as shown in Figure 11c.

### Crystalline form G of the compound of formula A

In one embodiment, the from is a crystalline form G of the compound of formula A, which has at least three, at least four, at least five, at least six or seven characteristic peaks at the following positions in an X-ray powder diffraction (XRPD) pattern in 2θ angle: 18.83 ± 0.2°, 13.88 ± 0.2°, 21.45 ± 0.2°, 26.75 ± 0.2°, 15.92 ± 0.2°, 17.95 ± 0.2° and 13.14 ± 0.2°.

In some preferred embodiments, the form has XRPD characteristic peaks at positions substantially as shown in Table 12 below:

**Table 12**

| Index | Angle | Intensity | Rel. Intensity |
|---|---|---|---|
| 1 | 7.355 ° | 94.2941 | 1.6% |
| 2 | 9.002 ° | 83.7517 | 1.1% |
| 3 | 11.598 ° | 86.9896 | 0.7% |
| 4 | 12.430 ° | 159.645 | 2.3% |
| 5 | 13.141 ° | 676.318 | 15.2% |
| 6 | 13.886 ° | 1545.5 | 37.2% |
| 7 | 14.673 ° | 356.66 | 7.2% |
| 8 | 15.927 ° | 859.852 | 20.1% |
| 9 | 16.531 ° | 211.2 | 3.8% |
| 10 | 17.137 ° | 208.944 | 3.6% |
| 11 | 17.953 ° | 869.03 | 19.9% |
| 12 | 18.837 ° | 4034.74 | 100.0% |
| 13 | 19.586 ° | 276.525 | 5.0% |
| 14 | 20.529 ° | 97.8431 | 0.6% |
| 15 | 21.031 ° | 331.751 | 6.3% |
| 16 | 21.455 ° | 1391.08 | 33.0% |
| 17 | 22.034 ° | 241.296 | 3.9% |
| 18 | 22.312 ° | 184.232 | **2.5%** |
| 19 | 22.896 ° | 270.145 | 4.7% |
| 20 | 23.233 ° | 163.817 | 2.0% |
| 21 | 23.647 ° | 157.872 | 1.7% |
| 22 | 24.503 ° | 393.162 | 7.4% |
| 23 | 24.761 ° | 519.971 | 10.5% |
| 24 | 25.068 ° | 677.033 | 14.4% |
| 25 | 25.362 ° | 434.118 | 8.2% |
| 26 | 25.997 ° | 361.374 | 6.2% |
| 27 | 26.455 ° | 411.917 | 7.4% |
| 28 | 26.755 ° | 918.346 | 20.2% |
| 29 | 27.109 ° | 293.309 | 4.4% |
| 30 | 27.873 ° | 558.659 | 11.3% |
| 31 | 28.552 ° | 343.613 | 6.1% |
| 32 | 29.454 ° | 413.626 | 7.7% |
| 33 | 29.783 ° | 571.263 | 11.6% |
| 34 | 30.186 ° | 267.033 | 3.9% |
| 35 | 30.407 ° | 203.296 | 2.3% |
| 36 | 30.832 ° | 419.995 | 7.9% |
| 37 | 31.832 ° | 428.625 | 8.2% |
| 38 | 32.516 ° | 416.376 | 8.0% |
| 39 | 32.783 ° | 252.051 | 3.9% |
| 40 | 33.646 ° | 186.674 | 2.3% |
| 41 | 33.822 ° | 179.265 | 2.1% |
| 42 | 34,403 ° | 300.504 | 5.1% |
| 43 | 34.900 ° | 190.403 | 2,4% |
| 44 | 35.937 ° | 236.792 | 3.7% |
| 45 | 36.233 ° | 176.214 | 2.1% |
| 46 | 36.440 ° | 175.915 | 2.0% |
| 47 | 38.278 ° | 319.277 | 5.3% |
| 48 | 38.892 ° | 326.188 | 5.6% |
| 49 | 39.595 ° | 134.695 | 0.8% |

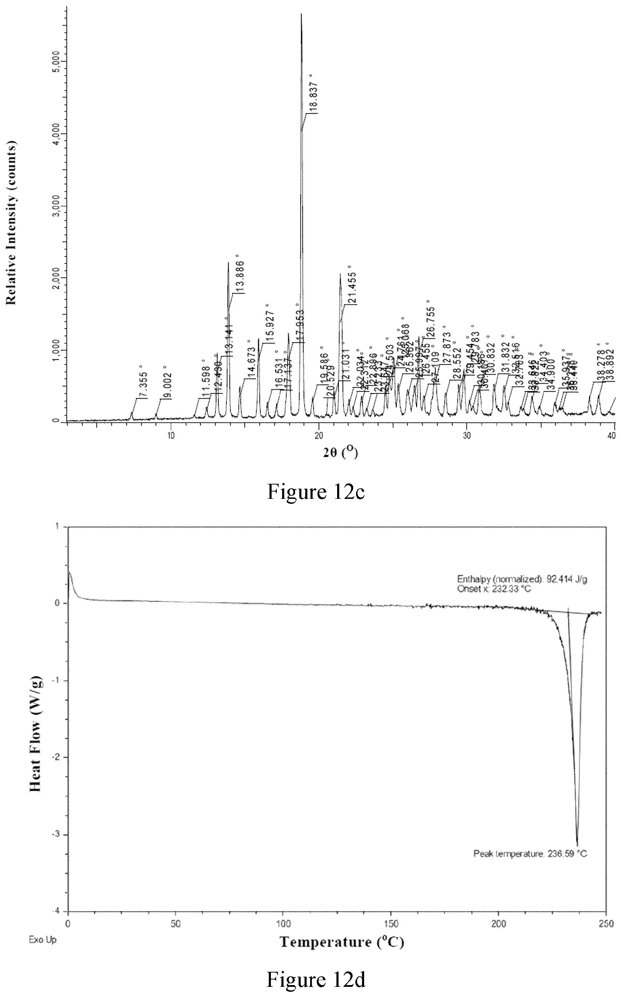

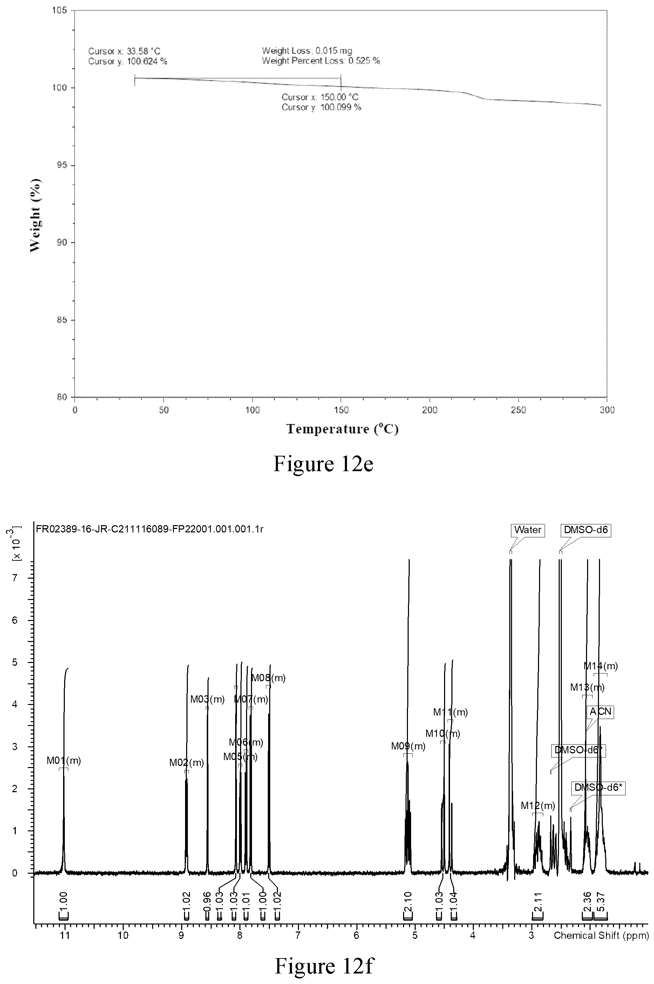

In some preferred embodiments, the form has an XRPD pattern substantially as shown in Figure 12c, and optionally has one or more of the following features:
1) having an endothermic peak at 236.59 °C ± 2 °C in DSC pattern;
2) having a weight loss of 0.52 ± 0.2 wt% before reaching 150 °C in TGA pattern;
3) having a DSC pattern substantially as shown in Figure 12d; and/or
4) having a TGA pattern substantially as shown in Figure 12e.

### Crystalline form H of the compound of formula A

In one embodiment, the from is a crystalline form H of the compound of formula A, which has at least three, at least four, at least five, at least six or seven characteristic peaks at the following positions in an X-ray powder diffraction (XRPD) pattern in 2θ angle: 18.30 ± 0.2°, 3.80 ± 0.2°, 19.05 ± 0.2°, 18.53 ± 0.2°, 11.81 ± 0.2°, 11.33 ± 0.2° and 16.04 ± 0.2°;

In some preferred embodiments, the form has XRPD characteristic peaks at positions substantially as shown in Table 13 below:

**Table 13**

| Index | Angle | Intensity | Rel. Intensity |
|---|---|---|---|
| 1 | 3.800 ° | 552.29 | 65.3% |
| 2 | 9.840 ° | 156.247 | 20.9% |
| 3 | 10.712 ° | 172.897 | 23.9% |
| 4 | 11.337 ° | 208.76 | 31.5% |
| 5 | 11.819 ° | 221.933 | 34.7% |
| 6 | 15.120 ° | 114.06 | 14.3% |
| 7 | 16.047 ° | 200.812 | 31.2% |
| 8 | 17.658 ° | 110.213 | 11.8% |
| 9 | 18.303 ° | 554.89 | 100.0% |
| 10 | 18.533 ° | 327.285 | 53.8% |
| 11 | 19.059 ° | 383.05 | 64.5% |
| 12 | 19.713 ° | 139.692 | 15.4% |
| 13 | 21.020 ° | 133.398 | 15.3% |
| 14 | 21.434 ° | 106.707 | 10.7% |
| 15 | 22.653 ° | 68.3525 | 4.1% |
| 16 | 23.806 ° | 70.6591 | 4.3% |
| 17 | 24.954 ° | 94.1567 | 7.2% |
| 18 | 25.326 ° | 123.831 | 12.9% |
| 19 | 26.401 ° | 110.757 | 10.6% |
| 20 | 27.395 ° | 152.237 | 19.2% |
| 21 | 29.056 ° | 122.835 | 14.3% |
| 22 | 30.563 ° | 80.4368 | 6.7% |
| 23 | 32.398 ° | 80.2633 | 4.9% |
| 24 | 33.722 ° | 93.7847 | 7.1% |

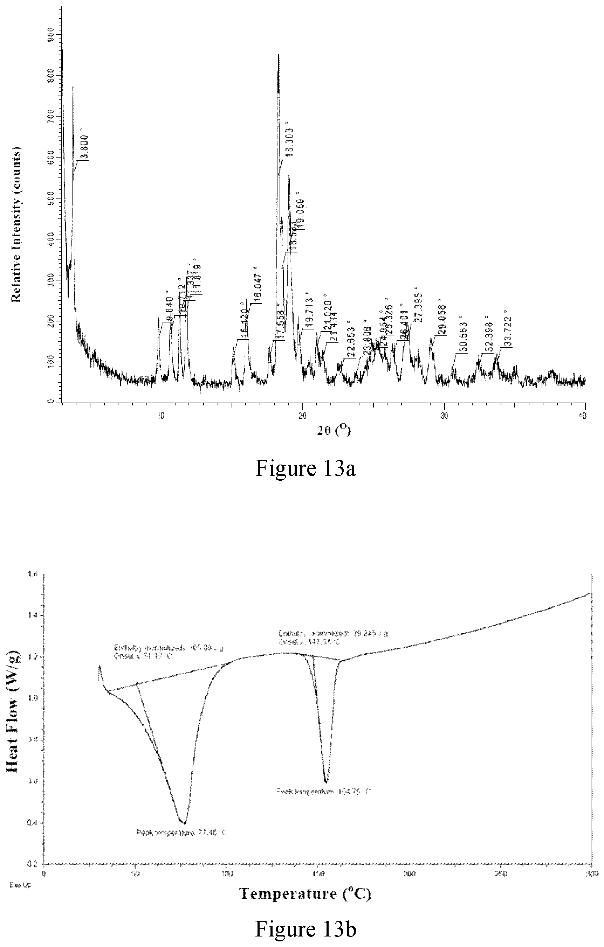

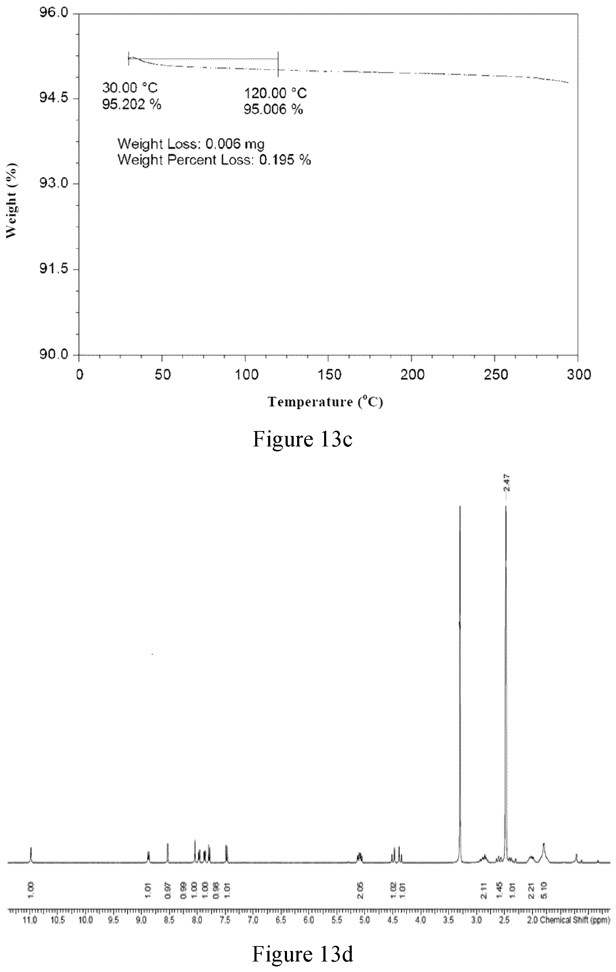

In some preferred embodiments, the form has an XRPD pattern substantially as shown in Figure 13a, and optionally has one or more of the following features:
1) having an endothermic peak at 77.45 °C ± 2 °C and an endothermic peak at 154.75 °C ± 2 °C in DSC pattern;
2) having a weight loss of 0.19 ± 0.2 wt% before reaching 120 °C in TGA pattern;
3) having a DSC pattern substantially as shown in Figure 13b; and/or
4) having a TGA pattern substantially as shown in Figure 13c.

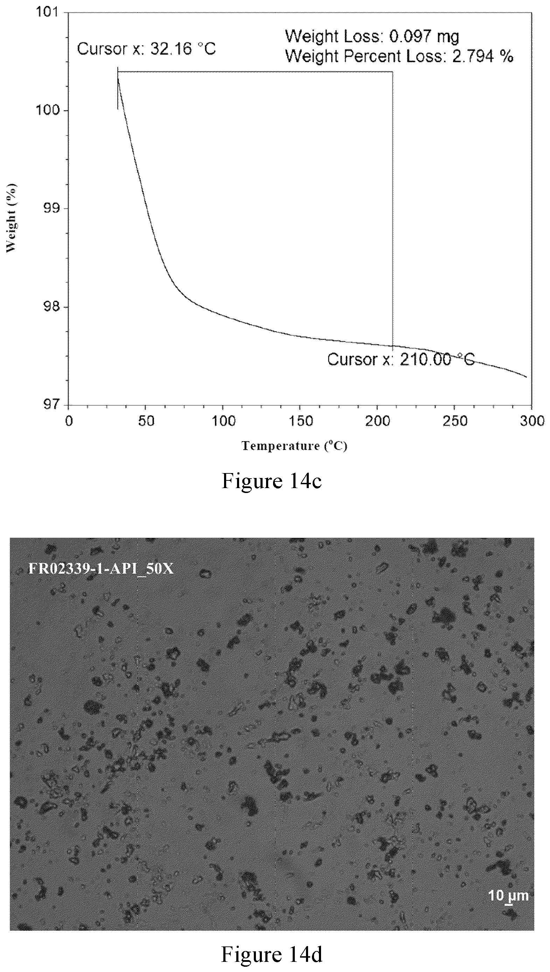

### The amorphous form of the compound of formula A as shown in PLM Figure 14d

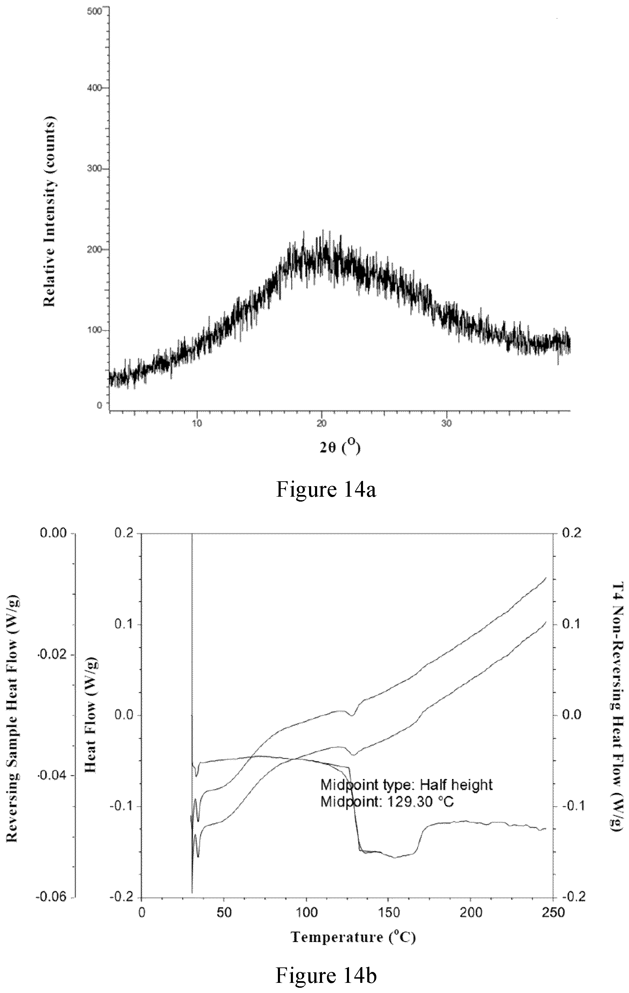

In one embodiment, the form is an amorphous form of the compound of formula A as shown in PLM Figure 14d, preferably, the form has an XRPD pattern as shown in Figure 14a, and optionally has one or more of the following features:
1) having a glass transition temperature at 129.30 °C ± 2.0 °C in mDSC pattern;
2) having a weight loss of 2.7 wt% before reaching 210 °C ± 2.0 °C in TGA pattern;
3) having a mDSC pattern substantially as shown in Figure 14b; and/ or
4) having a TGA pattern substantially as shown in Figure 14c.

**In the second aspect,** the present invention provides a method for preparing a crystalline form or an amorphous form of the compound of formula A or a pharmaceutically acceptable salt or solvate thereof, comprising the steps of: obtaining the crystalline form or amorphous form by subjecting the compound of formula A or a pharmaceutically acceptable salt thereof to suspend, slowly cool, rapidly cool, slowly volatilize, rapidly volatilize, add an antisolvent dropwise, add an antisolvent dropwise reversely, vapor diffusion or heat-cool DSC crystallization method, or spray dry, hot melt extrusion or solvent evaporation.

In the preparation method, the compound of formula A is synthesized in the laboratory as described in the specific examples. The solvent may be a commonly used solvent in the laboratory, for example, one or more selected from water, alkane solvents, alcohol solvents, ketone solvents, ester solvents, aromatic hydrocarbon solvents, halogenated hydrocarbon solvents, nitrile solvents, ether solvents, aliphatic hydrocarbon solvents, polar aprotic solvents such as DMF and DMSO. The weight to volume ratio of the compound of formula A to the solvent may be 100 mg: (0.1~1 mL).

In one embodiment, the present invention provides a method for preparing a crystalline form of a solvate of the compound of formula A, comprising the steps of mixing the compound of formula A with a solvent corresponding to the solvate, separating the resulting solid and drying it, thereby obtaining a solvate crystalline form of the compound of formula A.

In some preferred embodiments, the solvent corresponding to the solvate is, for example but not limited to, 1,4-dioxane, ethyl acetate, toluene, chloroform, 2-methyltetrahydrofuran, methyl tert-butyl ether, acetone, N,N-dimethylformamide, acetonitrile, and the like.

In one embodiment, the present invention provides a method for preparing an amorphous form of the compound of formula A, comprising the steps of mixing the compound of formula A with a solvent and spray-drying the resulting solution, thereby obtaining an amorphous form of the compound of formula A.

In some preferred embodiments, the solvent may be a commonly used solvent in the laboratory, for example, one or more selected from water, alkane solvents, alcohol solvents, ketone solvents, ester solvents, aromatic hydrocarbon solvents, halogenated hydrocarbon solvents, nitrile solvents, ether solvents, aliphatic hydrocarbon solvents, polar aprotic solvents such as DMF and DMSO. Dichloromethane (DCM) is preferred.

**In the third aspect,** the present invention provides a pharmaceutical composition comprising:
(a) a form described in the first aspect; and
(b) a pharmaceutically acceptable carrier or excipient.

The crystalline form or amorphous form of the compound of formula A or a salt or solvate thereof may be a therapeutically effective amount. The pharmaceutically acceptable excipient may be an excipient well known in the art, and in the case of solid formulations, they include but are not limited to: diluents, binders, disintegrants, lubricants, glidants, release rate control agents, plasticizers, preservatives, antioxidants, etc.

**In the fourth aspect,** the present invention provides a pharmaceutical formulation comprising the above-mentioned pharmaceutical composition; wherein the pharmaceutical formulation may be a solid formulation, or may be a powder, granule, tablet, capsule, pill or film.

**In the fifth aspect,** the present invention provides A use of the above-mentioned crystalline form, amorphous form or pharmaceutical composition in the manufacture of a medicament for treating a proliferative disease, wherein the proliferative disease includes breast cancer, colon cancer, brain cancer, prostate cancer, kidney cancer, pancreatic cancer, ovarian cancer, head and neck cancer, melanoma, colorectal cancer, gastric cancer, squamous cell carcinoma, small cell lung cancer, non-small cell lung cancer, testicular cancer, Merkel cell carcinoma, glioblastoma, neuroblastoma, lymphoid organ cancer and hematological malignancy including leukemia (acute lymphoblastic leukemia (ALL), acute myeloid leukemia (AML), chronic lymphocytic leukemia (CLL), chronic myeloid leukemia (CML), acute monocytic leukemia (AMOL), hairy cell leukemia (HCL), T cell prolymphocytic leukemia (T-PLL), large granular lymphocytic leukemia, adult T cell leukemia), lymphoma (small lymphocytic lymphoma (SLL), Hodgkin's lymphoma (nodular sclerosis, mixed cellular, lymphocyte-rich, lymphocyte depleted or not depleted, and nodular lymphocyte-predominant Hodgkin lymphoma), non-Hodgkin's lymphoma (all subtypes), chronic lymphocytic leukemia/small lymphocytic lymphoma, B cell prolymphocytic leukemia, lymphoplasmacytic lymphoma (such as Waldenström macroglobulinemia), splenic marginal zone lymphoma, plasma cell neoplasms (plasma cell myeloma, plasmacytoma, monoclonal immunoglobulin deposition disease, heavy chain disease), extranodal marginal zone B cell lymphoma (MALT lymphoma), lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, Burkitt lymphoma/leukemia, T cell prolymphocytic leukemia, T cell large granular lymphocytic leukemia, aggressive NK cell leukemia, adult T cell leukemia/lymphoma, extranodal NK/T cell lymphoma (nasal type), enteropathy-type T cell lymphoma, hepatosplenic T cell lymphoma, blastic NK cell lymphoma, mycosis fungoides/Sezary syndrome, primary cutaneous CD30-positive T cell lymphoma disease, primary cutaneous anaplastic large cell lymphoma, lymphomatoid papulosis, angioimmunoblastic T cell lymphoma, peripheral T cell lymphoma (unspecified), anaplastic large cell lymphoma, multiple myeloma (plasma cell myeloma or Kahler's disease).

**In the sixth aspect,** the present invention provides a method for treating a proliferative disease, comprising a step of administering to a subject in need thereof a therapeutically effective amount of the form of the first aspect of the present invention or the pharmaceutical composition of the third aspect of the present invention or the pharmaceutical formulation of the fourth aspect of the present invention.

In some preferred embodiments, the subject is a mammal, such as a human.

The crystalline form or amorphous form of the compound of formula A or a salt or solvate thereof of the present invention has the following advantages: the present invention discovered multiple unreported crystalline forms or amorphous forms of the compound of formula A or a salt or solvate thereof for the first time, and the forms can serve as an important basis for subsequent drug development, formulation development and production.

It should be understood that within the scope of the present invention, the above-mentioned technical features of the present invention and the technical features specifically described below (such as embodiments) may be combined with each other to form a new or preferred technical solution. Due to space limitations, they will not be repeated herein.

### BRIEF DESCRIPTION OF THE DRAWINGS

Figure 1a shows the chemical purity spectrum of the crystalline form A1 of the compound of formula A.
Figure 1b shows the chiral purity spectrum of crystalline form A1 of the compound of formula A.
Figure 1c shows the X-ray powder diffraction pattern of the crystalline form A1 of the compound of formula A.
Figure 1d shows the differential scanning calorimetry pattern of the crystalline form A1 of the compound of formula A.
Figure 1e shows the pattern of thermogravimetric analysis of the crystalline form A1 of the compound of formula A.
Figure 1f shows the ¹HNMR spectrum of the crystalline form A1 of the compound of formula A.
Figure 2a shows the X-ray powder diffraction pattern of the crystalline form A2 of the compound of formula A.
Figure 2b shows the differential scanning calorimetry pattern of the crystalline form A2 of the compound of formula A.
Figure 2c shows the pattern of thermogravimetric analysis of the crystalline form A2 of the compound of formula A.
Figure 2d shows the ¹HNMR spectrum of the crystalline form A2 of the compound of formula A.
Figure 3a shows the X-ray powder diffraction pattern of the crystalline form A3 of the compound of formula A.
Figure 3b shows the differential scanning calorimetry pattern of the crystalline form A3 of the compound of formula A.
Figure 3c shows the pattern of thermogravimetric analysis of the crystalline form A3 of the compound of formula A.
Figure 3d shows the ¹HNMR spectrum of the crystalline form A3 of the compound of formula A.
Figure 4a shows the X-ray powder diffraction pattern of the crystalline form A4 of the compound of formula A.
Figure 4b shows the differential scanning calorimetry pattern of the crystalline form A4 of the compound of formula A.
Figure 4c shows the pattern of thermogravimetric analysis of the crystalline form A4 of the compound of formula A.
Figure 4d shows the ¹HNMR spectrum of the crystalline form A4 of the compound of formula A.
Figure 5a shows the X-ray powder diffraction pattern of the crystalline form A5 of the compound of formula A.
Figure 5b shows the differential scanning calorimetry pattern of the crystalline form A5 of the compound of formula A.
Figure 5c shows the pattern of thermogravimetric analysis of the crystalline form A5 of the compound of formula A.
Figure 5d shows the ¹HNMR spectrum of the crystalline form A5 of the compound of formula A.
Figure 6a shows the chemical purity spectrum of crystalline form B of the compound of formula A.
Figure 6b shows the chiral purity spectrum of crystalline form B of the compound of formula A.
Figure 6c shows the X-ray powder diffraction pattern of crystalline form B of the compound of formula A.
Figure 6d shows the differential scanning calorimetry pattern of crystalline form B of the compound of formula A.
Figure 6e shows the pattern of thermogravimetric analysis of crystalline form B of the compound of formula A.
Figure 6f shows the ¹HNMR spectrum of crystalline form B of the compound of formula A.
Figure 7a shows the X-ray powder diffraction pattern of crystalline form C of the compound of formula A.
Figure 7b shows the differential scanning calorimetry pattern of crystalline form C of the compound of formula A.
Figure 7c shows the pattern of thermogravimetric analysis of crystalline form C of the compound of formula A.
Figure 7d shows the ¹HNMR spectrum of crystalline form C of the compound of formula A.
Figure 8a shows the X-ray powder diffraction pattern of crystalline form D1 of the compound of formula A.
Figure 8b shows the differential scanning calorimetry pattern of crystalline form D1 of the compound of formula A.
Figure 8c shows the pattern of thermogravimetric analysis of crystalline form D1 of the compound of formula A.
Figure 8d shows the ¹HNMR spectrum of crystalline form D1 of the compound of formula A.
Figure 9a shows the X-ray powder diffraction pattern of crystalline form D2 of the compound of formula A.
Figure 9b shows the differential scanning calorimetry pattern of crystalline form D2 of the compound of formula A.
Figure 9c shows the pattern of thermogravimetric analysis of crystalline form D2 of the compound of formula A.
Figure 9d shows the ¹HNMR spectrum of crystalline form D2 of the compound of formula A.
Figure 10a shows the chemical purity spectrum of crystalline form E of the compound of formula A.
Figure 10b shows the chiral purity spectrum of crystalline form E of the compound of formula A.
Figure 10c shows the X-ray powder diffraction pattern of crystalline form E of the compound of formula A.
Figure 10d shows the differential scanning calorimetry pattern of crystalline form E of the compound of formula A.
Figure 10e shows the pattern of thermogravimetric analysis of crystalline form E of the compound of formula A.
Figure 10f shows the ¹HNMR spectrum of crystalline form E of the compound of formula A.
Figure 11a shows the X-ray powder diffraction pattern of crystalline form F of the compound of formula A.
Figure 11b shows the differential scanning calorimetry pattern of crystalline form F of the compound of formula A.
Figure 11c shows the pattern of thermogravimetric analysis of crystalline form F of the compound of formula A.
Figure 11d shows the ¹HNMR spectrum of crystalline form F of the compound of formula A.
Figure 12a shows the chemical purity spectrum of crystalline form G of the compound of formula A.
Figure 12b shows the chiral purity spectrum of crystalline form G of the compound of formula A.
Figure 12c shows the X-ray powder diffraction pattern of crystalline form G of the compound of formula A.
Figure 12d shows the differential scanning calorimetry pattern of crystalline form G of the compound of formula A.
Figure 12e shows the pattern of thermogravimetric analysis of crystalline form G of the compound of formula A.
Figure 12f shows the ¹HNMR spectrum of crystalline form G of the compound of formula A.
Figure 13a shows the X-ray powder diffraction pattern of crystalline form H of the compound of formula A.
Figure 13b shows the differential scanning calorimetry pattern of crystalline form H of the compound of Formula A.
Figure 13c shows the pattern of thermogravimetric analysis of crystalline form H of the compound of formula A.
Figure 13d shows the ¹HNMR spectrum of crystalline form H of the compound of formula A.
Figure 14a shows the X-ray powder diffraction pattern of the amorphous form of the compound of Formula A.
Figure 14b shows the modulated differential scanning calorimetry (mDSC) pattern of the amorphous form of the compound of Formula A.
Figure 14c shows the pattern of thermogravimetric analysis of the amorphous form of the compound of formula A.
Figure 14d shows the polarized light microscopy (PLM) image of the amorphous form of the compound of Formula A.
Figure 15a shows the chemical purity of crystalline form B of the compound of formula A.
Figure 15b shows the chiral purity of crystalline form B of the compound of Formula A.
Figure 15c shows the chemical purity of crystalline form B of the compound of formula A.
Figure 15d shows the chiral purity of crystalline form B of the compound of Formula A.
Figure 15e shows the stacked X-ray powder diffraction pattern of the stability test of the crystalline form B of the compound of formula A.
Figure 15f shows the chemical purity of crystalline form B of the compound of formula A.
Figure 15g shows the chiral purity of crystalline form B of the compound of Formula A.
Figure 15h shows the chemical purity of crystalline form B of the compound of formula A.
Figure 15i shows the chiral purity of crystalline form B of the compound of Formula A.
Figure 16a shows the chemical purity of crystalline form E of the compound of formula A.
Figure 16b shows the chiral purity of crystalline form E of the compound of Formula A.
Figure 16c shows the chemical purity of crystalline form E of the compound of formula A.
Figure 16d shows the chiral purity of crystalline form E of the compound of Formula A.
Figure 16e shows the stacked X-ray powder diffraction pattern of the stability test of crystalline form E of the compound of formula A.
Figure 16f shows the chemical purity of crystalline form E of the compound of formula A.
Figure 16g shows the chiral purity of crystalline form E of the compound of Formula A.
Figure 16h shows the chemical purity of crystalline form E of the compound of formula A.
Figure 16i shows the chiral purity of crystalline form E of the compound of Formula A.

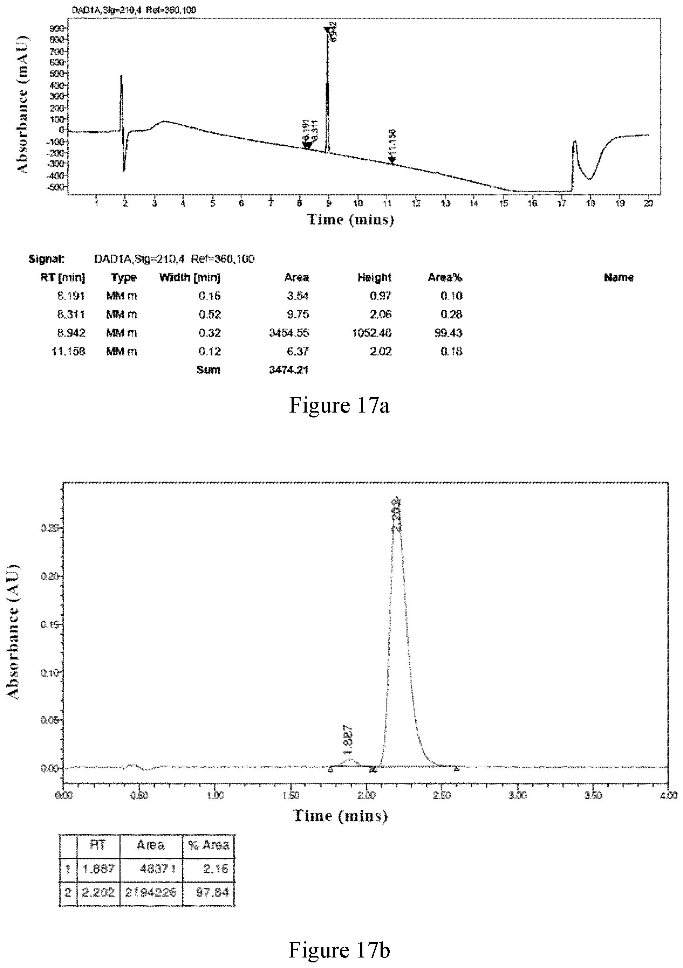

Figure 17a shows the chemical purity of crystalline form G of the compound of formula A.
Figure 17b shows the chiral purity of crystalline form G of the compound of Formula A.

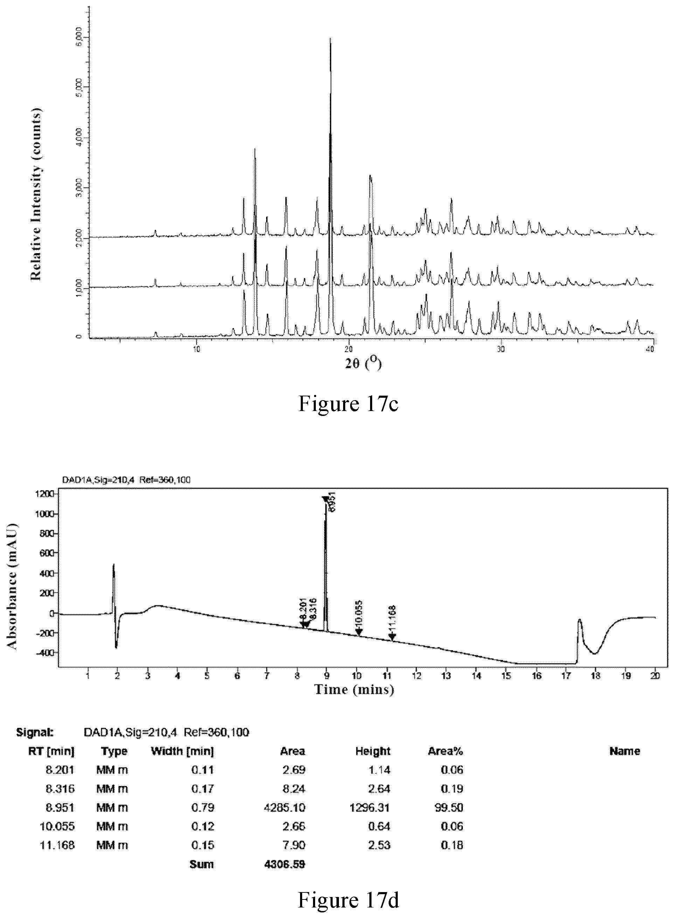

Figure 17c shows the stacked X-ray powder diffraction pattern of the stability test of crystalline form G of the compound of formula A.
Figure 17d shows the chemical purity of crystalline form G of the compound of formula A.

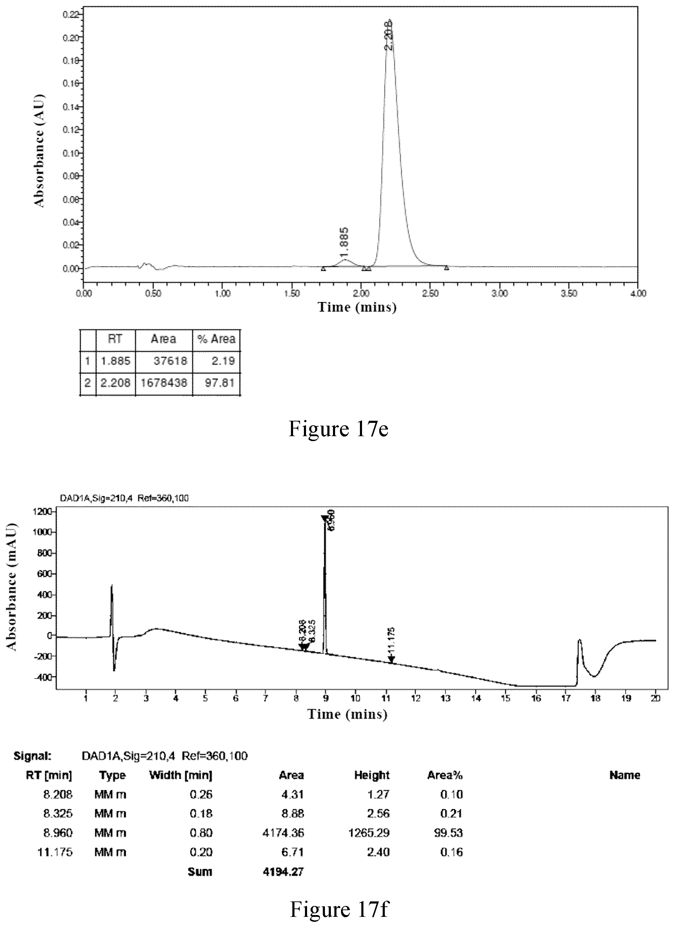

Figure 17e shows the chiral purity of crystalline form G of the compound of Formula A.
Figure 17f shows the chemical purity of crystalline form G of the compound of formula A.

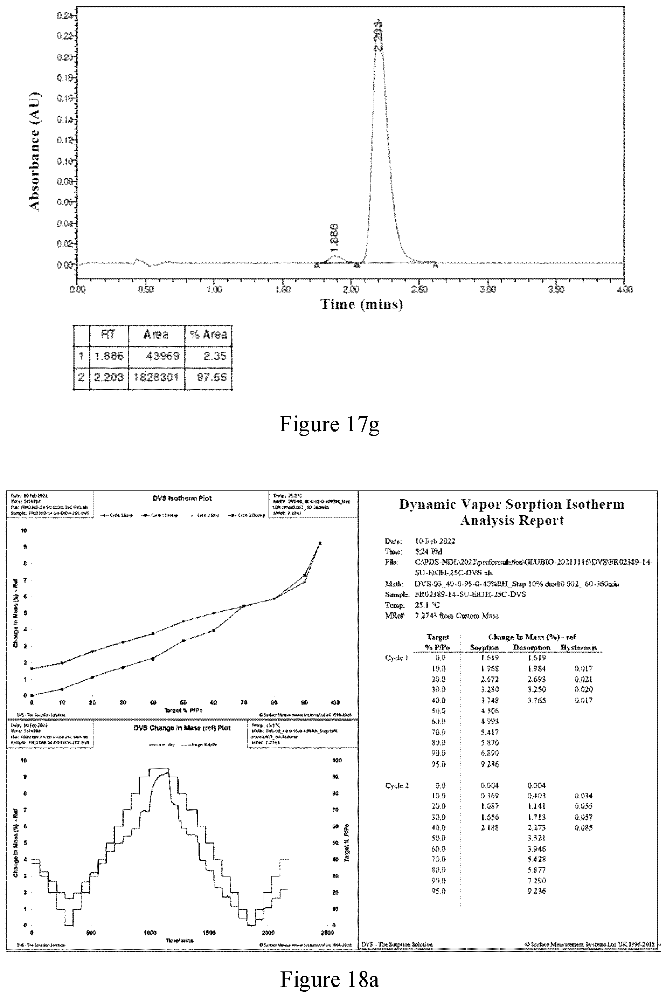

Figure 17g shows the chiral purity of crystalline form G of the compound of Formula A.
Figure 18a shows the DVS spectrum of crystalline form B of the compound of formula A.

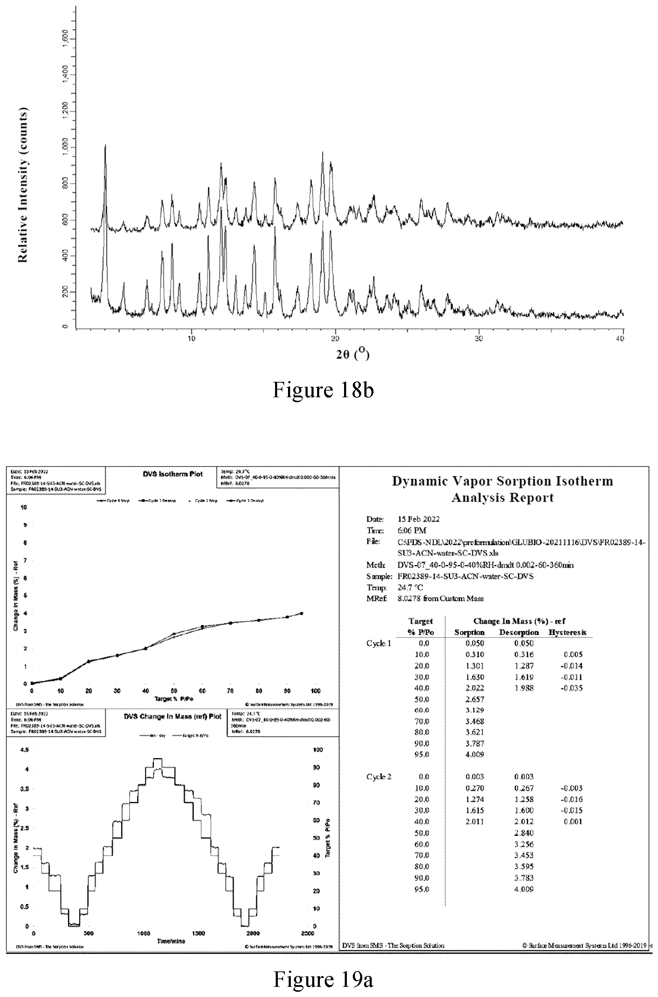

Figure 18b shows the X-ray powder diffraction pattern of crystalline form B of the compound of formula A before and after the hygroscopicity experiment.
Figure 19a shows the DVS spectrum of crystalline form E of the compound of formula A.

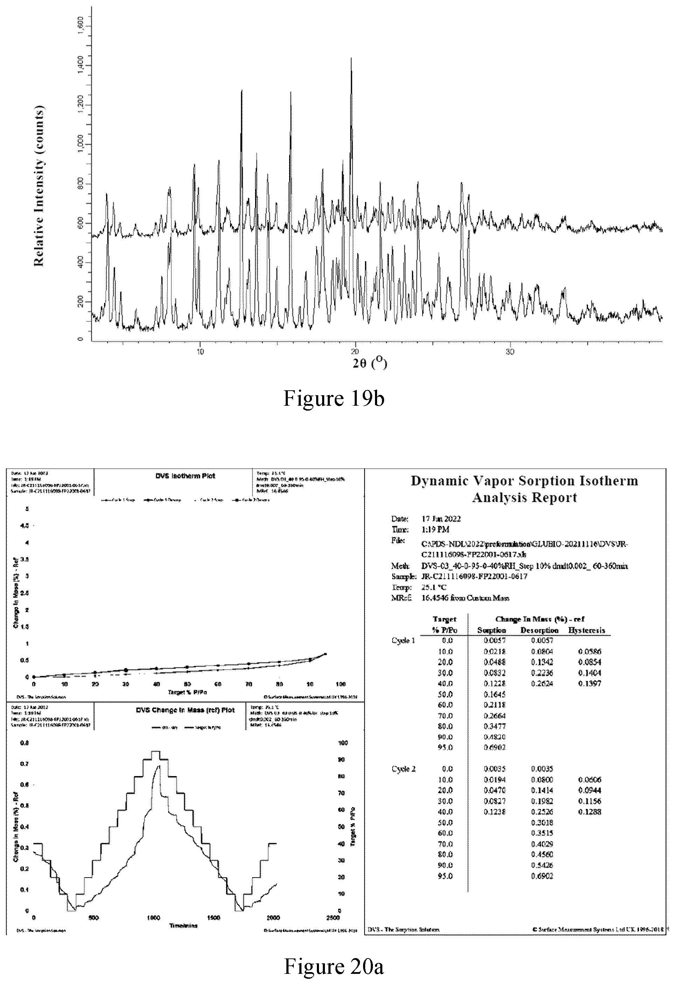

Figure 19b shows the X-ray powder diffraction pattern of crystalline form E of the compound of formula A before and after the hygroscopicity experiment.
Figure 20a shows the DVS spectrum of crystalline form G of the compound of formula A.

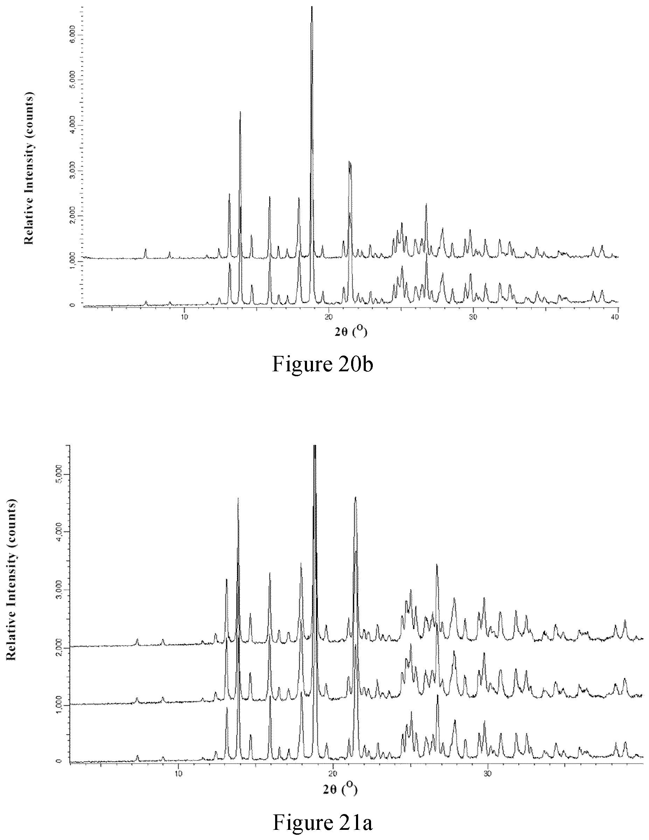

Figure 20b shows the X-ray powder diffraction pattern of crystalline form G of the compound of formula A before and after the hygroscopicity experiment.
Figure 21a shows the stacked XRPD pattern of a sample of crystalline form G of the compound of formula A obtained after simulated tableting experiment.

### DETAILED DESCRIPTION

The inventors discovered several crystalline forms and amorphous forms of the compound of formula A or a pharmaceutically acceptable salt, or solvate thereof through long-term and in-depth research. The crystalline forms and amorphous forms have better drug bioavailability, and the crystalline forms have high purity and high stability, and are suitable for preparing a pharmaceutical composition for treating proliferative diseases, thereby being more preferable for treating diseases such as cancer, myeloproliferative and inflammatory diseases. In addition, the crystalline forms and amorphous form of the present invention are not easy to be stirred up, easy to collect, not easy to cause waste, and are helpful in protecting the health of operators during the process of drug manufacturing such as packaging. On this basis, the inventors have completed the present invention.

### Active ingredients

In the present invention, the active ingredient refers to the amorphous form or crystalline form of the compound of formula A or a pharmaceutically acceptable salt or solvate thereof. Preferably, the active ingredient is crystalline form A1, crystalline form A2, crystalline form A3, crystalline form A4, crystalline form A5, crystalline form B, crystalline form C, crystalline form D1, crystalline form D2, crystalline form E, crystalline form F, crystalline form G, crystalline form H and amorphous form as described above.

### Abbreviation

| **Abbreviation** | **Full Name** |
|---|---|
| ACN | Acetonitrile |
| DSC | Differential Scanning Calorimetry |
| DVS | Dynamic Vapor Sorption |
| DCM | Dichloromethane |
| DMSO | Dimethyl Sulfoxide |
| DMAc | N,N-Dimethylacetamide |
| EtOH | Ethanol |
| EA | Ethyl Acetate |
| HPLC | High Performance Liquid Chromatography |
| KF | Karl Fischer |
| 2-MeTHF | 2-Methyltetrahydrofuran |
| MeOH | Methanol |
| MTBE | Methyl Tert-butyl Ether |
| NMR | Nuclear Magnetic Resonance |
| NMM | N-Methylmorpholine |
| TGA | Thermogravimetric Analysis |
| THF | Tetrahydrofuran |
| T₃P | Propyl Phosphoric Anhydride |
| IPAc | Isopropyl Acetate |
| IPA | Isopropyl Alcohol |
| PLM | Polarized Light Microscopy |
| onset | Initial value |
| peak | Peak value |
| XRPD | X-ray Powder Diffraction |
| RH | Relative Humidity |
| Angle | Angle |
| Intensity | Intensity |
| Rel. Intensity | Relative Intensity |

### Polymorph

Solids are present either in amorphous or crystalline form. In the case of crystalline form, the molecules are positioned in a three-dimensional lattice. When a compound crystallizes from a solution or slurry, it can crystallize in different spatial arrangements (a property known as "polymorphism phenomenon"), forming crystals with different crystalline forms, which are known as "polymorphs". Different polymorphs of a given substance may differ from each other in one or more physical properties, such as solubility and dissolution rate, true specific gravity, crystal form, packing pattern, flowability and/or solid stability.

### Crystalline form screening

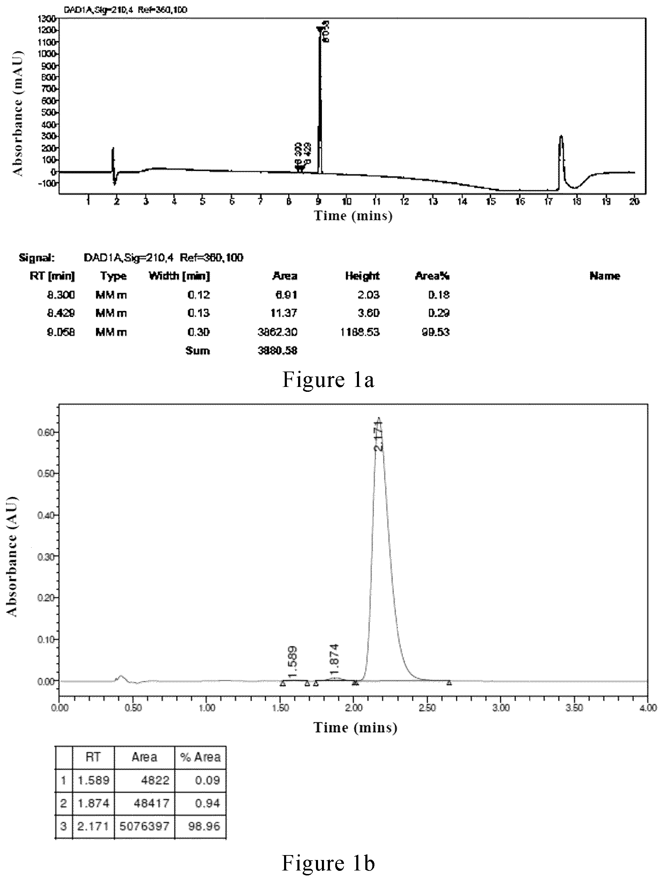

Crystalline form A1 may be used as the starting material, and its chemical purity is shown in Figure 1a, and its chiral purity is shown in Figure 1b. The polymorphism phenomenon of the compound of formula A was investigated by suspension, slow cooling, fast cooling, slow evaporation, fast evaporation, anti-solvent dropwise addition, anti-solvent reverse dropwise addition, vapor diffusion or heating-cooling DSC crystallization method.

The compound of formula A exhibits complex polymorphic behavior, and 13 polymorphs and pseudo-polymorphs were discovered and identified, including two anhydrous crystalline forms, named crystalline form F and crystalline form G. Three hydrates, named crystalline form B, crystalline form E and crystalline form H. Eight solvates, named crystalline form A1, crystalline form A2, crystalline form A3, crystalline form A4, crystalline form A5, crystalline form C, crystalline form D1 and crystalline form D2. In addition, amorphous samples were obtained in solvent systems such as acetone and tetrahydrofuran in this research.

Crystalline form A1, Crystalline form A2, Crystalline form A3, Crystalline form A4 and Crystalline form A5 have similar XRPD patterns, and it is speculated that they have similar crystal structures and are isomorphous solvates of each other. Crystalline form D1 and Crystalline form D2 also have similar XRPD patterns, and it is speculated that they also have similar crystal structures and are another group of isomorphous solvates of each other.

As used herein, the term "room temperature" generally refers to 4-30°C, preferably 20 ± 5°C.

After preparing the polymorph of the compound of formula A, the present invention uses the following multiple methods and instruments to study its properties.

### X-ray Powder Diffraction

Methods for determining the X-ray Powder Diffraction of a crystalline form are known in the art. For example, the patterns were obtained using a Bruker D8 Advance X-ray powder diffractometer with a scanning speed of 0.02 ° per minute and a Cu/K-Alphal (λ=1.5406Å) radiation target.

As is well known in the art, due to experimental variability, when measuring X-ray diffraction patterns on different instruments, if two θ (2θ) values differ within 0.2° (i.e., ± 0.2°), the positions of the peaks are assumed to be the same. For example, the United States Pharmacopeia states that if the angle settings of the 10 strongest diffraction peaks differ from the angle settings of the reference material within ± 0.2°, and the relative intensity of the peaks does not change by more than 20%, then the positions are confirmed to be the same. Therefore, it is assumed that the peak positions within 0.2° of the positions listed herein are the same. Unless otherwise indicated, all X-ray diffraction angles listed herein are based on a copper K-α source.

### Differential Scanning Calorimetry

Also known as "Differential Scanning Calorimetry" (DSC), it is a technique that measures the relationship between the energy difference of the test material relative to a reference material and the temperature during the heating process. The peak position, shape and number of peaks on the DSC spectrum are related to the properties of the substance, so it may be used to qualitatively identify the substance. This method is commonly used in the art to detect various parameters such as the phase transition temperature, glass transition temperature, reaction heat, etc. of the substance.

The DSC measurement method is known in the art. For example, a TA Discovery 2500 differential scanning calorimeter may be used to obtain a DSC scanning pattern of a crystal form by increasing the temperature from 0 °C to 250 °C at a heating rate of 10 °C per minute.

### Nuclear Magnetic Resonance

Nuclear magnetic resonance (NMR) can also be used to assist in determining the crystal structure, and its determination method is known in the art. The present invention preferably uses Bruker Avance-AV-400 MHz.

### Measurement of Thermogravimetric Analysis (TGA)

The sample (2-5 mg) may be placed on an aluminum pan and the test is run as follows:
The sample is heated from room temperature to 300 °C at a rate of 10 °C/min under atmospheric conditions. If the weight loss of the sample exceeds 20%, the test is completed.

### Measurement of Polarized Light Microscopy (PLM)

- Nikon LV100POL is equipped with a 5-megapixel CCD
- Physical lens: 10x ~ 50x.

### Measurement of Dynamic Vapor Sorption (DVS)

- DVS Intrinsic Dynamic Vapor Sorption Instrument
- Gas flow rate 200 sccm, furnace temperature 25 °C.

### Crystalline forms of the present invention

As used herein, the term "crystalline form of the present invention" comprises an amorphous form or a crystalline form of the compound of formula A or a pharmaceutically acceptable salt or a solvate thereof.

Preferably, the present invention includes but is not limited to crystalline form A1, crystalline form A2, crystalline form A3, crystalline form A4, crystalline form A5, crystalline form B, crystalline form C, crystalline form D1, crystalline form D2, crystalline form E, crystalline form F, crystalline form G, and crystalline form H.

### Amorphous form

"Amorphism" or "amorphous form" refers to a substance formed when the mass points (molecules, atoms, ions) of a substance are arranged without periodicity in three-dimensional space, characterized by a diffuse X-ray powder diffraction pattern without sharp peaks. Amorphism is a special physical form of solid substance, and its locally ordered structural features suggest that it is inextricably linked to crystalline substances.

The amorphous form of the present invention preferably has an XRPD pattern as shown in Figure 14 a, and optionally has one or more of the following features:
1) having a glass transition temperature at 129.30 °C ± 2.0 °C in mDSC pattern;
2) having a weight loss of 2.7 wt% before reaching 210 °C ± 2.0 °C in TGA pattern;
3) having a mDSC pattern substantially as shown in Figure 14b; and/ or
4) having a TGA pattern substantially as shown in Figure 14c.

### Preparation method of the crystalline form or amorphous form of the present invention

The present invention provides a method for preparing a crystalline form or an amorphous form of the compound of formula A or a pharmaceutically acceptable salt or solvate thereof, comprising the steps of: obtaining the crystalline form or amorphous form by subjecting the compound of formula A or a pharmaceutically acceptable salt thereof to suspend, slowly cool, rapidly cool, slowly volatilize, rapidly volatilize, add an antisolvent dropwise, add an antisolvent dropwise reversely, vapor diffusion or heat-cool DSC crystallization method, or spray dry, hot melt extrusion or solvent evaporation.

In the preparation method, the compound of formula A is synthesized in the laboratory as described in the specific embodiment. The solvent may be a commonly used solvent in the laboratory, for example, one or more selected from water, alkane solvents, alcohol solvents, ketone solvents, ester solvents, aromatic hydrocarbon solvents, halogenated hydrocarbon solvents, nitrile solvents, ether solvents, aliphatic hydrocarbon solvents, polar aprotic solvents such as N,N-dimethylformamide (DMF), dimethyl sulfoxide (DMSO). The weight to volume ratio of the compound of formula A to the solvent may be 100 mg: (0.1~1 mL).

In one embodiment, the present invention provides a method for preparing a crystalline form of a solvate of the compound of formula A, comprising the steps of mixing the compound of formula A with a solvent corresponding to the solvate, separating the resulting solid and drying it, thereby obtaining a solvate crystalline form of the compound of formula A.

Preferably, the preparation method of crystalline form A1 comprises: mixing and slurrying the compound of formula A with MTBE, separating, and drying to obtain crystalline form A1.

Preferably, the preparation method of crystalline form A2 comprises: suspending crystalline form A1 with 1,4-dioxane, and separating to obtain crystalline form A2.

Preferably, the preparation method of crystalline form A3 comprises: suspending crystalline form A1 with toluene, and separating to obtain crystalline form A3.

Preferably, the preparation method of crystalline form A4 comprises: mixing crystalline form A1 with dichloromethane, and rapidly volatilizing the mixture to obtain crystalline form A4.

Preferably, the preparation method of crystalline form A5 comprises: suspending crystalline form A1 with ethyl acetate, and separating to obtain crystalline form A5.

Preferably, the preparation method of crystalline form B comprises: suspending crystalline form A1 with ethanol, separating, and drying to obtain crystalline form B.

Preferably, the preparation method of crystalline form C comprises: suspending crystalline form A1 with dimethyl sulfoxide, and separating to obtain crystalline form C.

Preferably, the preparation method of crystalline form D1 comprises: suspending crystalline form A1 with isopropanol, and separating to obtain crystalline form D1.

Preferably, the preparation method of crystalline form D2 comprises: suspending crystalline form A1 with a mixed solution (DMSO: water = 24: 76, v/v), and separating to obtain crystalline form D2.

Preferably, the preparation method of crystalline form E comprises: mixing crystalline form A1 with a mixed solution of acetonitrile/water (v:v=80:20), heating-cooling, separating, and drying to obtain crystalline form E.

Preferably, the preparation method of crystalline form F comprises: suspending crystalline form A1 with water, and separating to obtain crystalline form F.

Preferably, the preparation method of crystalline form H comprises: mixing the crystalline form E with methanol, heating-cooling, and separating to obtain the crystalline form H.

In some preferred embodiments, the solvent corresponding to the solvate is, for example but not limited to, 1,4-dioxane, ethyl acetate, toluene, chloroform, 2-methyltetrahydrofuran, methyl tert-butyl ether, acetone, N,N-dimethylformamide, acetonitrile, and the like.

The solvent used in the amorphous preparation method of the present invention is not particularly limited, and any solvent that can dissolve the starting material to a certain extent and does not affect its properties is included in the present invention. In addition, many similar modifications, equivalent replacements, or solvents, solvent combinations, and different proportions of solvent combinations equivalent to those described in the present invention in the art are considered to be included in the scope of the present invention. The present invention provides preferred solvents used in each reaction step.

In one embodiment, the present invention provides a method for preparing an amorphous form of the compound of formula A, comprising the following steps of: mixing the compound of formula A with a solvent and spray drying the resulting solution, thereby obtaining an amorphous form of the compound of formula A.

In some preferred embodiments, the solvent may be a commonly used solvent in the laboratory, for example: the solvent is one or more selected from water, alcohol solvents, ester solvents, ketone solvents, halogenated hydrocarbon solvents, nitrile solvents and ether solvents, wherein the alcohol solvent is preferably ethanol and/or methanol; the ester solvent is preferably ethyl acetate; the ketone solvent is preferably acetone; the halogenated hydrocarbon solvent is preferably dichloromethane; the nitrile solvent is preferably acetonitrile; the ether solvent is preferably tetrahydrofuran; preferably, the solvent is one or more of ethyl acetate, acetone, tetrahydrofuran, methyl tert-butyl ether and acetonitrile.

Preferably, the separation is performed by centrifugal filtration using a 0.45 µm nylon filter membrane centrifuge tube at a certain speed (such as 14,000 rpm).

### Pharmaceutical composition

Since the crystalline forms and amorphous form of the present invention have excellent effects in treating proliferative diseases, the crystalline form and amorphous form of the present invention and pharmaceutical compositions containing the crystalline form and amorphous form of the present invention as main active ingredients may be used to treat, prevent and alleviate proliferative diseases. According to the prior art, the form described in the present invention may be used to treat the following diseases: cancer, myeloproliferative and inflammatory diseases, etc.

The pharmaceutical composition of the present invention comprises a safe and effective amount of the crystalline form or amorphous form of the present invention and a pharmaceutically acceptable excipient and/or carrier.

The crystalline and amorphous forms of the present invention may be administered alone or in combination with other pharmaceutically acceptable compounds.

The pharmaceutical composition may be in a dosage form suitable for human administration, such as tablet, capsule, granule, powder, or pill, etc., preferably tablet, capsule, granule, disintegrating tablet, sustained-release or controlled-release tablet, etc.

The pharmaceutical composition of the present invention may be prepared by various methods well known in the art, which may be prepared by mixing a therapeutically effective amount of one or more crystalline form or amorphous form of the compound of formula A or a salt, a solvate thereof with one or more pharmaceutically acceptable excipients into a dosage form suitable for human administration, such as tablet, capsule, granule, etc.

"Therapeutically effective amount" refers to an amount of the form of the compound according to the present invention that, when administered to a patient in need thereof, is sufficient to achieve treatment of a disease state, condition or disorder for which the compound has efficacy. Such an amount will be sufficient to elicit the biological or medical response sought by a researcher or clinician in a tissue system or patient.

"Safe and effective amount" means: the amount of the compound (polymorph) is sufficient to significantly improve the condition without causing serious side effects. Generally, the pharmaceutical composition contains 0.1-2000 mg/dose of the crystalline form and amorphous form of the present invention, and more preferably, contains 0.1-200 mg/dose of the crystalline form and amorphous form of the present invention. Preferably, the "one dose" is one capsule or tablet.

"Pharmaceutically acceptable carrier" refers to: one or more compatible solid or liquid fillers or gel substances, which are suitable for use in human and must have sufficient purity and sufficiently low toxicity. "Compatibility" used herein means that the components in the composition may be mixed with the active ingredients of the present invention and with each other without significantly reducing the efficacy of the active ingredients. Some examples of pharmaceutically acceptable carriers include cellulose and derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agents (such as sodium lauryl sulfate), colorants, flavoring agents, stabilizers, antioxidants, preservatives, pyrogen-free water, etc.

The administration of the crystalline form, amorphous form or pharmaceutical composition of the present invention is not particularly limited, and representative administration include (but are not limited to): oral, intratumoral, rectal, parenteral (intravenous, intramuscular or subcutaneous), and topical administration.

When using the pharmaceutical composition, a safe and effective amount of the crystalline form and amorphous form of the present invention is applied to a mammal (such as a human) in need of treatment, wherein the dosage during administration is a pharmaceutically effective dosage, and for a person weighing 60 kg, the daily dosage is usually 0.1 to 2000 mg, preferably 0.1 to 200 mg. Of course, the specific dosage should also take into account factors such as the route of administration and the health condition of the patient, which are all within the skill range of skilled physicians.

Solid dosage forms for oral administration include capsules, tablets, pills, powders and granules. In these solid dosage forms, the active ingredient is mixed with at least one conventional inert excipient (or carrier), such as sodium citrate or dicalcium phosphate, or with the following ingredients: (a) fillers or extenders, such as microcrystalline cellulose, starch, lactose, sucrose, glucose, mannitol and silicic acid; (b) binders, such as hydroxymethylcellulose, alginates, gelatin, polyvinyl pyrrolidone, sucrose and acacia; (c) humectants, such as glycerol; (d) disintegrants, such as agar, calcium carbonate, potato starch or tapioca starch, alginic acid, certain complex silicates, sodium carbonate, cross-linked polyvidone, cross-linked sodium carboxymethylcellulose; (e) solubilizers, such as paraffin; (f) absorption accelerators, such as quaternary ammonium compounds; (g) wetting agents, such as cetyl alcohol and glyceryl monostearate; (h) adsorbents, such as kaolin; and (i) lubricants, for example, talc, calcium stearate, magnesium stearate, solid polyethylene glycol, sodium lauryl sulfate, or mixtures thereof. In capsules, tablets and pills, the dosage form may also contain a buffering agent.

Solid dosage forms such as tablets, pills, capsules, pills and granules may be prepared using coatings and shell materials, such as enteric coatings and other materials known in the art. They may contain opacifying agents, and the release of the active ingredient in such compositions may be delayed in a certain part of the digestive tract. Examples of embedding components that may be used are polymeric substances and waxes. If necessary, the active ingredient can also be formed into microcapsules with one or more of the above-mentioned excipients.

Liquid dosage forms for oral administration include pharmaceutically acceptable emulsions, solutions, suspensions, syrups or tinctures. In addition to the active ingredient, the liquid dosage form may contain an inert diluent conventionally used in the art, such as water or other solvents, solubilizers and emulsifiers, for example, ethanol, isopropanol, ethyl carbonate, ethyl acetate, propylene glycol, 1,3-butylene glycol, dimethylformamide and oils, in particular cottonseed oil, peanut oil, corn germ oil, olive oil, castor oil and sesame oil or mixtures of these substances.

In addition to such inert diluents, the composition may also include adjuvants such as wetting agents, emulsifying and suspending agents, sweetening, flavoring, and perfuming agents.

In addition to the active ingredients, suspensions may contain suspending agents such as, for example, ethoxylated isostearyl alcohols, polyoxyethylene sorbitol and sorbitan esters, microcrystalline cellulose, aluminum methanol and agar, or mixtures of these substances.

Compositions for parenteral injection may include physiologically acceptable sterile aqueous or anhydrous solutions, dispersions, suspensions or emulsions, and sterile powders for reconstitution into sterile injectable solutions or dispersions. Suitable aqueous and non-aqueous carriers, diluents, solvents or excipients include water, ethanol, polyols and suitable mixtures thereof.

Dosage forms of the crystalline form and amorphous form of the present invention for topical administration include ointments, powders, patches, sprays and inhalants. The active ingredient is mixed under sterile conditions with a physiologically acceptable carrier and any preservative, buffer, or propellant that may be required if necessary.

### The main advantages of the present invention include:

1. Provided are a series of novel crystalline forms or amorphous form of *N-*((*S*)-(5-chloropyridin-2-yl)(cyclobutyl)methyl)-2-((*S*)-2,6-dioxopiperidin-3-yl)-1-oxoisoindoline-5-carboxamide or a salt or solvate thereof.
2. The obtained amorphous form or crystalline form has excellent stability, solubility and bioavailability.

### Example 1 Preparation of crystalline form A1

Preparation method: the compound of formula A was prepared by referring to the method of Example 15 of PCT/CN2022/097236, and then was slurried in MTBE to give crystalline form A1.

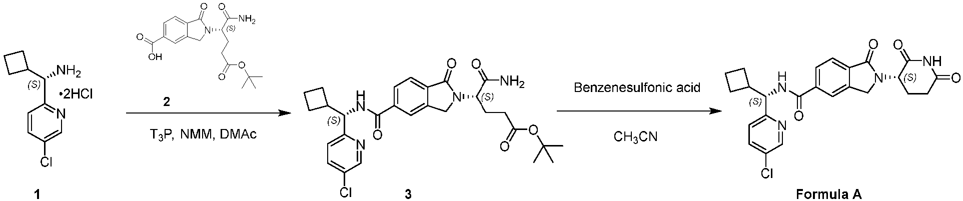

Compound **1** (3.25 g, 12.3 mmol, hydrochloride), compound 2 (4.67 g, 12.9 mmol), NMM (6.2 g, 61.3 mmol) and T₃P (5.6 g, 17.6 mmol) were added into a bottom flask containing dimethylacetamide (DMAc) (50 mL). The mixture was degassed and purged with N₂ three times. The mixture was stirred at 25 °C for 12 hours under N₂. The mixture was poured into saturated aqueous sodium chloride solution (100 mL), then filtered and washed with water (100 mL). The filter cake was then dissolved in DCM (200 mL) and washed with saturated NaHCO₃. The organic phase was dried with anhydrous Na₂SO₄, filtered under reduced pressure and concentrated to give compound **3 (6.5 g,** yield 99.7% based on compound 1) as a white solid.

Compound **3** (6.5 g) was dissolved in CH₃CN (13 mL) and added with benzenesulfonic acid (1.1 g, 6.95 mmol). The mixture was stirred at 70°C for 14 hours under N₂, and the mixture was diluted with DCM (60 mL), washed with saturated NaHCO₃ aqueous solution (30 mL x 2), and then washed with H₂O (30 mL x 2). The organic phase was dried with anhydrous Na₂SO₄, filtered and concentrated under reduced pressure to give a crude product. The crude product was then slurried with EA (5 mL) and MTBE (5 mL) at 25 °C for 0.5 h to give the compound of formula A (4.9 g), white solid, with a yield of 87% based on compound **1.** The chemical name of the compound of formula A is *N*-((*S*)-(5-chloropyridin-2-yl)(cyclobutyl)methyl)-2-((*S*)-2,6-dioxopiperidin-3-yl)-1-oxoisoindoline-5-carboxamide.

The compound of formula A (4.9 g) was slurried in MTBE (25 ml) at room temperature for 24 h, filtered and dried to obtain 4.41 g of a white solid with a yield of 90%. It was identified as crystalline form A1 by XRPD (Figure 1c).

Its chemical purity spectrum is shown in Figure 1a, the chiral purity spectrum is shown in Figure 1b, the X-ray powder diffraction pattern is shown in Figure 1c, and the parameters of each peak are shown in Table 1, the differential scanning calorimetry (DSC) pattern is shown in Figure 1d, the thermogravimetric analysis pattern is shown in Figure 1e, and the ¹H NMR spectrum is shown in Figure 1f.

**Table 1**

| Index | Angle | Intensity | Rel. Intensity |
|---|---|---|---|
| 1 | 3.263 ° | 265.832 | 13.2% |
| 2 | 6.548 ° | 786.273 | 100.0% |
| 3 | 9.217 ° | 583.802 | 70.2% |
| 4 | 9.797 ° | 347.535 | 36.7% |
| 5 | 10.833° | 135.383 | 7.2% |
| 6 | 13.062 ° | 285.795 | 28.0% |
| 7 | 15.687 ° | 264.759 | 22.4% |
| 8 | 16.355 ° | 548.609 | 61.3% |
| 9 | 17.238 ° | 353.582 | 32.6% |
| 10 | 17.532 ° | 350.953 | 31.6% |
| 11 | 18.487 ° | 546.661 | 57.9% |
| 12 | 19.640 ° | 773.791 | 89.5% |
| 13 | 20.137 ° | 224.539 | 12.6% |
| 14 | 21.100 ° | 239.25 | 15.7% |
| 15 | 21.244 ° | 236.479 | 15.5% |
| 16 | 21.723 ° | 156.74 | 5.1% |
| 17 | 22.503 ° | 232.72 | 17.0% |
| 18 | 22.876 ° | 234.013 | 17.8% |
| 19 | 24.673 ° | 317.436 | 28.4% |
| 20 | 25.066 ° | 326.442 | 29.3% |
| 21 | 26.225 ° | 243.521 | 17.5% |
| 22 | 27.442 ° | 267.3 | 22.0% |
| 23 | 28.980 ° | 122.264 | 3.4% |
| 24 | 29.169 ° | 151.168 | 7.7% |
| 25 | 31.556 ° | 277.499 | 24.5% |
| 26 | 35.036 ° | 120.866 | 3.1% |
| 27 | 37.471 ° | 1146.855 | 7.2% |

### Example 2 Preparation of crystalline form A2

Preparation method. About 40 mg of crystalline form A1 was weighed, and 0.2-1 mL of 1,4-dioxane was added, and the mixture was suspended with stirring at 25 °C and 300 rpm for 2 weeks. The resulting suspension was centrifuged and filtered at 14,000 rpm using a 0.45 µm nylon filter centrifuge tube to obtain crystalline form A2.

Its X-ray powder diffraction pattern is shown in Figure 2a, and the parameters of each peak are shown in Table 2, the differential scanning calorimetry (DSC) pattern is shown in Figure 2b, the thermogravimetric analysis pattern is shown in Figure 2c and the ¹H NMR spectrum is shown in Figure 2d.

**Table 2**

| Index | Angle | Intensity | Rel. Intensity |
|---|---|---|---|
| 1 | 3.285 ° | 324.214 | 11.5% |
| 2 | 6.564 ° | 583.794 | 30.5% |
| 3 | 9.287 ° | 923.068 | 50.1% |
| 4 | 9.828 ° | 407.678 | 19.5% |
| 5 | 10.876 ° | 308.096 | 13.6% |
| 6 | 13.118 ° | 624.252 | 32.2% |
| 7 | 15.723 ° | 449.546 | 21.9% |
| 8 | 16.383 ° | 963.664 | 51.9% |
| 9 | 17.387 ° | 789.735 | 41.1% |
| 10 | 17.694 ° | 429.127 | 19.5% |
| 11 | 18.069 ° | 141.442 | 2.3% |
| 12 | 18.579 ° | 1794.86 | 100.0% |
| 13 | 19.267 ° | 225.12 | 7.1% |
| 14 | 19.676 ° | 1262.25 | 68.6% |
| 15 | 20.122 ° | 473.809 | 22.2% |
| 16 | 20.886 ° | 116.081 | 1.3% |
| 17 | 21.179 ° | 547.507 | 26.9% |
| 18 | 21.801 ° | 233.406 | 8.5% |
| 19 | 122.632 ° | 559.87 | 28.1% |
| 20 | 22.987 ° | 194.161 | 6.4% |
| 21 | 23.962 ° | 164.277 | 4.4% |
| 22 | 24.610 ° | 389.116 | 17.4% |
| 23 | 24.757 ° | 470.533 | 22.1% |
| 24 | 25.185 ° | 652.793 | 32.9% |
| 25 | 26.060 ° | 296.637 | 11.8% |
| 26 | 26.358 ° | 329.123 | 13.7% |
| 27 | 26.608 ° | 220.216 | 7.3% |
| 28 | 27.299 ° | 159.418 | 4.0% |
| 29 | 27.619 ° | 361.905 | 16.2% |
| 30 | 128.016 ° | 153.524 | 4.2% |
| 31 | 28.998 ° | 99.3357 | 1.2% |
| 32 | 29.351 ° | 274.275 | 11.5% |
| 33 | 30.301 ° | 121.754 | 2.3% |
| 34 | 30.834 ° | 106.824 | 1.2% |
| 35 | 31.666 ° | 552.308 | 27.2% |
| 36 | 31.988 ° | 164.339 | 4.2% |
| 37 | 32.722 ° | 162.666 | 4.1% |
| 38 | 32.933 ° | 192.538 | 5.9% |
| 39 | 33.166 ° | 157.172 | 3.9% |
| 40 | 34.270 ° | 111.997 | 11.4% |
| 41 | 35.096 ° | 181.867 | 5.6% |
| 42 | 36.542 ° | 197.133 | 6.7% |
| 43 | 36.563 ° | 157.907 | 14.4% |
| 44 | 36.999 ° | 122.046 | 2.1% |
| 45 | 37.543 ° | 189.35 | 6.2% |
| 46 | 38.446 ° | 113.449 | 1.6% |
| 47 | 39.904 ° | 244.172 | 3.2% |

### Example 3 Preparation of crystalline form A3

Preparation method. About 40 mg of crystalline form A1 was weighed, and 0.2-1 mL of toluene was added, and the mixture was suspended with stirring at 300 rpm and 25 °C for 2 weeks. The resulting suspension was centrifuged and filtered at 14,000 rpm using a 0.45 µm nylon filter centrifuge tube to obtain crystalline form A3.

Its X-ray powder diffraction pattern is shown in Figure 3a, and the parameters of each peak are shown in Table 3, the differential scanning calorimetry (DSC) pattern is shown in Figure 3b, the thermogravimetric analysis pattern is shown in Figure 3c, and the ¹H NMR spectrum is shown in Figure 3d.

**Table 3**

| Index | Angle | Intensity | Rel. Intensity |
|---|---|---|---|
| 1 | 3.273 ° | 216.139 | 10.6% |
| 2 | 6.546 ° | 669.056 | 64.9% |
| 3 | 9.220 ° | 843.144 | 82.9% |
| 4 | 9.802 ° | 398.84 | 35.3% |
| 5 | 10.813 ° | 329.24 | 127.8% |
| 6 | 12.570 ° | 85.9328 | 1.9% |
| 7 | 13.051 ° | 372.198 | 32.4% |
| 8 | 15.651 ° | 318.097 | 26.6% |
| 9 | 16.332 ° | 761.577 | 74.2% |
| 10 | 17.243 ° | 405.083 | 35.8% |
| 11 | 17.550 ° | 293.501 | 23.4% |
| 12 | 18.458 ° | 998.066 | 98.1% |
| 13 | 18.942 ° | 121.497 | 3.9% |
| 14 | 19612 ° | 1018.29 | 100.0% |
| 15 | 19.853 ° | 181.844 | 10.4% |
| 16 | 20.193 ° | 281.808 | 121.3% |
| 17 | 20.437 ° | 114.124 | 3.5% |
| 18 | 20.849 ° | 122.084 | 4.8% |
| 19 | 21.343 ° | 162.29 | 9.3% |
| 20 | 21.668 ° | 124.884 | 5.3% |
| 21 | 22.248 ° | 195.438 | 13.0% |
| 22 | 22.913 ° | 210.459 | 14.9% |
| 23 | 24.042 ° | 113.894 | 4.2% |
| 24 | 24.580 ° | 242.769 | 17.5% |
| 25 | 24.915 ° | 373.519 | 31.3% |
| 26 | 25.706 ° | 140.247 | 6.2% |
| 27 | 26.170 ° | 335.118 | 27.2% |
| 28 | 27.086 ° | 232.238 | 16.7% |
| 29 | 27.416 ° | 145.74 | 7.5% |
| 30 | 27.793 ° | 159.028 | 9.3% |
| 31 | 28.084 ° | 107.172 | 4.0% |
| 32 | 28.787 ° | 91.7793 | 2.9% |
| 33 | 29.137 ° | 166.444 | 10.8% |
| 34 | 29.675 ° | 95.3432 | 3.1% |
| 35 | 30.950 ° | 107.785 | 4.4% |
| 36 | 31.458 ° | 235.675 | 17.7% |
| 37 | 31.912 ° | 105.23 | 3.6% |
| 38 | 32.703 ° | 154.353 | 8.6% |
| 39 | 33.612 ° | 118.412 | 5.0% |
| 40 | 34.507 ° | 104.555 | 3.7% |
| 41 | 35.063 ° | 106.2 | 3.9% |
| 42 | 36.285 ° | 111.087 | 4.5% |
| 43 | 37.404 ° | 144.34 | 7.6% |
| 44 | 39.706 ° | 129.602 | 6.0% |

### Example 4 Preparation of crystalline form A4

Preparation method. About 30 mg of crystalline form A1 was weighed, and 5 mL of DCM was added to fully dissolve, and the mixture was filtered using a 0.45 µm nylon filter syringe filter to obtain a clear solution. The obtained clear solution was purged with nitrogen at room temperature to quickly evaporate the solvent. The solvent was evaporated and the solid obtained was collected to obtain crystalline form A4.

Its X-ray powder diffraction pattern is shown in Figure 4a, and the parameters of each peak are shown in Table 4, the differential scanning calorimetry (DSC) pattern is shown in Figure 4b, the thermogravimetric analysis pattern is shown in Figure 4c, and the ¹H NMR spectrum is shown in Figure 4d.

**Table 4**

| Index | Angle | Intensity | Rel. Intensity |
|---|---|---|---|
| 1 | 3.275 ° | 74.8549 | 25.9% |
| 2 | 6.548 ° | 147.037 | 81.2% |
| 3 | 9.225 ° | 192.479 | 100.0% |
| 4 | 9.809 ° | 118.394 | 28.6% |
| 5 | 16.379 ° | 192.229 | 61.7% |
| 6 | 17.510 ° | 239.22 | 94.5% |
| 7 | 18.514 ° | 206.525 | 60.5% |
| 8 | 19.059 ° | 163.093 | 20.3% |
| 9 | 19.591 ° | 227.215 | 77.0% |
| 10 | 21.170 ° | 167.727 | 27.4% |
| 11 | 22.486 ° | 1167.613 | 133.3% |
| 12 | 24.641 ° | 184.115 | 151.7% |
| 13 | 25.036 ° | 202.345 | 68.1% |
| 14 | 31.539 ° | 128.091 | 36.4% |

### Example 5 Preparation of crystalline form A5

Preparation method. About 60 mg of crystalline form A1 was weighed, and 0.2-1 mL of ethyl acetate was added, and the mixture was stirred at 50 °C and 300 rpm for 1 week. The resulting suspension was centrifuged and filtered at 14,000 rpm using a 0.45 µm nylon filter centrifuge tube to obtain crystalline form A5.

Its X-ray powder diffraction pattern is shown in Figure 5a, the parameters of each peak are shown in Table 5, the differential scanning calorimetry (DSC) pattern is shown in Figure 5b, the thermogravimetric analysis pattern is shown in Figure 5c, and the ¹H NMR spectrum is shown in Figure 5d.

**Table 5**

| Index | Angle | Intensity | Rel. Intensity |
|---|---|---|---|
| 1 | 6.589 ° | 289.596 | 85.7% |
| 2 | 8.030 ° | 127.435 | 18.2% |
| 3 | 9.272 ° | 297.6 | 79.9% |
| 4 | 9.889 ° | 199.759 | 40.9% |
| 5 | 13.139 ° | 153.748 | 18.0% |
| 6 | 14.222 ° | 128.837 | 10.5% |
| 7 | 15.804 ° | 182.386 | 26.3% |
| 8 | 16.430 ° | 385.534 | 100.0% |
| 9 | 17.370 ° | 262.044 | 145.8% |
| 10 | 17.590° | 299.516 | 59.1% |
| 11 | 18.531 ° | 354.582 | 76.1% |
| 12 | 19.744 ° | 424.384 | 100.0% |
| 13 | 23.062 ° | \|222.304 | 26.7% |
| 14 | 24.719 ° | 217.249 | 22.8% |
| 15 | 25.119 ° | 206.485 | 18.0% |
| 16 | 27.532 ° | 192.727 | 15.0% |
| 17 | 31.605 ° | 184.447 | 24.5% |

### Example 6 Preparation of crystalline form B

Preparation method. 410 mg of crystalline Form A1 was weighed and placed in an 8 mL glass bottle. 5 mL of ethanol was added and the mixture was stirred at 25 °C to obtain a suspension. About 5 mg of Form B seed crystal was added to the above suspension, and the mixture was stirred at 25°C for about 5 days. The solid was collected by filtration, and the obtained solid was dried in vacuum at 50 °C for about 8 hours and at 25 °C for about 3 hours. About 350 mg of Form B off-white powder was prepared, and the yield was about 87%.

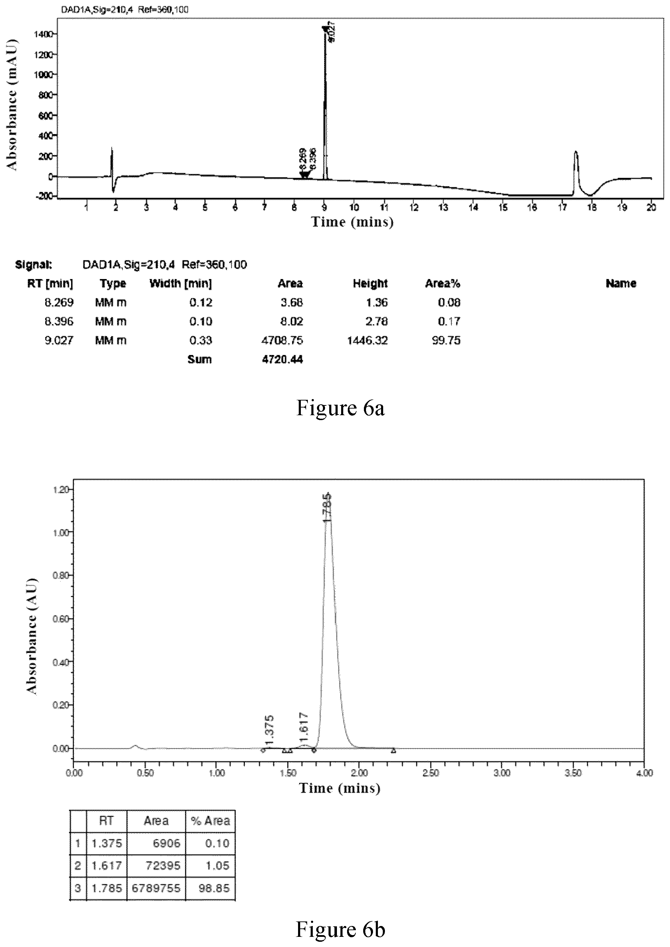

Its chemical purity is shown in Figure 6a, the chiral purity is shown in Figure 6b, the X-ray powder diffraction pattern is shown in Figure 6c, the parameters of each peak are shown in Table 6, the differential scanning calorimetry (DSC) pattern is shown in Figure 6d, the thermogravimetric analysis pattern is shown in Figure 6e, and the ¹H NMR spectrum is shown in Figure 6f.

**Table 6**

| Index | Angle | Intensity | Rel. Intensity |
|---|---|---|---|
| 1 | 3.980 ° | 738.592 | 100.0% |
| 2 | 5.255 ° | 204.407 | 19.7% |
| 3 | 6.868 ° | 251.308 | 27.8% |
| 4 | 7.193 ° | 122.394 | 7.4% |
| 5 | 7.926 ° | 523.18 | 69.3% |
| 6 | 8.623 ° | 495.923 | 65.2% |
| 7 | 9.111 ° | 274.148 | 31.2% |
| 8 | 10.518 ° | 328.353 | 38.0% |
| 9 | 11.108 ° | 377.686 | 44.8% |
| 10 | 12.057 ° | 697.594 | 93.8% |
| 11 | 12.357 ° | 716.292 | 96.6% |
| 12 | 13.057 ° | 244.358 | 23.6% |
| 13 | 13.732 ° | 257.976 | 26.1% |
| 14 | 14.320 ° | 512.673 | 66.2% |
| 15 | 15.069 ° | 188.075 | 16.6% |
| 16 | 15.780 ° | 509.646 | 66.7% |
| 17 | 16.196 ° | 199.737 | 18.9% |
| 18 | 17.032 ° | 121.533 | 6.8% |
| 19 | 17.317 ° | 218.754 | 121.5% |
| 20 | 18.259 ° | 393.42 | 48.0% |
| 21 | 19.099 ° | 549.835 | 72.5% |
| 22 | 19.622° | 476.19 | 61.6% |
| 23 | 19.796 ° | 397.212 | 49.6% |
| 24 | 20.606 ° | 112.485 | 6.5% |
| 25 | 21.023 ° | 182.181 | 17.7% |
| 26 | 21.197 ° | 143.221 | 11.8% |
| 27 | 21.681 ° | 91.3707 | 3.9% |
| 28 | 22.381 ° | 194.785 | 19.5% |
| 29 | 22.611 ° | 220.275 | 23.3% |
| 30 | 23.525 ° | 141.68 | 11.1% |
| 31 | 24.238 ° | 130.074 | 9.9% |
| 32 | 24.824 ° | 107.332 | 6.7% |
| 33 | 25.026 ° | 145.034 | 12.5% |
| 34 | 25.933 ° | 185.825 | 19.0% |
| 35 | 26.392 ° | 98.6315 | 5.6% |
| 36 | 26.754 ° | 122.012 | 9.6% |
| 37 | 27.965 ° | 100.238 | 6.6% |
| 38 | 28.719 ° | 109.597 | 7.9% |
| 39 | 30.720 ° | 85.0549 | 3.5% |
| 40 | 31.228° | 137.216 | 11.3% |
| 41 | 31.551 ° | 91.5736 | 4.1% |
| 42 | 32.132 ° | 93.4716 | 4.6% |
| 43 | 33.654 ° | 73.8956 | 2.9% |
| 44 | 34.217 ° | 75.3033 | 3.5% |

### Example 7 Preparation of crystalline form C

Preparation method. About 40 mg of crystalline form A1 was weighed, 0.2-1 mL of DMSO was added, and the mixture was suspended with stirring at 25 °C and 300 rpm for 2 weeks. The resulting suspension was centrifuged and filtered at 14,000 rpm using a 0.45 µm nylon filter centrifuge tube to obtain crystalline form C.

Its X-ray powder diffraction pattern is shown in Figure 7a, the parameters of each peak are shown in Table 7, the differential scanning calorimetry (DSC) pattern is shown in Figure 7b, the thermogravimetric analysis pattern is shown in Figure 7c, and the ¹H NMR spectrum is shown in Figure 7d.

**Table 7**

| Index | Angle | Intensity | Rel. Intensity |
|---|---|---|---|
| 1 | 6.003 ° | 137.809 | 3.6% |
| 2 | 8.975° | 193.481 | 6.2% |
| 3 | 9.183 ° | 1107.904 | 2.3% |
| 4 | 9.621 ° | 118.055 | 2.7% |
| 5 | 10.354 ° | 95.7835 | 1.6% |
| 6 | 11.104 ° | 966.108 | 41.7% |
| 7 | 11.953 ° | 716.162 | 30.1% |
| 8 | 13.110 ° | 1198.11 | 52.4% |
| 9 | 14.357 ° | 113.888 | 2.8% |
| 10 | 15.440° | 171.301 | 5.7% |
| 11 | 16.811 ° | 82.4975 | 1.5% |
| 12 | 17.941 ° | 1501.19 | 66.2% |
| 13 | 18.306 ° | 1582257 | 23.5% |
| 14 | 19.244 ° | 540.03 | 21.2% |
| 15 | 19.426 ° | 1035.74 | 44.1% |
| 16 | 19.992 ° | 106.812 | 1.2% |
| 17 | 20.606 ° | 2247.29 | 100.0% |
| 18 | 21.174 ° | 100.313 | 1.0% |
| 19 | 21.545 ° | 123.426 | 2.3% |
| 20 | 21.805° | 95.3241 | 1.1% |
| 21 | 22.157 ° | 155.752 | 4.0% |
| 22 | 22.867 ° | 104.101 | 1.7% |
| 23 | 23.408 ° | 219.072 | 6.8% |
| 24 | 23.970 ° | 987.271 | 42.2% |
| 25 | 24.248 ° | 99.374 | 1.2% |
| 26 | 24.954 ° | 212.46 | 6.5% |
| 27 | 25.357 ° | 152.189 | 3.6% |
| 28 | 26.314 ° | 933.192 | 39.3% |
| 29 | 27.023 ° | 533.026 | 20.6% |
| 30 | 27.788 ° | 173.402 | 4.2% |
| 31 | 27.971 ° | 257.6 | 8.1% |
| 32 | 28.554 ° | 157.91 | 3.8% |
| 33 | 29.622 ° | 98.9549 | 1.2% |
| 34 | 30.189 ° | 107.021 | 1.6% |
| 35 | 30.729 ° | 204.806 | 6.1% |
| 36 | 31.115 ° | 141.78 | 3.2% |
| 37 | 31.740 ° | 114.936 | 2.0% |
| 38 | 32.134 ° | 85.671 | 0.8% |
| 39 | 33.138 ° | 149.303 | 3.5% |
| 40 | 33.614 ° | 94.5579 | 0.9% |
| 41 | 35.065 ° | 146.555 | 3.1% |
| 42 | 36.311 ° | 107.151 | 1.3% |
| 43 | 36.681 ° | 184.799 | 5.0% |
| 44 | 37.605 ° | 140.555 | 3.1% |
| 45 | 38.111 ° | 136.83 | 2.7% |
| 46 | 39.059 ° | 162.956 | 3.8% |
| 47 | 39.825 ° | 119.636 | 1.2% |

### Example 8 Preparation of crystalline form D1

Preparation method. About 40 mg of crystalline form A1 was weighed, and 0.2-1 mL of IPA was added, and the mixture was suspended with stirring at 25 °C and 300 rpm for 2 weeks. The resulting suspension was centrifuged and filtered at 14,000 rpm using a 0.45 µm nylon filter centrifuge tube to obtain crystalline form D1.

Its X-ray powder diffraction pattern is shown in Figure 8a, the parameters of each peak are shown in Table 8, the differential scanning calorimetry (DSC) pattern is shown in Figure 8b, the thermogravimetric analysis pattern is shown in Figure 8c, and the ¹H NMR spectrum is shown in Figure 8d.

**Table 8**

| Index | Angle | Intensity | Rel. Intensity |
|---|---|---|---|
| 1 | 3.283 ° | 157.732 | 1.9% |
| 2 | 6.572 ° | 387.739 | 9.6% |
| 3 | 8.827 ° | 3337.38 | 96.5% |
| 4 | 9.263 ° | 1379.71 | 38.5% |
| 5 | 9.955 ° | 1092.28 | 30.0% |
| 6 | 10.859 ° | 215.017 | 4.1% |
| 7 | 14.358 ° | 631.665 | 16.5% |
| 8 | 15.693 ° | 193.573 | 3.5% |
| 9 | 16.374 ° | 283.937 | 5.9% |
| 10 | 17.324 ° | 2899.12 | 82.8% |
| 11 | 17.612 ° | 656.795 | 16.3% |
| 12 | 17.991 ° | 3485.93 | 100.0% |
| 13 | 18.507 ° | 743.752 | 18.8% |
| 14 | 19.149 ° | 1315.22 | 35.8% |
| 15 | 19.645° | 386.619 | 8.6% |
| 16 | 20.585 ° | 157.054 | 2.3% |
| 17 | 21.177 ° | 202.178 | 3.7% |
| 18 | 21.859 ° | 210.223 | 4.0% |
| 19 | 22.659 ° | 155.342 | 2.4% |
| 20 | 22.976 ° | 126.824 | 1.5% |
| 21 | 23.530 ° | 102.439 | 0.6% |
| 22 | 23.857 ° | 102.269 | 0.6% |
| 23 | 24.653 ° | 270.25 | 5.2% |
| 24 | 24.876 ° | 369.714 | 8.1% |
| 25 | 25.138 ° | 402.791 | 9.0% |
| 26 | 25.523 ° | 304.884 | 6.0% |
| 27 | 26.092 ° | 491.584 | 11.5% |
| 28 | 26.519 ° | 577.703 | 14.1% |
| 29 | 27.868 ° | 292.978 | 6.0% |
| 30 | 28.503 ° | 190.294 | 3.0% |
| 31 | 29.214 ° | 557.815 | 13.8% |
| 32 | 30.023 ° | 162.067 | 2.0% |
| 33 | 30.217 ° | 158.007 | 1.8% |
| 34 | 31.537 ° | 718.526 | 18.2% |
| 35 | 31.824 ° | 292.53 | 5.5% |
| 36 | 32.595 ° | 136.1 | 1.0% |
| 37 | 32.778 ° | 229.837 | 3.8% |
| 38 | 33.035 ° | 155.219 | 1.7% |
| 39 | 33.603 ° | 117.545 | 0.8% |
| 40 | 33.889 ° | 118.053 | 0.8% |
| 41 | 34.225 ° | 109.361 | 0.5% |
| 42 | 34.913 ° | 532.114 | 12.9% |
| 43 | 35.560 ° | 137.994 | 1.4% |
| 44 | 36.337 ° | 230.035 | 4.2% |
| 45 | 36.829 ° | 174.24 | 2.5% |
| 46 | 37.447 ° | 120.306 | 1.0% |
| 47 | 38.747 ° | 120.562 | 0.8% |
| 48 | 39.866 ° | 199.184 | 2.0% |

### Example 9 Preparation of crystalline form D2

Preparation method. About 40 mg of crystalline form A1 was weighed, and 0.2-1 mL of a mixed solution (DMSO: water = 24:76, v/v) was added, and the mixture was suspended with stirring at 25 °C and 300 rpm for 2 weeks. The resulting suspension was centrifuged and filtered at 14,000 rpm using a 0.45 µm nylon filter centrifuge tube to obtain crystalline form D2.

Its X-ray powder diffraction pattern is shown in Figure 9a, the parameters of each peak are shown in Table 9, the differential scanning calorimetry (DSC) pattern is shown in Figure 9b, the thermogravimetric analysis pattern is shown in Figure 9c, and the ¹H NMR spectrum is shown in Figure 9d.

**Table 9**

| Index | Angle | Intensity | Rel. Intensity |
|---|---|---|---|
| 1 | 3.278 ° | 143.79 | 6.5% |
| 2 | 6.550 ° | 266.267 | 37.4% |
| 3 | 8.806 ° | 216.663 | 26.7% |
| 4 | 9.252 ° | 199.82 | 23.1% |
| 5 | 9.806 ° | 330.932 | 46.0% |
| 6 | 10.828 ° | 107.527 | 6.6% |
| 7 | 13.067 ° | 112.021 | 5.9% |
| 8 | 14.362 ° | 158.266 | 13.9% |
| 9 | 16.339 ° | 161.615 | 15.3% |
| 10 | 17.384 ° | 654.666 | 100.0% |
| 11 | 17.996 ° | 581.611 | 85.3% |
| 12 | 18.519 ° | 271.771 | 29.0% |
| 13 | 19.151 ° | 288.651 | 31.0% |
| 14 | 19.573 ° | 569.078 | 80.3% |
| 15 | 20.016 ° | 149.399 | 5.5% |
| 16 | 20.553 ' | 257.543 | 24.7% |
| 17 | 21.131 ° | 242.818 | 22.4% |
| 18 | 21.818° | 294.712 | 32.4% |
| 19 | 22.590 ° | 146.784 | 6.3% |
| 20 | 22.904 ° | 161.374 | 8.5% |
| 21 | 23.456 ° | 162,708 | 8.0% |
| 22 | 24.838 ° | 319.096 | 33.9% |
| 23 | 25.057 ° | 349.065 | 39.1% |
| 24 | 25.475 ° | 278.68 | 26.6% |
| 25 | 26.536 ° | 229.539 | 18.6% |
| 26 | 27.662 ° | 151.236 | 6.8% |
| 27 | 30.134 ° | 131.3 | 7.4% |
| 28 | 31.558 ° | 351.989 | 44.4% |
| 29 | 33.055 ° | 127.673 | 4.6% |
| 30 | 34.958 ° | 130.661 | 6.1% |
| 31 | 36.330 ° | 133.373 | 6.7% |
| 32 | 39.819 ° | 179.373 | 6.2% |

### Example 10 Preparation of crystalline form E

Preparation method. 860 mg of crystalline form A1 was weighed and dissolved in 10 mL of ACN/water (v: v=80: 20) mixed solution at 50 °C. The resulting solution was filtered using a 0.45 µm nylon membrane syringe filter to obtain a clear solution. The clear solution was cooled to 5 °C at a rate of 0.1°C/min. When the temperature was cooled to about 30 °C, about 5 mg seed crystal of crystalline form E were added, and the resulting suspension was further cooled to 5 °C at a rate of 0.1°C/min and maintained at 5°C with stirring for 1 day. The solid was collected by filtration at 5°C, and the resulting solid was dried in vacuum at 50 °C for about 4.5 hours to obtained about 350 mg of crystalline form E, as an off-white powder, with a yield of about 41%.

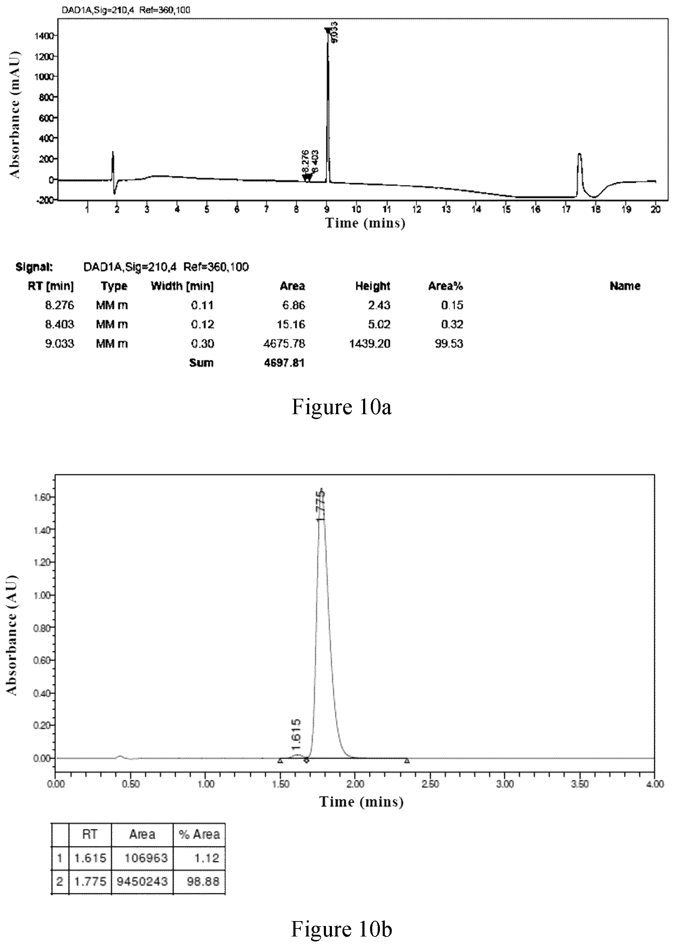

Its chemical purity is shown in Figure 10a, the chiral purity is shown in Figure 10b, the X-ray powder diffraction pattern is shown in Figure 10c, the parameters of each peak are shown in Table 10, its differential scanning calorimetry (DSC) pattern is shown in Figure 10d, its thermogravimetric analysis pattern is shown in Figure 10e, and the ¹H NMR spectrum is shown in Figure 10f.

**Table 10**

| Index | Angle | Intensity | Rel. Intensity |
|---|---|---|---|
| 1 | 3.278 ° | 143.79 | 6.5% |
| 2 | 6.550 ° | 266.267 | 37.4% |
| 3 | 8.806 ° | 216.663 | 26.7% |
| 4 | 9.252 ° | 199.82 | 23.1% |
| 5 | 9.806 ° | 330.932 | 46.0% |
| 6 | 10.828 ° | 107.527 | 6.6% |
| 7 | 13.067 ° | 112.021 | 5.9% |
| 8 | 14.362 ° | 158.266 | 13.9% |
| 9 | 16.339 ° | 161.615 | 15.3% |
| 10 | 17.384 ° | 654.666 | 100.0% |
| 11 | 17.996 ° | 581.611 | 85.3% |
| 12 | 18.519 ° | 271.771 | 29.0% |
| 13 | 19.151 ° | 288.651 | 31.0% |
| 14 | 19.573 ° | 569.078 | 80.3% |
| 15 | 20.016 ° | 149.399 | 5.5% |
| 16 | 20.553 ° | 257.543 | 24.7% |
| 17 | 21.131 ° | 242.818 | 22.4% |
| 18 | 21.818° | 294.712 | 32.4% |
| 19 | 22.590 ° | 146.784 | 6.3% |
| 20 | 22.904 ° | 161.374 | 8.5% |
| 21 | 23.456 ° | 162.708 | 8.0% |
| 22 | 24.838 ° | 319.096 | 33.9% |
| 23 | 25.057 ° | 349.065 | 39.1% |
| 24 | 25.475 ° | 278.68 | 26.6% |
| 25 | 26.536 ° | 229.539 | 18.6% |
| 26 | 27.662 ° | 151.236 | 6.8% |
| 27 | 30.134 ° | 131.3 | 7.4% |
| 28 | 31.558 ° | 351.989 | 44.4% |
| 29 | 33.055 ° | 127.673 | 4.6% |
| 30 | 34.958 ° | 130.661 | 6.1% |
| 31 | 36.330 ° | 133.373 | 6.7% |
| 32 | 39.819° | 179.373 | 6.2% |

### Example 11 Preparation of crystalline form F

Preparation method. About 40 mg of crystalline form A1 was weighed, and 0.2-1 mL of water was added, and the mixture was suspended with stirring at 25 °C and 300 rpm for 2 weeks. The resulting suspension was centrifuged and filtered at 14,000 rpm using a 0.45 µm nylon filter centrifuge tube to obtain crystalline form F.

Its X-ray powder diffraction pattern is shown in Figure 11a, the parameters of each peak are shown in Table 11, the differential scanning calorimetry (DSC) pattern is shown in Figure 11b, the thermogravimetric analysis pattern is shown in Figure 11c, and the ¹H NMR spectrum is shown in Figure 11d.

**Table 11**

| Index | Angle | Intensity | Rel. Intensity |
|---|---|---|---|
| 1 | 4.604 ° | 109.552 | 1.9% |
| 2 | 5.546 ° | 170.319 | 6.4% |
| 3 | 5.806 ° | 188.453 | 7.8% |
| 4 | 6.150 ° | 164.024 | 6.1% |
| 5 | 7.887 ° | 137.324 | 4.2% |
| 6 | 8.315 ° | 154.104 | 5.2% |
| 7 | 8.872 ° | 268.894 | 13.3% |
| 8 | 10.488 ° | 382.153 | 20.5% |
| 9 | 11.019 ° | 185.002 | 6.2% |
| 10 | 11.548 ° | 569.554 | 33.3% |
| 11 | 13.015 ° | 496.565 | 27.7% |
| 12 | 13.247 ° | 297.978 | 13.5% |
| 13 | 13.759 ° | 289.336 | 12.9% |
| 14 | 14.966 ° | 162.817 | 3.5% |
| 15 | 15.791 ° | 501.556 | 25.5% |
| 16 | 16.583 ° | 389.482 | 15.0% |
| 17 | 17.177 ° | 998.268 | 56.1% |
| 18 | 17.709 ° | 1640.35 | 100.0% |
| 19 | 18.283 ° | 727.739 | 33.7% |
| 20 | 18.894 ° | 497.223 | 16.0% |
| 21 | 19.201 ° | 1318.69 | 73.6% |
| 22 | 19.447 ° | 993.646 | 50.1% |
| 23 | 19.822 ° | 376.284 | 5.8% |
| 24 | 20.155 ° | 465.805 | 11.7% |
| 25 | 20.680 ° | 748.442 | 31.1% |
| 26 | 20.963 ° | 541.226 | 16.1% |
| 27 | 21.462° | 1375.01 | 74.8% |
| 28 | 21.749 ° | 965.132 | 45.6% |
| 29 | 22.019 ° | 1008.26 | 48.5% |
| 30 | 22.343 ° | 501.928 | 12.6% |
| 31 | 22.810 ° | 492.802 | 12.0% |
| 32 | 23.232 ° | 543.864 | 15.8% |
| 33 | 23.523 ° | 609.678 | 20.6% |
| 34 | 23.903 ° | 407.481 | 6.6% |
| 35 | 24.377 ° | 630518 | 22.5% |
| 36 | 24.631 ° | 458.758 | 10.3% |
| 37 | 25.393 ° | 495.092 | 13.2% |
| 38 | 25.912 ° | 582.672 | 19.8% |
| 39 | 26.777 ° | 405.138 | 8.3% |
| 40 | 27.210 ° | 403.998 | 9.0% |
| 41 | 27.660 ° | 1317.18 | 74.7% |
| 42 | 28.106° | 724.397 | 33.7% |
| 43 | 28.977 ° | 390.177 | 12.4% |
| 44 | 29.742 ° | 316.504 | 7.5% |
| 45 | 30.118 ° | 283.089 | 4.9% |
| 46 | 30.545 ° | 252.713 | 2.6% |
| 47 | 31.146 ° | 282.776 | 4.8% |
| 48 | 31.937 ° | 304.295 | 6.7% |
| 49 | 32.690 ° | 325.014 | 8.9% |
| 50 | 33.634 ° | 289.331 | 6.9% |

### Example 12 Preparation of crystalline form G

Preparation method. The compound of formula A was prepared by referring to the method of Example 15 of PCT/CN2022/097236, and then the compound was ring-closed under acidic conditions and crystallized in acetonitrile-water to obtain crystalline form G.

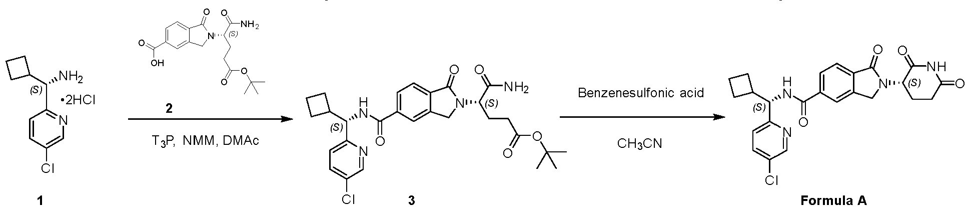

Compound 1 (32.5 g, 121 mmol) and compound **2** (56 g, 155 mmol) were added to DMAc (500 mL), and T₃P (46.7 g, 147 mmol) and NMM (62 g, 613 mmol) were added. The mixture was degassed and purged with N₂ three times. The mixture was stirred at 25 °C for 12 hours under N₂ environment. The mixture was poured into saturated sodium chloride aqueous solution (1000 mL), then filtered and washed with water. The filter cake was dissolved in DCM (2000 mL), washed with saturated aqueous NaHCO₃ solution (1000 mL). The organic phase was dried with anhydrous Na₂SO₄, filtered under reduced pressure and concentrated to obtain compound 3 (66 g) as a white solid (purity greater than 98%, chiral purity greater than 98%).

Compound **3** (66 g) was dissolved in CH₃CN (660 mL), and benzenesulfonic acid (57.4 g, 363 mmol) was added. The mixture was stirred at 50 °C for 16 hours under N₂ atmosphere, cooled to 0°C, and the pH was adjusted to 7-8 with 7% NaHCO₃. The above solution was slowly added dropwise to water (3300 mL), stirred for 3 hours, suction filtered, and the filter cake was washed with water (300 mL). The filter cake was dried in vacuum to obtain the compound of formula A (50 g) as a white solid, and the yield of the two steps was 89%.

The compound of formula A was identified as crystalline form G by XRPD (Figure 12c).

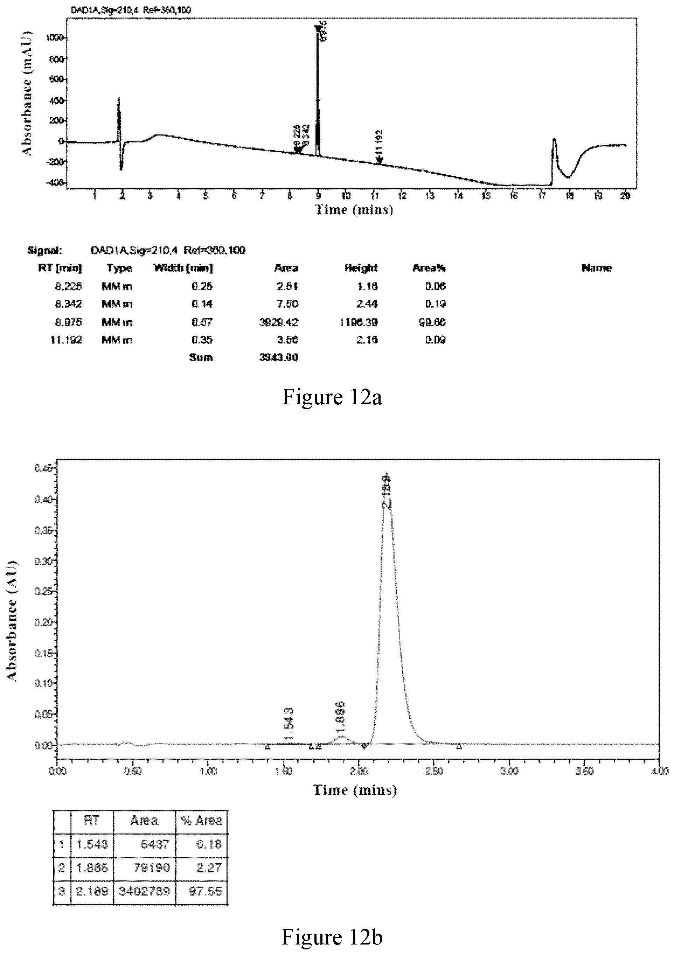

Its chemical purity spectrum is shown in Figure 12a, the chiral purity spectrum is shown in Figure 12b, the X-ray powder diffraction pattern is shown in Figure 12c, the parameters of each peak are shown in Table 12, the differential scanning calorimetry (DSC) pattern is shown in Figure 12d, the thermogravimetric analysis pattern is shown in Figure 12e, and the ¹H NMR spectrum is shown in Figure 12f.

**Table 12**

| Index | Angle | Intensity | Rel. Intensity |
|---|---|---|---|
| 1 | 7.355 ' | 94.2941 | 1.6% |
| 2 | 9.002 ° | 83.7517 | 1.1% |
| 3 | 11.598 ° | 86.9896 | 0.7% |
| 4 | 12.430 ' | 159.645 | 2.3% |
| 5 | 13.141 ° | 676.318 | 15.2% |
| 6 | 13.886 ° | 1545.5 | 37.2% |
| 7 | 14.673 ° | 356.66 | 7.2% |
| 8 | 15.927 ° | 859.852 | 20.1% |
| 9 | 16.531 ° | 211.2 | 3.8% |
| 10 | 17.137 ° | 208.944 | 3.6% |
| 11 | 17.953 ° | 869.03 | 19.9% |
| 12 | 18.837 ° | 4034.74 | 100.0% |
| 13 | 19.586 ° | 276.525 | 5.0% |
| 14 | 20.529 ° | 97.8431 | 0.6% |
| 15 | 21.031° | 331.751 | 6.3% |
| 16 | 21.455 ° | 1391.08 | 33.0% |
| 17 | 22.034 ° | 241.296 | 3.9% |
| 18 | 22.312 ° | 184.232 | 2.5% |
| 19 | 22.896 ° | 270.145 | 4.7% |
| 20 | 23.233 ° | 163.817 | 2.0% |
| 21 | 23.647 ° | 157.872 | 1.7% |
| 22 | 24.503 ° | 393.162 | 7.4% |
| 23 | 24.761° | 519.971 | 10.5% |
| 24 | 25.068 ° | 677.033 | 14.4% |
| 25 | 25.362 ° | 434.118 | 8.2% |
| 26 | 25.997 ° | 361.374 | 6.2% |
| 27 | 26.455 ° | 411.917 | 7.4% |
| 28 | 26.755 ° | 918.346 | 20.2% |
| 29 | 27.109 ° | 293.309 | 4.4% |
| 30 | 27.873 ° | 558.659 | 11.3% |
| 31 | 28.552 ° | 343.613 | 6.1% |
| 32 | 29.454 ° | 413.626 | 7.7% |
| 33 | 29.783 ° | 571.263 | 11.6% |
| 34 | 30.186 ° | 267.033 | 3.9% |
| 35 | 30.407 ° | 203.296 | 2.3% |
| 36 | 30.832 ° | 419.995 | 7.9% |
| 37 | 31.832 " | 428.625 | 8.2% |
| 38 | 32.516 ° | 416.376 | 8.0% |
| 39 | 32.783 ° | 252.051 | 3.9% |
| 40 | 33.646 ° | 186.674 | 2.3% |
| 41 | 33.822 ° | 179.265 | 2.1% |
| 42 | 34.403 ° | 300.504 | 5.1% |
| 43 | 34.900 ° | 190.403 | 2.4% |
| 44 | 35.937 ° | 236.792 | 3.7% |
| 45 | 36.233 ° | 176.214 | 2.1% |
| 46 | 36.440 ° | 175.915 | 2.0% |
| 47 | 38.278 ° | 319.277 | 5.3% |
| 48 | 38.892 ° | 326.188 | 5.6% |
| 49 | 39.595 ° | 134.695 | 0.8% |

### Example 13 Preparation of crystalline form H

Preparation method. About 60 mg of crystalline form E was weighed, and 0.2-1 mL of methanol was added, and the mixture was stirred at 50 °C and 300 rpm for 1 week. The resulting suspension was centrifuged and filtered at 14,000 rpm using a 0.45 µm nylon filter centrifuge tube to obtain crystalline form H.

Its X-ray powder diffraction pattern is shown in Figure 13a, the parameters of each peak are shown in Table 13, the differential scanning calorimetry (DSC) pattern is shown in Figure 13b, the thermogravimetric analysis pattern is shown in Figure 13c, and the ¹H NMR spectrum is shown in Figure 13d.

**Table 13**

| Index | Angle | Intensity | Rel. Intensity |
|---|---|---|---|
| 1 | 3.800° | 552.29 | 65.3% |
| 2 | 9.840 ° | 156.247 | 20.9% |
| 3 | 10.712 ° | 172.897 | 23.9% |
| 4 | 11.337 ° | 208.76 | 31.5% |
| 5 | 11.819 ° | 221.933 | 34.7% |
| 6 | 15.120 ° | 114.06 | 14.3% |
| 7 | 16.047 ° | 200.812 | 31.2% |
| 8 | 17.658 ° | 110.213 | 11.8% |
| 9 | 18.303 ° | 554.89 | 100.0% |
| 10 | 18.533 ° | 327.285 | 53.8% |
| 11 | 19.059 ° | 383.05 | 64.5% |
| 12 | 19.713 ° | 139.692 | 15.4% |
| 13 | 21.020° | 133.398 | 15.3% |
| 14 | 21.434 ° | 106.707 | 10.7% |
| 15 | 22.653 ° | 68.3525 | 4.1% |
| 16 | 23.806 ° | 70.6591 | 4.3% |
| 17 | 24.954 ° | 94.1567 | 7.2% |
| 18 | 25.326 ° | 123.831 | 12.9% |
| 19 | 26.401 ° | 110.757 | 10.6% |
| 20 | 27.395 ° | 152.237 | 19.2% |
| 21 | 29.056 ° | 122.835 | 14.3% |
| 22 | 30.563 ° | 80.4368 | 6.7% |
| 23 | 32.398 ° | 80.2633 | 4.9% |
| 24 | 33.722° | 93.7847 | 7.1% |

### Example 14 Preparation of amorphous form of compound A

Preparation method 1. About 40 mg of crystalline form A1 was weighed, and 0.2-1 mL of EA was added, and the mixture was suspended with stirring at 25 °C and 300 rpm for 2 weeks. The resulting suspension was centrifuged and filtered at 14,000 rpm using a 0.45 µm nylon filter centrifuge tube to obtain an amorphous form of the compound of formula A.

Preparation method 2. About 40 mg of crystalline form A1 was weighed, and 0.2-1 mL of acetone was added, and the mixture was suspended with stirring at 25 °C and 300 rpm for 2 weeks. The resulting suspension was centrifuged and filtered at 14,000 rpm using a 0.45 µm nylon filter centrifuge tube to obtain an amorphous form of the compound of formula A.

Preparation method 3. About 40 mg of crystalline form A1 was weighed, and 0.2-1 mL of THF was added, and the mixture was suspended with stirring at 25 °C and 300 rpm for 2 weeks. The resulting suspension was centrifuged and filtered at 14,000 rpm using a 0.45 µm nylon filter centrifuge tube to obtain an amorphous form of the compound of formula A.

Preparation method 4. About 50 mg of crystalline form A1 was weighed, and 0.2-1 mL of EA was added. 10 temperature rise and fall cycles were performed between 5 °C and 50 °C at a rate of 0.2 °C/min, and the mixture was suspended with 300 rpm magnetic stirring at the same time. The resulting suspension was centrifuged at 14,000 rpm at 5 °C using a 0.45 µm nylon filter centrifuge tube to obtain an amorphous form of the compound of formula A.

Preparation method 5. A crude product was obtained according to the method of Example 1, and then the crude product was treated with EA (5 mL) and MTBE (5 mL) to obtain a filter cake. The filter cake was added to 30 mL of acetonitrile and ultrasonicated for 5 minutes to uniformly disperse it into a slurry solution, and then 70 mL of water was added to make the whole solution clear and transparent, and after freeze-drying, an amorphous off-white solid of compound of formula A was obtained.

Preparation method 6. A crude product was obtained according to the method of Example 1, and then the crude product is spray-dried with a solvent such as DCM and EtOH, with the air inlet temperature set at 80 ° C to 120°C to obtain an amorphous form of the compound of formula A.

The X-ray powder diffraction pattern of the amorphous form of the compound of formula A is shown in Figure 14a.

### Example 15 Stability test of crystalline form B

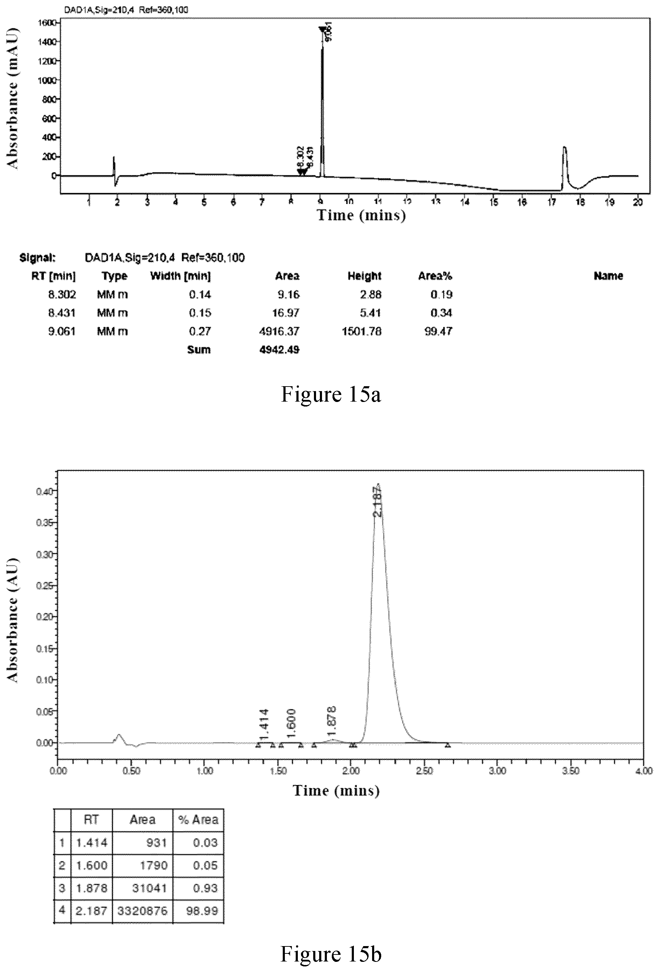

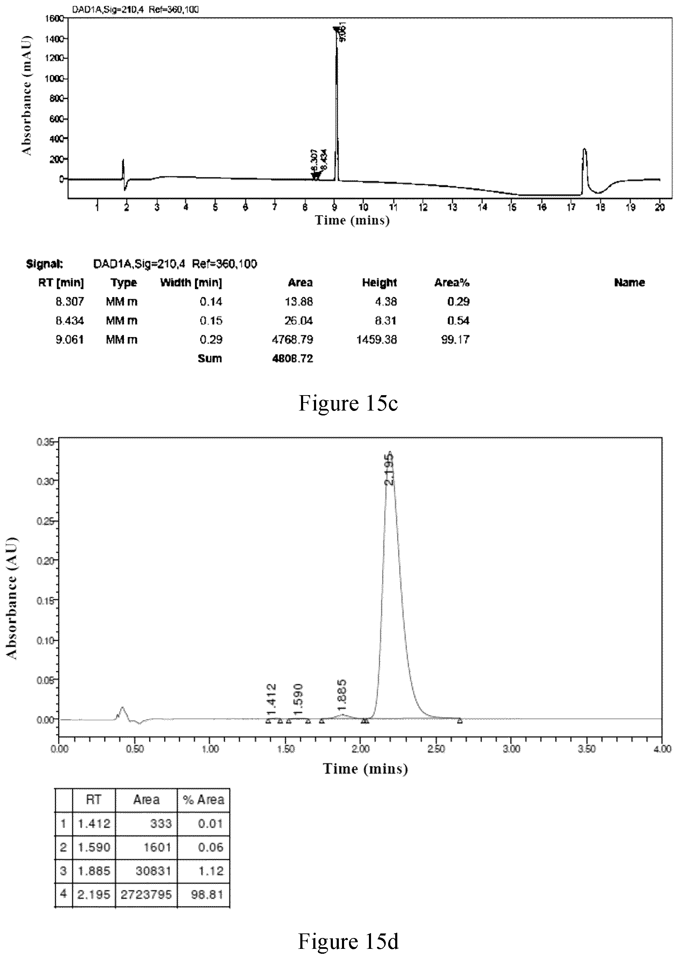

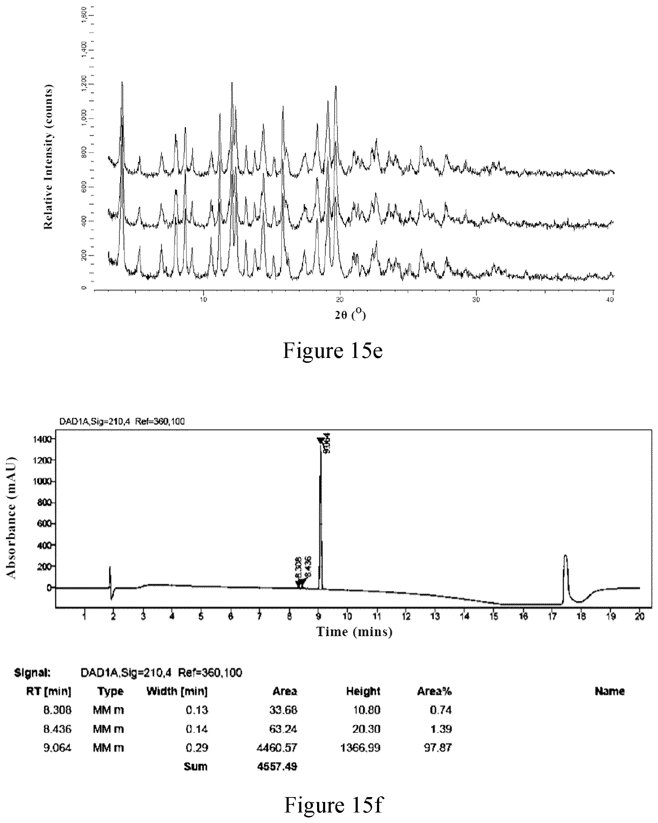

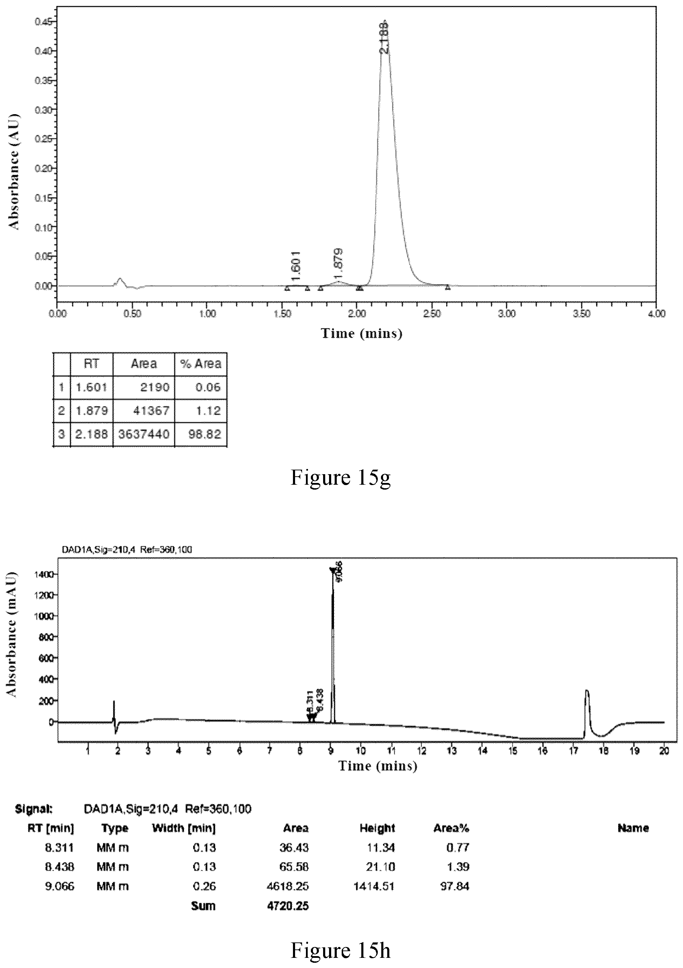

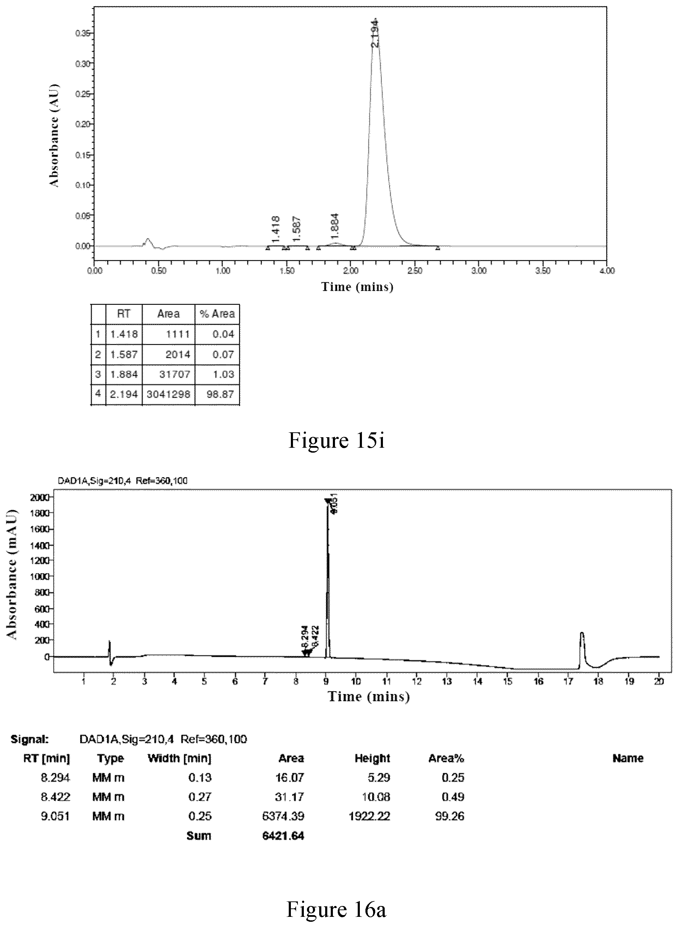

The chemical purity of crystalline form B is shown in Figure 15a, and its chiral purity is shown in Figure 15b. Placed in a open container at 25°C/92% RH and 40°C/75% RH for 13 days, respectively, or in a sealed container at 60°C for 13 days. The stability sample was tested by XRPD and HPLC and the sample was observed for color change. The data is shown in Table 14.

**Table 14**

| NO. | | Crystalline form B | | |
|---|---|---|---|---|
| | Initial purity (HPLC/SFC) | Chemical purity | Chiral purity | |
| | | 99.5% See Figure 15a | 99.0% See Figure 15b | |
| | | Chemical purity | Chiral purity | Color |
| 1 | Solid, 25 °C/92% RH, open container, 13 days | | | |
| | Solid (HPLC/SFC) | 99.2% See Figure 15c | 98.8% See Figure 15d | No color change |
| | Solid (XRPD) | The crystal form did not change, see Figure 15e | | |
| 2 | Solid, 40 °C/75% RH, open container, 13 days | | | |
| | Solid (HPLC/SFC) | 97.9% See Figure 15f | 98.8% See Figure 15g | No color change |
| | Solid (XRPD) | The crystal form did not change, see Figure 15e | | |
| 3 | Solid, 60 °C, sealed container, 13 days | | | |
| | Solid (HPLC/SFC) | 97.8% See Figure 15h | 98.9% See Figure 15i | No color change |
| | Solid (XRPD) | The crystal form did not change, see Figure 15e | | |

### Example 16 Stability test of crystalline form E

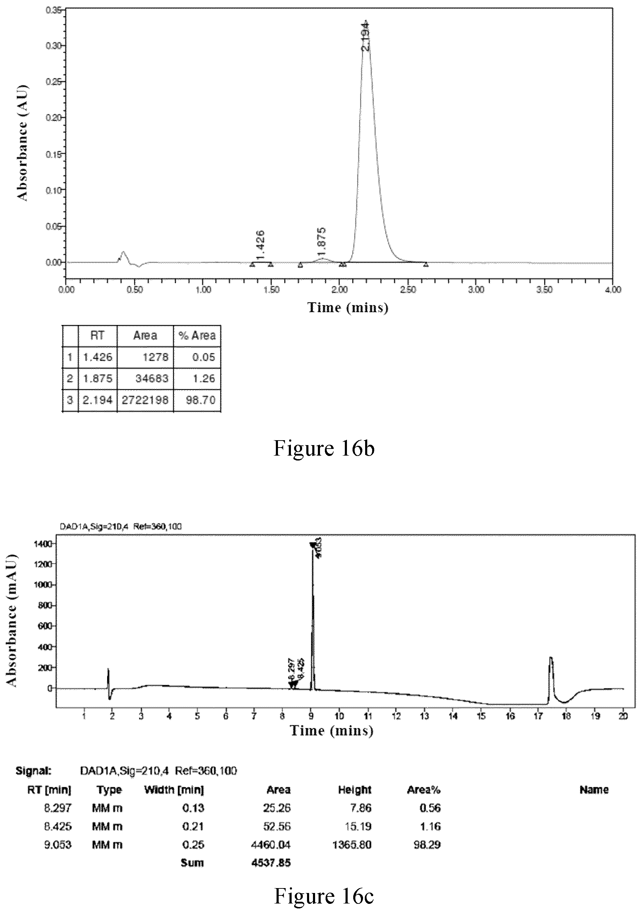

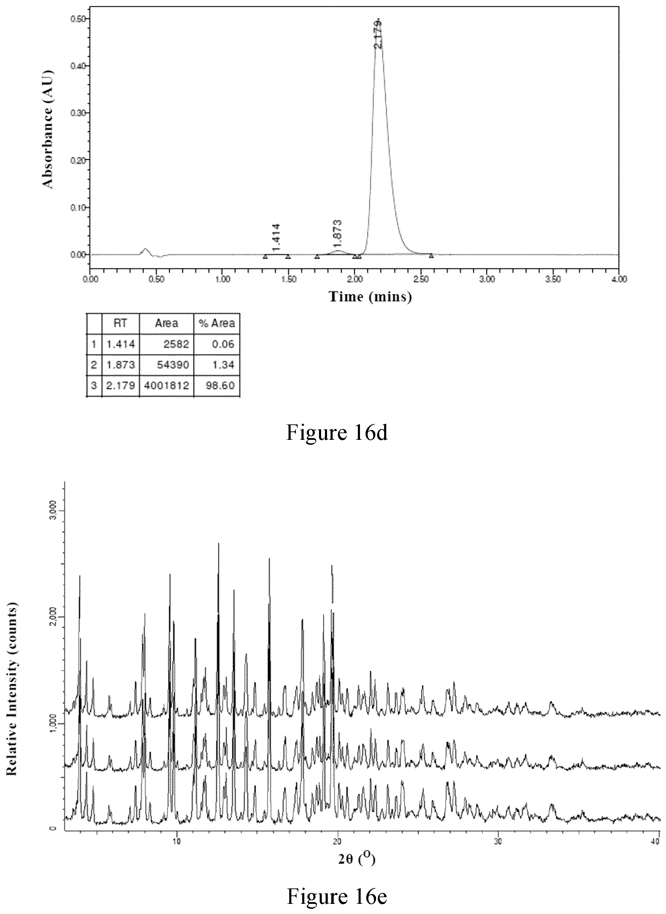

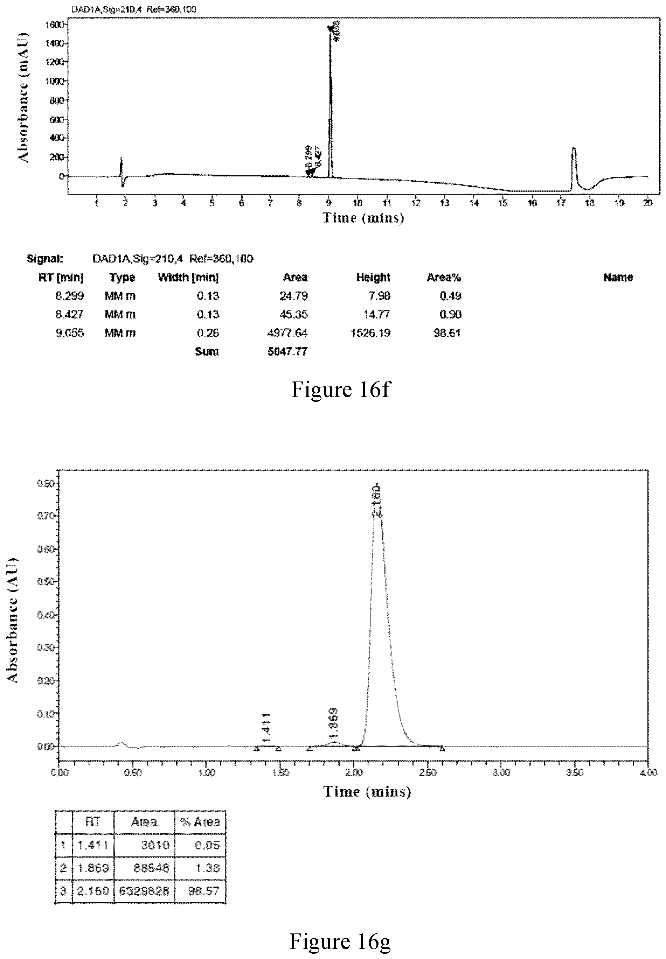

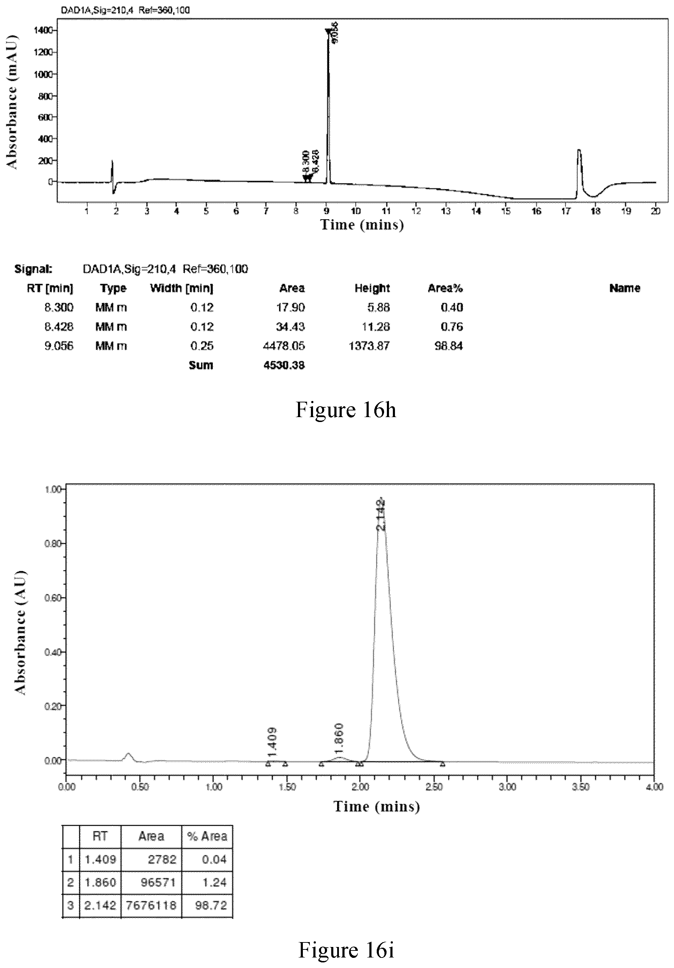

The chemical purity of crystalline form E is shown in Figure 16a, and its chiral purity is shown in Figure 16b. Placed in a open container at 25 °C/92% RH and 40 °C/75% RH for 13 days, respectively, or in a sealed container at 60 °C for 13 days. The stability sample was tested by XRPD and HPLC and the sample was observed for color change. The data is shown in Table 15.

**Table 15**

| NO. | | Crystalline form E | | |
|---|---|---|---|---|
| | Initial purity (HPLC/SFC) | Chemical purity | Chiral purity | |
| | | 99.3% See Figure 16a | 98.7% See Figure 16b | |
| | | Chemical purity | Chiral purity | Color |
| 1 | Solid, 25 °C/92% RH, open container, 1 week | | | |
| | Solid (HPLC/SFC) | 98.3% See Figure 16c | 98.6% See Figure 16d | No color change |
| | Solid (XRPD) | The crystal form did not change, see Figure 16e | | |
| 2 | Solid, 40 °C/75% RH, open container, 1 week | | | |
| | Solid (HPLC/SFC) | 98.6% See Figure 16f | 98.6% See Figure 16g | No color change |
| | Solid (XRPD) | The crystal form did not change, see Figure 16e | | |
| 3 | Solid, 60 °C, sealed container, 1 week | | | |
| | Solid (HPLC/SFC) | 98.8% See Figure 16h | 98.7% See Figure 16i | No color change |
| | Solid (XRPD) | The crystal form did not change, see Figure 16e | | |

### Example 17 Stability test of crystalline form G

The chemical purity of crystalline form G is shown in Figure 12a, and its chiral purity is shown in Figure 12b. Placed in a open container at 25 °C/92% RH and 40 °C/75% RH for 13 days, respectively, or in a sealed container at 60 °C for 13 days. The stability sample was tested by XRPD and HPLC and the sample was observed for color change. The data is shown in Table 16.

**Table 16**

| NO. | | Crystalline form G | | |
|---|---|---|---|---|
| | Initial purity (HPLC/SFC) | Chemical purity | Chiral purity | |
| | | 99.7% | 97.6% | |
| | | See Figure 12a | See Figure 12b | |
| | | Chemical purity | Chiral purity | Color |
| 1 | Solid, 25 °C/92% RH, open container, 1 week | | | |
| | Solid (HPLC/SFC) | 99.4% | 97.8% | No color change |
| | | See Figure 17a | See Figure 17b | |
| | Solid (XRPD) | The crystal form did not change, see Figure 17c | | |
| 2 | Solid, 40 °C75% RH, open container, 1 week | | | |
| | Solid (HPLC/SFC) | 99.5% | 97.8% | No color change |
| | | See Figure 17d | See Figure 17e | |
| | Solid (XRPD) | The crystal form did not change, see Figure 17c | | |
| 3 | Solid, 60 °C, sealed container, 1 week | | | |
| | Solid (HPLC/SFC) | 99.5% | 97.7% | No color change |
| | | See Figure 17f | See Figure 17g | |
| | Solid (XRPD) | The crystal form did not change, see Figure 17c | | |

### Example 18 Hygroscopicity test of crystalline form B

The hygroscopicity and dehydration behavior of crystalline form B was investigated by DVS. Humidity program: 25 °C, 40-0-95-0-40% RH, dm/dt 0.002, shortest equilibrium time was 60 min, and longest equilibrium time was 360 min. XRPD was also performed on the samples after DVS test to determine whether the crystal transformation occurred. The results are shown in Table 17.

**Table 17**

| Crystal form | Crystalline form B | | | |
|---|---|---|---|---|
| Method | 40-0-95-0-40% RH, dm/dt 0.002, min. adsorption equilibrium time 60 min, maximum adsorption equilibrium time 360 min, 25 °C | | | |
| 25 °C Relative humidity | 1^{st} desorption weight change percentage | 1^{st} adsorption weight change percentage | 2^{nd} desorption weight change percentage | 2^{nd} adsorption weight change percentage |
| 0% | 1.6 | 1.6 | 0.0 | 0.0 |
| 10% | 2.0 | 2.0 | 0.4 | 0.4 |
| 20% | 2.7 | 2.7 | 1.1 | 1.1 |
| 30% | 3.3 | 3.2 | 1.7 | 1.7 |
| 40% | 3.8 | 3.7 | 2.3 | 2.2 |
| 50% | N/A | 4.5 | 3.3 | N/A |
| 60% | N/A | 5.0 | 3.9 | N/A |
| 70% | N/A | 5.4 | 5.4 | N/A |
| 80% | N/A | 5.9 | 5.9 | N/A |
| 90% | N/A | 6.9 | 7.3 | N/A |
| 95% | N/A | 9.2 | 9.2 | N/A |
| DVS | | | | |
| 2.2% of moisture was adsorbed under 40%R H-80%RH, and 5.5% of moisture was adsorbed under 40%RH-95%RH, see Figure 18a | | | | |
| XRPD after DVS test | | | | |
| The crystal form did not change, see Figure 18b | | | | |

| | | | | |
|---|---|---|---|---|
| Note "N/A": Not tested. | | | | |

### Example 19 Hygroscopicity test of crystalline form E

The hygroscopicity and dehydration behavior of crystalline form E was investigated by DVS. Humidity program: 25 °C, 40-0-95-0-40% RH, dm/dt 0.002, shortest equilibrium time was 60 min, and longest equilibrium time was 360 min. XRPD was also performed on the samples after DVS test to determine whether the crystal transformation occurred. The results are shown in Table 18.

**Table 18**

| Crystal form | Crystalline form E | | | |
|---|---|---|---|---|
| Method | 40-0-95-0-40%RH, dm/dt 0.002, min. adsorption equilibrium time 60 min, maximum adsorption equilibrium time 360 min, 25 °C | | | |
| 25 °C Relative humidity | 1^{st} desorption weight change percentage | 1^{st} adsorption weight change percentage | 2^{nd} desorption weight change percentage | 2^{nd} adsorption weight change percentage |
| 0% | 0.1 | 0.1 | 0.0 | 0.0 |
| 10% | 0.3 | 0.3 | 0.3 | 0.3 |
| 20% | 1.3 | 1.3 | 1.3 | 1.3 |
| 30% | 1.6 | 1.6 | 1.6 | 1.6 |
| 40% | 2.0 | 2.0 | 2.0 | 2.0 |
| 50% | N/A | 2.7 | 2.8 | N/A |
| 60% | N/A | 3.1 | 3.3 | N/A |
| 70% | N/A | 3.5 | 3.5 | N/A |
| 80% | N/A | 3.6 | 3.6 | N/A |
| 90% | N/A | 3.8 | 3.8 | N/A |
| 95% | N/A | 4.0 | 4.0 | N/A |
| DVS | | | | |
| 2.0% of moisture was adsorbed under 40%RH-95%RH, see Figure 19a | | | | |
| XRPD after DVS test | | | | |
| The crystal form did not change, see Figure 19b | | | | |

| | | | | |
|---|---|---|---|---|
| Note "N/A": Not tested. | | | | |

### Example 20 Hygroscopicity test of crystalline form G

The hygroscopicity and dehydration behavior of crystalline form G was investigated by DVS. Humidity program: 25 °C, 40-0-95-0-40% RH, dm/dt 0.002, shortest equilibrium time was 60 min, and longest equilibrium time was 360 min. XRPD was also performed on the samples after DVS test to determine whether the crystal transformation occurred. The results are shown in Table 19.

**Table 19**

| Crystal form | Crystalline form G | | | |
|---|---|---|---|---|
| Method | 40-0-95-0-40%RH, dm/dt 0.002, min. adsorption equilibrium time 60 min, maximum adsorption equilibrium time 360 min, 25 °C | | | |
| 25°C Relative humidity | 1^{st} desorption weight change percentage | 1^{st} adsorption weight change percentage | 2^{nd} desorption weight change percentage | 2^{nd} adsorption weight change percentage |
| 0% | 0.0 | 0.0 | 0.0 | 0.0 |
| 10% | 0.1 | 0.0 | 0.1 | 0.0 |
| 20% | 0.1 | 0.0 | 0.1 | 0.0 |
| 30% | 0.2 | 0.1 | 0.2 | 0.1 |
| 40% | 0.3 | 0.1 | 0.3 | 0.1 |
| 50% | N/A | 0.2 | 0.3 | N/A |
| 60% | N/A | 0.2 | 0.4 | N/A |
| 70% | N/A | 0.3 | 0.4 | N/A |
| 80% | N/A | 0.3 | 0.5 | N/A |
| 90% | N/A | 0.5 | 0.5 | N/A |
| 95% | N/A | 0.7 | 0.7 | N/A |
| DVS | | | | |
| 0.6% of moisture was adsorbed under 40%RH-95%RH, see Figure 20a | | | | |
| XRPD after DVS test | | | | |
| The crystal form did not change, see Figure 20b | | | | |

| | | | | |
|---|---|---|---|---|
| Note "N/A": Not tested. | | | | |

### Example 21 Simulated tableting experiment

About 10 mg of crystalline form G was weighed and pressed at 2 MPa, 5 MPa or 10 MPa for 5 min. The crystal transformation and the change of crystallinity were investigated by XRPD characterization. See Table 20.

**Table 20**

| **NO.** | **Pressure** | **XRPD** | **Notes** |
|---|---|---|---|
| 1 | 2 MPa | Crystalline form G, see Figure 21a | No significant change in crystallinity |
| 2 | 5 MPa | Crystalline form G, see Figure 21a | No significant change in crystallinity |
| 3 | 10 MPa | Crystalline form G, see Figure 21a | No significant change in crystallinity |

### Example 22 Pharmaceutical Composition

**Table 21**

| | |
|---|---|
| Crystalline form B or Crystalline form E or Crystalline form G | 200 g |
| starch | 180 g |
| Microcrystalline cellulose | 40 g |

According to the conventional method, as shown in Table 21, the above substances were mixed evenly and filled into ordinary gelatin capsules to obtain 10,000-100,000 capsules.

### Example 23 Pharmacokinetic evaluation in mice by gavage of the crystalline form G and amorphous form of the compound of formula A

CD1 male mice were selected and the test compound was administered orally. The drug concentration in plasma at different time points was quantitatively determined by LC/MS/MS to evaluate the pharmacokinetic characteristics of the test drug in mice.

Experimental materials: CD1 Mouse (male, 20-30 g, 6-8 weeks old, Zhejiang Weitong Lihua).

Experimental procedure: as shown in Table 22, the test compounds were prepared in 25 mM citrate buffer (pH 3) containing 5% Tween 80 and administered orally to CD1 mice (free access to food and water). Blood was collected from the dorsal plantar vein at 0.25 h, 0.5 h, 1 h, 2 h, 4 h, 6 h, 8 h and 24 h after administration, placed in an anticoagulant tube containing EDTA-K2 and mixed, and centrifuged at 4°C and 4000 g for 5 minutes. The drug concentration in blood was determined by LC-MS/MS, and the relevant pharmacokinetic parameters were calculated by the non-compartmental linear/log trapezoidal method using Phoenix WinNonlin 6.3 pharmacokinetic software.

**Table 22**

| PK parameters | Crystalline form G | Amorphous |
|---|---|---|
| Dosage (mg/kg) | 30 | 30 |
| Dosing volume (mL/kg) | 10 | 10 |
| Tₘₐₓ (h)/T_{1/2} (h) | 0.42 / 8.7 | 0.42 / 3.1 |
| Cₘₐₓ (ng/mL) | 1367 ± 365 | 17800 ± 3132 |
| AUCₗₐₛₜ (h*ng/mL) | 9172 ± 1048 | 35328 ± 3479 |
| AUC_{Inf} (h*ng/mL) | 10652 ± 2105 | 35872 ± 3759 |
| AUCₗₐₛₜ /D (h*mg/mL) | 306 ± 35 | 1178 ± 116 |

As shown in Table 22, the *in vivo* pharmacokinetic parameters of the compound of formula A are outstanding, and the area under the drug-time curve is 3 to 4 times that of the crystalline form G. As shown in Table 14, Table 15 and Table 16, the stability of crystalline form B, crystalline form E and crystalline form G is outstanding, and the crystal form did not change. As shown in the hygroscopic tests of Table 17, Table 18 and Table 19, the stability of crystalline form B, crystalline form E and crystalline form G is outstanding, and the crystal form did not change. In particular, the crystalline form G was tableted at 2 MPa, 5 MPa and 10 MPa pressures, respectively, as shown in Table 20, and no significant change in crystallinity was observed. Therefore, the forms described in the present invention are very suitable for use in pharmaceutical compositions. Moreover, the crystalline form and amorphous form of the present invention are not easy to be stirred up, easy to collect, and not easy to cause waste during the drug manufacturing process such as packaging, which helps to protect the health of operators.

All literatures mentioned in the present invention are incorporated by reference herein, as though individually incorporated by reference. Additionally, it should be understood that after reading the above teaching, many variations and modifications may be made by the skilled in the art, and these equivalent forms also fall within the scope defined by the appended claims.

## Claims

1. A amorphous form or crystalline form of the compound of formula A or a pharmaceutically acceptable salt or a solvate thereof

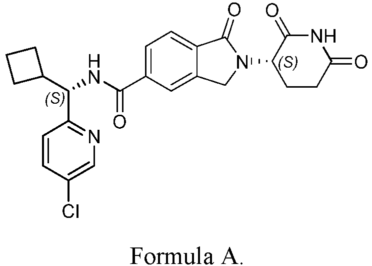

2. The form according to claim 1, wherein the form is a crystalline form G of the compound of formula A, which has at least three, at least four, at least five, at least six or seven characteristic peaks at the following positions in an X-ray powder diffraction (XRPD) pattern in 2θ angle: 18.83 ± 0.2°, 13.88 ± 0.2°, 21.45 ± 0.2°, 26.75 ± 0.2°, 15.92 ± 0.2°, 17.95 ± 0.2° and 13.14 ± 0.2°; preferably, the form has XRPD characteristic peaks at positions substantially as shown in Table 12; more preferably, the form has an XRPD pattern substantially as shown in Figure 12c; and optionally has one or more of the following features:
1) having an endothermic peak at 236.59°C ± 2°C in DSC pattern;
2) having a weight loss of 0.52 ± 0.2 wt% before reaching 150°C in TGA pattern;
3) having a DSC pattern substantially as shown in Figure 12d; and/or
4) having a TGA pattern substantially as shown in Figure 12e.

3. The form according to claim 1, wherein the form is a solvate crystalline form A1 of the compound of formula A, which has at least three, at least four, at least five, at least six or seven characteristic peaks at the following positions in an X-ray powder diffraction (XRPD) pattern in 2θ angle: 6.54 ± 0.2°, 19.64 ± 0.2°, 9.21 ± 0.2°, 16.35 ± 0.2°, 18.48 ± 0.2°, 9.79 ± 0.2° and 17.23 ± 0.2°; preferably, the form has XRPD characteristic peaks at positions substantially as shown in Table 1; more preferably, the form has an XRPD pattern substantially as shown in Figure 1c; and optionally has one or more of the following features:
1) having an endothermic peak at 147.77°C ± 2°C and an endothermic peak at 159.25°C ± 2°C in DSC pattern;
2) having a weight loss of 10.64 ± 0.2 wt% before reaching 240°C in TGA pattern;
3) having a DSC pattern substantially as shown in Figure Id; and/or
4) having a TGA pattern substantially as shown in Figure 1e.

4. The form according to claim 1, wherein the form is a solvate crystalline form A2 of the compound of formula A, which has at least three, at least four, at least five, at least six or seven characteristic peaks at the following positions in an X-ray powder diffraction (XRPD) pattern in 2θ angle: 18.57 ± 0.2°, 19.67 ± 0.2°, 16.38 ± 0.2°, 9.28 ± 0.2°, 17.38 ± 0.2°, 25.18 ± 0.2° and 13.11 ± 0.2°; preferably, the form has XRPD characteristic peaks at positions substantially as shown in Table 2; more preferably, the form has an XRPD pattern substantially as shown in Figure 2a; and optionally has one or more of the following features:
1) having an endothermic peak at 153.88°C ± 2°C and an endothermic peak at 178.46°C ± 2°C in DSC pattern;
2) having a weight loss of 11.32 ± 0.2 wt% before reaching 165.00°C, and a weight loss of 2.83 ± 0.2 wt% between 165.00°C and 230.00°C in TGA pattern;
3) having a DSC pattern substantially as shown in Figure 2b; and/or
4) having a TGA pattern substantially as shown in Figure 2c.

5. The form according to claim 1, wherein the form is a solvate crystalline form A3 of the compound of formula A, which has at least three, at least four, at least five, at least six or seven characteristic peaks at the following positions in an X-ray powder diffraction (XRPD) pattern in 2θ angle: 19.61 ± 0.2°, 18.45 ± 0.2° 9.22 ± 0.2°, 16.33 ± 0.2°, 6.54 ± 0.2°, 17.24 ± 0.2° and 9.80 ± 0.2°; preferably, the form has XRPD characteristic peaks at positions substantially as shown in Table 3; more preferably, the form has an XRPD pattern substantially as shown in Figure 3a; and optionally has one or more of the following features:
1) having an exothermic peak at 90.56°C ± 2°C, and an endothermic peak at 152.55°C ± 2°C and an endothermic peak at 177.33°C ± 2°C respectively in DSC pattern;
2) having a weight loss of 12.08 ± 0.2 wt% before reaching 165.00°C, and a weight loss of 2.12 ± 0.2 wt% between 165.00°C and 230.00°C in TGA pattern;
3) having a DSC pattern substantially as shown in Figure 3b; and/or
4) having a TGA pattern substantially as shown in Figure 3c.

6. The form according to claim 1, wherein the form is a solvate crystalline form A4 of the compound of formula A, which has at least three, at least four, at least five, at least six or seven characteristic peaks at the following positions in an X-ray powder diffraction (XRPD) pattern in 2θ angle: 9.22 ± 0.2°, 17.51 ± 0.2°, 6.54 ± 0.2°, 19.59 ± 0.2°, 25.03 ± 0.2°, 16.37 ± 0.2° and 18.51 ± 0.2°; preferably, the form has XRPD characteristic peaks at positions substantially as shown in Table 4; more preferably, the form has an XRPD pattern substantially as shown in Figure 4a; and optionally has one or more of the following features:
1) having an endothermic peak at 149.48°C ± 2°C in DSC pattern;
2) having a weight loss of 3.01 ± 0.2 wt% before reaching 160°C in TGA pattern;
3) having a DSC pattern substantially as shown in Figure 4b; and/or
4) having a TGA pattern substantially as shown in Figure 4c.

7. The form according to claim 1, wherein the form is a solvate crystalline form A5 of the compound of formula A, which has at least three, at least four, at least five, at least six or seven characteristic peaks at the following positions in an X-ray powder diffraction (XRPD) pattern in 2θ angle: 16.43 ± 0.2°, 19.74 ± 0.2°, 6.58 ± 0.2°, 9.27 ± 0.2°, 18.53 ± 0.2°, 17.37 ± 0.2° and 9.88 ± 0.2°; preferably, the form has XRPD characteristic peaks at positions substantially as shown in Table 5; more preferably, the form has an XRPD pattern substantially as shown in Figure 5a; and optionally has one or more of the following features:
1) having an endothermic peak at 154.86°C ± 2°C in DSC pattern;
2) having a weight loss of 6.75 ± 0.2 wt% before reaching 220°C in TGA pattern;
3) having a DSC pattern substantially as shown in Figure 5b; and/or
4) having a TGA pattern substantially as shown in Figure 5c.

8. The form according to claim 1, wherein the form is a crystalline form B of the compound of formula A, which has at least three, at least four, at least five, at least six or seven characteristic peaks at the following positions in an X-ray powder diffraction (XRPD) pattern in 2θ angle: 3.98 ± 0.2°, 12.35 ± 0.2°, 12.05 ± 0.2°, 19.09 ± 0.2°, 7.92 ± 0.2°, 15.78 ± 0.2° and 14.32 ± 0.2°; preferably, the form has XRPD characteristic peaks at positions substantially as shown in Table 6: more preferably, the form has an XRPD pattern substantially as shown in Figure 6c; and optionally has one or more of the following features:
1) having an endothermic peak at 25.54°C ± 2°C and an endothermic peak at 183.52°C ± 2°C in DSC pattern;
2) having a weight loss of 1.03 ± 0.2 wt% before reaching 100°C in TGA pattern;
3) having a DSC pattern substantially as shown in Figure 6d; and/or
4) having a TGA pattern substantially as shown in Figure 6e.

9. The form according to claim 1, wherein the form is a solvate crystalline form C of the compound of formula A, which has at least three, at least four, at least five, at least six or seven characteristic peaks at the following positions in an X-ray powder diffraction (XRPD) pattern in 2θ angle: 20.60 ± 0.2°, 17.94 ± 0.2°, 13.11 ± 0.2°, 19.42 ± 0.2°, 23.97 ± 0.2°, 26.31 ± 0.2° and 11.95 ± 0.2°; preferably, the form has XRPD characteristic peaks at positions substantially as shown in Table 7; more preferably, the form has an XRPD pattern substantially as shown in Figure 7a; and optionally has the following features:
1) having an endothermic peak at 127.33°C ± 2°C in DSC pattern;
2) having a weight loss of 16.21 ± 0.2 wt% before reaching 160.00°C, and a weight loss of 9.29 ± 0.2 wt% between 160.00°C and 260.00°C in TGA pattern;
3) having a DSC pattern substantially as shown in Figure 7b; and/or
4) having a TGA pattern substantially as shown in Figure 7c.

10. The form according to claim 1, wherein the form is a solvate crystalline form D1 of the compound of formula A, which has at least three, at least four, at least five, at least six or seven characteristic peaks at the following positions in an X-ray powder diffraction (XRPD) pattern in 2θ angle: 17.99 ± 0.2°, 8.82 ± 0.2°, 17.32 ± 0.2°, 9.26 ± 0.2°, 19.14 ± 0.2°, 9.95 ± 0.2° and 31.53 ± 0.2°; preferably, the form has XRPD characteristic peaks at positions substantially as shown in Table 8; more preferably, the form has an XRPD pattern substantially as shown in Figure 8a; and optionally has one or more of the following features:
1) having an endothermic peak at 161.17°C ± 2°C in DSC pattern;
2) having a weight loss of 11.30 ± 0.2 wt% before reaching 210°C in TGA pattern;
3) having a DSC pattern substantially as shown in Figure 8b; and/or
4) having a TGA pattern substantially as shown in Figure 8c.

11. The form according to claim 1, wherein the form is a solvate crystalline form D2 of the compound of formula A, which has at least three, at least four, at least five, at least six or seven characteristic peaks at the following positions in an X-ray powder diffraction (XRPD) pattern in 2θ angle: 17.38 ± 0.2°, 17.99 ± 0.2°, 19.57 ± 0.2°, 9.80 ± 0.2°, 31.55 ± 0.2°, 25.05 ± 0.2° and 6.55 ± 0.2°; preferably, the form has XRPD characteristic peaks at positions substantially as shown in Table 9; more preferably, the form has an XRPD pattern substantially as shown in Figure 9a; and optionally has one or more of the following features:
1) having an endothermic peak at 83.30°C ± 2°C and an endothermic peak at 126.89°C ± 2°C in DSC pattern;
2) having a weight loss of 12.51 ± 0.2 wt% before reaching 110.00°C, and a weight loss of 12.26 ± 0.2 wt% between 110.00°C and 250.00°C in TGA pattern;
3) having a DSC pattern substantially as shown in Figure 9b; and/or
4) having a TGA pattern substantially as shown in Figure 9c.

12. The form according to claim 1, wherein the form is a crystalline form E of the compound of formula A, which has at least three, at least four, at least five, at least six or seven characteristic peaks at the following positions in an X-ray powder diffraction (XRPD) pattern in 2θ angle: 11.14 ± 0.2°, 15.76 ± 0.2°, 12.59 ± 0.2°, 19.71 ± 0.2°, 9.56 ± 0.2°, 17.83 ± 0.2° and 13.58 ± 0.2°; preferably, the form has XRPD characteristic peaks at positions substantially as shown in Table 10; more preferably, the form has an XRPD pattern substantially as shown in Figure 10c; and optionally has one or more of the following features:
1) having an endothermic peak at 37.94°C ± 2°C and an endothermic peak at 190.71°C ± 2°C in DSC pattern;
2) having a weight loss of 1.17 ± 0.2 wt% before reaching 130°C in TGA pattern;
3) having a DSC pattern substantially as shown in Figure 10d; and/or
4) having a TGA pattern substantially as shown in Figure 10e.

13. The form according to claim 1, wherein the form is a crystalline form F of the compound of formula A, which has at least three, at least four, at least five, at least six or seven characteristic peaks at the following positions in an X-ray powder diffraction (XRPD) pattern in 2θ angle: 17.70 ± 0.2°, 21.46 ± 0.2°, 27.66 ± 0.2°, 19.20 ± 0.2°, 17.17 ± 0.2°, 19.44 ± 0.2° and 22.01 ± 0.2°; preferably, the form has XRPD characteristic peaks at positions substantially as shown in Table 11; more preferably, the form has an XRPD pattern substantially as shown in Figure 11a; and optionally has one or more of the following features:
1) having an endothermic peak at 216.58°C ± 2°C in DSC pattern;
2) having a weight loss of 0.598 ± 0.2 wt% before reaching 100°C in TGA pattern;
3) having a DSC pattern substantially as shown in Figure 11b; and/or
4) having a TGA pattern substantially as shown in Figure 11c.

14. The form according to claim 1, wherein the form is a crystalline form H of the compound of formula A, which has at least three, at least four, at least five, at least six or seven characteristic peaks at the following positions in an X-ray powder diffraction (XRPD) pattern in 2θ angle: 18.30 ± 0.2°, 3.80 ± 0.2 °, 19.05 ± 0.2°, 18.53 ± 0.2°, 11.81 ± 0.2°, 11.33 ± 0.2° and 16.04 ± 0.2°; preferably, the form has XRPD characteristic peaks at positions substantially as shown in Table 13: more preferably, the form has an XRPD pattern substantially as shown in Figure 13a; and optionally has one or more of the following features:
1) having an endothermic peak at 77.45°C ± 2°C and an endothermic peak at 154.75°C ± 2°C in DSC pattern;
2) having a weight loss of 0.19 ± 0.2 wt% before reaching 120°C in TGA pattern;
3) having a DSC pattern substantially as shown in Figure 13b; and/or
4) having a TGA pattern substantially as shown in Figure 13c.

15. The form according to claim 1, wherein the form is an amorphous form of the compound of formula A, preferably, the form has an XRPD pattern as shown in Figure 14a, more preferably, the form optionally has one or more of the following features:
1) having a glass transition temperature at 129.30°C ± 2.0°C in mDSC pattern;
2) having a weight loss of 2.7 wt% before reaching 210°C ± 2.0°C in TGA pattern;
3) having a mDSC pattern substantially as shown in Figure 14b; and/or
4) having a TGA pattern substantially as shown in Figure 14c.

16. A method for preparing the form according to any one of claims 1 to 15, wherein the method comprises the steps of: obtaining the form according to any one of claims 1 to 15 by subjecting the compound of formula A or a pharmaceutically acceptable salt thereof to suspend, slowly cool, rapidly cool, slowly volatilize, rapidly volatilize, add an antisolvent dropwise, add an antisolvent dropwise reversely, vapor diffusion or heat-cool DSC crystallization method, or spray dry, hot melt extrusion or solvent evaporation.

17. A pharmaceutical composition comprising:
(a) a form according to any one of claims 1 to 15; and
(b) a pharmaceutically acceptable carrier or excipient.

18. A pharmaceutical formulation comprising a pharmaceutical composition according to claim 17; wherein the pharmaceutical formulation is a solid formulation, and the dosage form of the solid formulation is selected from a group consisting of: powder, granules, tablet, capsule, pill or film.

19. Use of the form according to any one of claims 1 to 15, or a pharmaceutical composition according to claim 17, or a pharmaceutical formulation according to claim 18 in the manufacture of a medicament for treating a proliferative disease, preferably, the proliferative disease is selected from: breast cancer, colon cancer, brain cancer, prostate cancer, kidney cancer, pancreatic cancer, ovarian cancer, head and neck cancer, melanoma, colorectal cancer, gastric cancer, squamous cell carcinoma, small cell lung cancer, non-small cell lung cancer, testicular cancer, Merkel cell carcinoma, glioblastoma, neuroblastoma, lymphoid organ cancer and hematological malignancy including leukemia (acute lymphoblastic leukemia (ALL), acute myeloid leukemia (AML), chronic lymphocytic leukemia (CLL), chronic myeloid leukemia (CML), acute monocytic leukemia (AMOL), hairy cell leukemia (HCL), T cell prolymphocytic leukemia (T-PLL), large granular lymphocytic leukemia, adult T cell leukemia), lymphoma (small lymphocytic lymphoma (SLL), Hodgkin's lymphoma (nodular sclerosis, mixed cellular, lymphocyte-rich, lymphocyte depleted or not depleted, and nodular lymphocyte-predominant Hodgkin lymphoma), non-Hodgkin's lymphoma (all subtypes), chronic lymphocytic leukemia/small lymphocytic lymphoma, B cell prolymphocytic leukemia, lymphoplasmacytic lymphoma (such as Waldenstrom macroglobulinemia), splenic marginal zone lymphoma, plasma cell neoplasms (plasma cell myeloma, plasmacytoma, monoclonal immunoglobulin deposition disease, heavy chain disease), extranodal marginal zone B cell lymphoma (MALT lymphoma), lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, Burkitt lymphoma/leukemia, T cell prolymphocytic leukemia, T cell large granular lymphocytic leukemia, aggressive NK cell leukemia, adult T cell leukemia/lymphoma, extranodal NK/T cell lymphoma (nasal type), enteropathy-type T cell lymphoma, hepatosplenic T cell lymphoma, blastic NK cell lymphoma, mycosis fungoides/Sezary syndrome, primary cutaneous CD30-positive T cell lymphoma disease, primary cutaneous anaplastic large cell lymphoma, lymphomatoid papulosis, angioimmunoblastic T cell lymphoma, peripheral T cell lymphoma (unspecified), anaplastic large cell lymphoma, multiple myeloma (plasma cell myeloma or Kahler's disease).
